(19) Europäisches Patentamt
European Patent Office
Office européen des brevets

(11) EP 3 299 478 A1

(12) # EUROPEAN PATENT APPLICATION

(43) Date of publication:
**28.03.2018 Bulletin 2018/13**

(51) Int Cl.:
***C12Q 1/6886*** *(2018.01)*

(21) Application number: **17193243.7**

(22) Date of filing: **26.09.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **27.09.2016 US 201615277750**

(71) Applicant: **Oncology Venture ApS**
**2970 Hørsholm (DK)**

(72) Inventor: **KNUDSEN, Steen**
**Scottsdale, AZ 85258 (US)**

(74) Representative: **Carpmaels & Ransford LLP**
**One Southampton Row**
**London WC1B 5HA (GB)**

Remarks:
Claims filed after the date of filing of the application (Rule 68(4) EPC).

(54) **METHODS FOR PREDICTING DRUG RESPONSIVENESS IN CANCER PATIENTS**

(57) The present invention features methods, devices, and kits for detecting gene expression in a patient having cancer or determining responsive of a patient having cancer to a treatment, such as APO010. The invention further includes methods of treating a patient having cancer by administering, e.g., APO010.



Printed by Jouve, 75001 PARIS (FR)

## Description

## FIELD OF THE INVENTION

The invention features methods to detect the expression levels of genes coding biomarkers in cancer patients and to predict the responsiveness of cancer patients to APO010.

## BACKGROUND

DNA microarrays have been used to measure gene expression in tumor samples from patients and to facilitate diagnosis. Gene expression can reveal the presence of cancer in a patient in addition to the type, stage, and origin. Gene expression may even have a role in predicting the efficacy of cancer therapies. In recent decades, the National Cancer Institute (NCI) has tested cancer therapeutics for their effect in limiting the growth of 60 human cancer cell lines. The NCI has also measured gene expression in those 60 cancer cell lines using DNA microarrays. Various studies have explored the relationship between gene expression and therapeutic effect using the NCI datasets.

During cancer treatment, critical time is often lost due to a trial and error approach to finding an effective therapy. In addition, cancer cells often develop resistance to a previously effective therapy. In such situations, patient outcome would be greatly improved by early detection of such resistance.

Thus, there exists a need in the art for methods and devices that can predict the responsiveness of cancer patients to a medical treatment.

## SUMMARY OF THE INVENTION

The invention features methods for detecting gene expression of a biomarker (e.g., one, two, three, four, five, ten, twenty, or all of the biomarkers shown in Tables 1 and/or 2, such as CORO1A (SEQ ID NO: 2)) in a patient, such as a patient having recurrence of cancer (e.g., a patient having recurrence of multiple myeloma or breast cancer), and for determining responsiveness of a cancer patient (e.g., a patient with multiple myeloma or breast cancer)) to treatment with Apo010. The invention also features methods of treating cancer in a patient in need thereof (e.g., a patient with multiple myeloma or breast cancer or a recurrence thereof) that include administering APO010 to the patient, in which the patient is or has been determined to be responsive to APO010 according to the diagnostic methods described herein.

Exemplary types of cancer that can be diagnosed or treated with the methods include, e.g., myeloma (e.g., multiple myeloma), colorectal cancer (e.g., colon cancer and rectal cancer), leukemia (e.g., acute myeloid leukemia, acute lymphoid leukemia, chronic myeloid leukemia, chronic lymphocytic leukemia, acute myeloblastic leukemia, acute promyelocytic leukemia, acute myelomonocytic leukemia, acute monocytic leukemia, acute erythroleukemia, and chronic leukemia), myelodysplastic syndrome, lymphoma (e.g., diffuse large B-cell lymphoma, cutaneous T-cell lymphoma, peripheral T-cell lymphoma, Hodgkin's lymphoma, non-Hodgkin's lymphoma, Waldenstrom's macroglobulinemia, and lymphocytic lymphoma), cervical cancer, prostate cancer, esophageal cancer, melanoma, glioma (e.g., oligodendroglioma), pancreatic cancer (e.g., adenosquamous carcinoma, signet ring cell carcinoma, hepatoid carcinoma, colloid carcinoma, islet cell carcinoma, and pancreatic neuroendocrine carcinoma), ovarian cancer (e.g., ovarian adenocarcinoma or embryonal carcinoma), gastrointestinal stromal tumor, sarcoma (e.g., fibrosarcoma, myxosarcoma, liposarcoma, chondrosarcoma, osteogenic sarcoma, angiosarcoma, endotheliosarcoma, lymphangiosarcoma, lymphangioendotheliosarcoma, leiomyosarcoma, Ewing's sarcoma, and rhabdomyosarcoma), breast cancer (e.g., medullary carcinoma), ER-positive cancer, bladder cancer, head and neck cancer (e.g., squamous cell carcinoma of the head and neck), lung cancer (e.g., non-small cell lung carcinoma, large cell carcinoma, bronchogenic carcinoma, and papillary adenocarcinoma), metastatic cancer, oral cavity cancer, uterine cancer, testicular cancer (e.g., seminoma and embryonal carcinoma), skin cancer (e.g., squamous cell carcinoma, and basal cell carcinoma), thyroid cancer (e.g., papillary carcinoma and medullary carcinoma), brain cancer (e.g., astrocytoma and craniopharyngioma), stomach cancer, intra-epithelial cancer, bone cancer, biliary tract cancer, eye cancer, liver cancer (e.g., hepatocellular carcinoma or hepatoma), larynx cancer, kidney cancer (e.g., renal cell carcinoma and Wilms tumor), gastric cancer, blastoma (e.g., nephroblastoma, medulloblastoma, hemangioblastoma, neuroblastoma, and retinoblastoma), polycythemia vera, chordoma, synovioma, mesothelioma, adenocarcinoma, sweat gland carcinoma, sebaceous gland carcinoma, cystadenocarcinoma, bile duct carcinoma, choriocarcinoma, epithelial carcinoma, ependymoma, pinealoma, acoustic neuroma, schwannoma, meningioma, pituitary adenoma, nerve sheath tumor, cancer of the small intestine, cancer of the endocrine system, cancer of the penis, cancer of the urethra, cutaneous or intraocular melanoma, a gynecologic tumor, solid tumors of childhood, or neoplasms of the central nervous system. For example, the cancer may be a solid tumor or a hematological cancer.

A first aspect of the invention features a method for detecting expression of a biomarker (e.g., one, two, three, four, five, ten, twenty, or all of the biomarkers shown in Tables 1 and/or 2, such as CORO1A (SEQ ID NO: 2)) in a patient having cancer, such as recurrence of cancer (e.g., a patient having recurrence of multiple myeloma or breast cancer).

The method includes (a) contacting a sample (e.g., a tumor sample) from the patient including one or more nucleic acid molecules with a device (e.g., a microarray) including: i) one or more single-stranded nucleic acid molecules capable of specifically hybridizing with the nucleotides of one or more biomarkers of sensitivity selected from the biomarkers of Table 1 (e.g., CORO1A (SEQ ID NO: 2)); and/or ii) one or more single-stranded nucleic acid molecules capable of specifically hybridizing with the nucleotides of one or more biomarkers of resistance selected from the biomarkers of Table 2 (e.g., PDE8A (SEQ ID NO: 100)); and (b) detecting a level of expression of one or more of the biomarkers of sensitivity and/or one or more of the biomarkers of resistance by performing microarray analysis or quantitative reverse transcriptase polymerase chain reaction (qRT-PCR). Expression of the biomarker(s) may be detected by determining the level of a messenger RNA (mRNA) transcribed from a nucleic acid molecule corresponding to a gene of the biomarker (e.g., a mRNA expressed from the CORO1A gene (SEQ ID NO: 2)) or a complementary DNA (cDNA) thereof.

A second aspect of the invention features a method of determining responsiveness of a patient having cancer (e.g., one of the cancers noted above, such as multiple myeloma or breast cancer) to APO010. In particular, the patient may have recurrence of cancer, such as recurrence of multiple myeloma or breast cancer. The method includes a) contacting a sample (e.g., a tumor sample) from the patient including one or more nucleic acid molecules with a device (e.g., a microarray) including: i) one or more single-stranded nucleic acid molecules capable of specifically hybridizing with the nucleotides of one or more biomarkers of sensitivity selected from the biomarkers of Table 1 (e.g., CORO1A (SEQ ID NO: 2)); and/or ii) one or more single-stranded nucleic acid molecules capable of specifically hybridizing with the nucleotides of one or more biomarkers of resistance selected from the biomarkers of Table 2 (e.g., PDE8A (SEQ ID NO: 100)); and(b) measuring hybridization between the one or more nucleic acid molecules from the patient and the single-stranded nucleic acid molecules of the device to detect a level of expression of one or more of the biomarkers of sensitivity and/or one or more of the biomarkers of resistance. The patient is determined to be responsive to APO010 if: i) the level of expression of the biomarkers of sensitivity is substantially similar to the level of expression of the biomarkers of sensitivity (e.g., CORO1A (SEQ ID NO: 2)) in a cell (e.g., a cancer cell) or tissue (e.g., a tumor tissue) known to be sensitive to APO010; and/or ii) the level of expression of the biomarkers of resistance (e.g., PDE8A (SEQ ID NO: 100)) is substantially dissimilar to the level of expression of the biomarkers of resistance in a cell (e.g., a cancer cell) or tissue (e.g., a tumor tissue) known to be resistant to APO010.

The method of the second aspect can further include administering APO010 to the patient if: i) the level of expression of the biomarkers of sensitivity (e.g., CORO1A (SEQ ID NO: 2)) is substantially similar to the level of expression of the biomarkers of sensitivity in a cell (e.g., a cancer cell) or tissue (e.g., a tumor tissue) known to be sensitive to APO010; and/or ii) the level of expression of the biomarkers of resistance (e.g., PDE8A (SEQ ID NO: 100)) is substantially dissimilar to the level of expression of the biomarkers of resistance in a cell (e.g., a cancer cell) or tissue (e.g., a tumor tissue) known to be resistant to APO010. The method can further include administering one or more cancer therapies other than APO010 to the patient if: i) the level of expression of the biomarkers of sensitivity (e.g., CORO1A (SEQ ID NO: 2)) is substantially dissimilar to the level of expression of the biomarkers of sensitivity in a cell (e.g., a cancer cell) or tissue (e.g., a tumor tissue) known to be sensitive to APO010; and/or ii) the level of expression of the biomarkers of resistance is substantially similar to the level of expression of the biomarkers of resistance (e.g., PDE8A (SEQ ID NO: 100)) in a cell (e.g., a cancer cell) or tissue (e.g., a tumor tissue) known to be resistant to APO010. In particular, the one or more of the cancer therapies includes surgery, radiation, or a therapeutic agent, such as a histone deacetylase (HDAC) inhibitor, ipilimumab, bortezomib, carfilzomib, thalidomide, lenalidomide, pomalidomide, prednisone, dexamethasone, cyclophosphamide, vincristine, doxorubicin, melphalan, capecitabine, tegafur, irinotecan, oxaliplatin, cetuximab, leucovorin, SN-38, everolimus, temsirolimus, bleomycin, lomustine, depsipeptide, carboplatin, erlotinib, gemcitabine, mitoxantrone, cisplatin, busulfan, epirubicin, arsenic trioxide, bendamustine, fulvestrant, teniposide, adriamycin, decitabine, estramustine, etoposide, azaguanine, aclarubicin, mitoxantrone, mitomycin, paclitaxel, taxotere, Irofulven, 5-FU, ara-c, methylprednisolone, methotrexate, methyl-gag, belinostat, carboplatin, idarubicin, IL4-PR38, valproic acid, all-trans retinoic acid (ATRA), cytoxan, topotecan, suberoylanilide hydroxamic acid, leukeran, fludarabine, vinblastine, dacarbazine, hydroxyurea, tegafur, daunorubicin, mechlorethamine, streptozocin, carmustine, mercaptopurine, dactinomycin, tretinoin, ifosfamide, tamoxifen, floxuridine, thioguanine, PSC 833, herceptin, bevacizumab, celecoxib, iressa, anastrozole, letrozole, or rituximab.

The invention also features a method of treating cancer in a patient in need thereof (e.g., one of the cancers noted above, such as multiple myeloma or breast cancer) that includes administering APO010 to the patient, in which the patient has been determined to be responsive to APO010 according to the method of the first or second aspect of the invention. In particular, the patient may have recurrence of cancer, such as recurrence of multiple myeloma or breast cancer.

A third aspect of the invention features a method of treating a patient having cancer (e.g., one of the cancers noted above, such as multiple myeloma or breast cancer). In particular, the patient may have recurrence of cancer, such as recurrence of multiple myeloma or breast cancer. The method includes a) contacting a sample (e.g., a tumor sample) from the patient including one or more nucleic acid molecules with a device including: i) one or more single-stranded nucleic acid molecules capable of specifically hybridizing with the nucleotides of one or more biomarkers of sensitivity selected from the biomarkers of Table 1(e.g., CORO1A (SEQ ID NO: 2)); and/or ii) one or more single-stranded nucleic acid molecules capable of specifically hybridizing with the nucleotides of one or more biomarkers of resistance selected from the biomarkers of Table 2 (e.g., PDE8A (SEQ ID NO: 100)); b) measuring hybridization between the one or more nucleic acid molecules from the patient and the single-stranded nucleic acid molecules of the device to detect a level of expression of one or more of the biomarkers of sensitivity and/or one or more of the biomarkers of resistance; and c) administering APO010 to the patient if: i) the level of expression of the biomarkers of sensitivity is substantially similar to the level of expression of the biomarkers of sensitivity in a cell (e.g., a cancer cell) or tissue (e.g., a tumor tissue) known to be sensitive to APO010; and/or ii) the level of expression of the biomarkers of resistance is substantially dissimilar to the level of expression of the biomarkers of resistance in a cell (e.g., a cancer cell) or tissue (e.g., a tumor tissue) known to be resistant to APO010.

The method of the third aspect of the invention may further include administering one or more additional therapies (e.g., surgery, radiation, or a therapeutic agent) to the patient prior to, concurrently, or after administration of APO010. In particular, the therapeutic agent may be selected from the group consisting of a histone deacetylase (HDAC) inhibitor, ipilimumab, bortezomib, carfilzomib, thalidomide, lenalidomide, pomalidomide, prednisone, dexamethasone, cyclophosphamide, vincristine, doxorubicin, melphalan, capecitabine, tegafur, irinotecan, oxaliplatin, cetuximab, leucovorin, SN-38, everolimus, temsirolimus, bleomycin, lomustine, depsipeptide, carboplatin, erlotinib, gemcitabine, mitoxantrone, cisplatin, busulfan, epirubicin, arsenic trioxide, bendamustine, fulvestrant, teniposide, adriamycin, decitabine, estramustine, etoposide, azaguanine, aclarubicin, mitoxantrone, mitomycin, paclitaxel, taxotere, Irofulven, 5-FU, ara-c, methylprednisolone, methotrexate, methyl-gag, belinostat, carboplatin, idarubicin, IL4-PR38, valproic acid, all-trans retinoic acid (ATRA), cytoxan, topotecan, suberoylanilide hydroxamic acid, leukeran, fludarabine, vinblastine, dacarbazine, hydroxyurea, tegafur, daunorubicin, mechlorethamine, streptozocin, carmustine, mercaptopurine, dactinomycin, tretinoin, ifosfamide, tamoxifen, floxuridine, thioguanine, PSC 833, herceptin, bevacizumab, celecoxib, iressa, anastrozole, letrozole, and rituximab. The therapeutic agent can be administered parenterally (e.g. intravenously, intramuscularly, transdermally, intradermally, intra-arterially, intracranially, subcutaneously, intraorbitally, intraventricularly, intraspinally, intraperitoneally, or intranasally), enterally, or topically.

In the second or third aspect of the invention, APO010 may be administered parenterally (e.g. intravenously, intramuscularly, transdermally, intradermally, intra-arterially, intracranially, subcutaneously, intraorbitally, intraventricularly, intraspinally, intraperitoneally, or intranasally), enterally, or topically. Preferably, APO010 is administered by intravenous injection. APO010 may be administered to the patient two or more times, such as one or more times daily (e.g., once daily for up to six days), weekly, every two weeks, every three weeks, or monthly. The method may further include administering a second dose of APO010 to the patient two weeks, three weeks, four weeks, or five weeks after administration of a first dose of APO010. APO010 may be administered in a particular dosage form (e.g., liquid, tablet, capsule, etc.) and it may be administered at a dose of about 2 $\mu g/m^2$ to 500 $\mu g/m^2$ (e.g., about 45 $\mu g/m^2$ to 200 $\mu g/m^2$). In particular, the contacting (a) and measuring (b) steps occur prior to, concurrent, or after administration of APO010 to the patient, such as the contacting (a) and measuring (b) steps occur multiple times.

In any of the above aspects of the invention, the device can include at least two, at least three, at least four, at least five, at least six, at least seven, at least eight, at least nine, at least ten, or more single-stranded nucleic acid molecules capable of specifically hybridizing with the nucleotides of one or more biomarkers of sensitivity selected from the biomarkers of Table 1 (e.g., CORO1A (SEQ ID NO: 2)); and/or at least two, at least three, at least four, at least five, at least six, at least seven, at least eight, at least nine, at least ten, or more single-stranded nucleic acid molecules capable of specifically hybridizing with the nucleotides of one or more biomarkers of resistance selected from the biomarkers of Table 2 (e.g., PDE8A (SEQ ID NO: 100)). In particular, one or more of the single-stranded nucleic acid molecules of the device have a length in the range of 10 to 100 nucleotides in length (e.g., .a length in the range of 20 to 60 nucleotides).

In any of the above aspects of the invention, the method may include converting the level of expression of one or more of the biomarkers of sensitivity (e.g., one, two, three, four, five, ten, twenty, or all of the biomarkers shown in Table 1, such as CORO1A (SEQ ID NO: 2)) and/or one or more of the biomarkers of resistance (e.g., one, two, three, four, five, ten, twenty, or all of the biomarkers shown in Table 2, such as PDE8A (SEQ ID NO: 100)) into a mean score, in which the mean score indicates the responsiveness of the patient to APO010. The method can further include subtracting the mean score for one or more of the biomarkers of resistance (e.g., one, two, three, four, five, ten, twenty, or all of the biomarkers shown in Table 2, such as PDE8A (SEQ ID NO: 100)) from the mean score for one or more of the biomarkers of sensitivity (e.g., one, two, three, four, five, ten, twenty, or all of the biomarkers shown in Table 1, such as CORO1A (SEQ ID NO: 2)) to obtain a difference score, in which the difference score indicates the responsiveness of the patient to APO010. In particular, the mean score and/or the difference score above a cutoff value indicates that the patient is responsive to APO010, such as if the cutoff value is about 0.1, about 0.15, about 0.2, about 0.25, about 0.3, about 0.35, about 0.4, about 0.45, about 0.5, or greater.

In any of the above aspects of the invention, the biomarker of sensitivity may be selected from one or more of CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), and ITGA4 (SEQ ID NO: 36). Moreover, the biomarker of resistance may be selected from one or more of PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and CCND1 (SEQ ID NO: 129).

For example, the biomarkers of sensitivity may include: i) CORO1A (SEQ ID NO: 2) and CD47 (SEQ ID NO: 1); ii) CORO1A (SEQ ID NO: 2), CD47 (SEQ ID NO: 1), and ICAM3 (SEQ ID NO: 3); iii) CORO1A (SEQ ID NO: 2), CD47 (SEQ ID NO: 1), ICAM3 (SEQ ID NO: 3), and HCLS1 (SEQ ID NO: 4); iv) CORO1A (SEQ ID NO: 2), CD47 (SEQ ID NO: 1), ICAM3 (SEQ ID NO: 3), HCLS1 (SEQ ID NO: 4), and DOCK2 (SEQ ID NO 5); v) CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), and ARHGAP15 (SEQ ID NO: 6); vi) CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), and CXCR4 (SEQ ID NO: 7 or 9 or 16); vii) CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), and NCKAP1L (SEQ ID NO: 8); viii) CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), and MAP4K1 (SEQ ID NO: 10 or 17 or 19); or ix) CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), and SLA (SEQ ID NO: 11).

For example, the biomarkers of resistance may include: i) PDE8A (SEQ ID NO: 100) and HOXB7 (SEQ ID NO: 101 or 103); ii) PDE8A (SEQ ID NO: 100), HOXB7 (SEQ ID NO: 101 or 103), and PRC1 (SEQ ID NO: 102); iii) PDE8A (SEQ ID NO: 100), HOXB7 (SEQ ID NO: 101 or 103), PRC1 (SEQ ID NO: 102), and SCRN1 (SEQ ID NO: 104); iv) PDE8A (SEQ ID NO: 100), HOXB7 (SEQ ID NO: 101 or 103), PRC1 (SEQ ID NO: 102), SCRN1 (SEQ ID NO: 104), and ACTN1 (SEQ ID NO: 105 or 109); v) PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), and NGRN (SEQ ID NO: 106); vi) PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), and DKK1 (SEQ ID NO: 107); vii) PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), and IER3 (SEQ ID NO: 108); viii) PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), and NQO1 (SEQ ID NO: 110 or 121 or 127); or ix) PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), and ANXA2 (SEQ ID NO: 111 or 116 or 122).

In any of the above aspects of the invention, the device can be a microarray, such as a deoxyribonucleic acid (DNA)-based platform. The expression level of the biomarkers of sensitivity (e.g., one, two, three, four, five, ten, twenty, or all of the biomarkers shown in Table 1, such as CORO1A (SEQ ID NO: 2)) and/or the biomarkers of resistance (e.g., one, two, three, four, five, ten, twenty, or all of the biomarkers shown in Table 2, such as PDE8A (SEQ ID NO: 100)) can be measured using qRT-PCR. In particular, the level of expression of the biomarkers of sensitivity and/or biomarkers of resistance is determined by detecting the level of mRNA transcribed from a gene coding one or more of the biomarkers of Tables 1 and 2.

In any of the above aspects of the invention, the cancer is selected from a solid tumor cancer and a hematological cancer. For example, the cancer is, e.g., multiple myeloma, breast cancer, acute myelogenous leukemia (AML), acute lympho-blastic leukemia (ALL), chronic lymphocytic leukemia (CLL), myelodysplastic syndrome (MDS), chronic myelogenous leukemia - chronic phase (CMLCP), diffuse large B-cell lymphoma (DLBCL), cutaneous T-cell lymphoma (CTCL), peripheral T-cell lymphoma (PTCL), Hodgkin's lymphoma, hepatocellular carcinoma (HCC), cervical cancer, prostate cancer, renal cell carcinoma (RCC), esophageal cancer, melanoma, glioma, pancreatic cancer, ovarian cancer, gastrointestinal stromal tumors (GIST), sarcoma, estrogen receptor-positive (ERpos) breast cancer, non-small cell lung carcinoma (NSCLC), colon cancer, bladder cancer, and squamous cell carcinoma of the head and neck (SCCHN).

**Definitions**

As used herein, "a" or "an" means "at least one" or "one or more" unless otherwise indicated. In addition, the singular forms "a", "an", and "the" include plural referents unless the context clearly dictates otherwise.

As used herein, "about" refers to an amount ± 10% of the recited value.

The term "APO010" as used herein refers to a recombinant, soluble, hexameric fusion protein consisting of three human Fas ligand (FasL) extracellular domains fused to the dimer-forming collagen domain of human adiponectin. FasL is a transmembrane protein of the tumor necrosis factor (TNF) superfamily and a pro-apoptotic ligand for the death receptor Fas. The hexameric APO010 structure is a Fas receptor agonist that mimics the ligand clustering activity of endogenous FasL, which results in caspase-dependent apoptosis in susceptible tumor cell populations. APO010 is also known as Mega-Fas-Ligand. APO010 is also described in Eisele et al. (Neuro. Oncol. 13:155-164, 2010), hereby incorporated by reference.

By "biomarker" is meant a nucleic acid molecule (e.g., a mRNA or its complement, for example, a cDNA) or a protein encoded by the nucleic acid molecule present in, or from, a cell or tissue. The expression of the biomarker correlates to the responsiveness (e.g., sensitivity or resistance) of the cell or tissue (and thus, the patient containing the cell or tissue or the patient from which the cell or tissue was obtained) to a cancer treatment (e.g., APO010). In particular, a biomarker of sensitivity is a nucleic acid molecule (e.g., a mRNA or its complement) expressed from any one of the genes shown in Table 1, or the protein encoded by the nucleic acid molecule, and a biomarker of resistance is a nucleic acid molecule (e.g., a mRNA or its complement) expressed from any one of the genes shown in Table 2, or the protein encoded by the nucleic acid molecule.

The terms "cancer" and "cancerous" refer to or describe the physiological condition in mammals (e.g., humans) that is typically characterized by unregulated cell proliferation. Examples of cancer include, but are not limited to, myeloma (e.g., multiple myeloma), colorectal cancer (e.g., colon cancer and rectal cancer), leukemia (e.g., acute myeloid leukemia, acute lymphoid leukemia, chronic myeloid leukemia, chronic lymphocytic leukemia, acute myeloblastic leukemia, acute promyelocytic leukemia, acute myelomonocytic leukemia, acute monocytic leukemia, acute erythroleukemia, and chronic leukemia), myelodysplastic syndrome, lymphoma (e.g., diffuse large B-cell lymphoma, cutaneous T-cell lymphoma, peripheral T-cell lymphoma, Hodgkin's lymphoma, non-Hodgkin's lymphoma, Waldenstrom's macroglobulinemia, and lymphocytic lymphoma), cervical cancer, prostate cancer, esophageal cancer, melanoma, glioma (e.g., oligodendroglioma), pancreatic cancer (e.g., adenosquamous carcinoma, signet ring cell carcinoma, hepatoid carcinoma, colloid carcinoma, islet cell carcinoma, and pancreatic neuroendocrine carcinoma), ovarian cancer (e.g., ovarian adenocarcinoma or embryonal carcinoma), gastrointestinal stromal tumor, sarcoma (e.g., fibrosarcoma, myxosarcoma, liposarcoma, chondrosarcoma, osteogenic sarcoma, angiosarcoma, endotheliosarcoma, lymphangiosarcoma, lymphangioendotheliosarcoma, leiomyosarcoma, Ewing's sarcoma, and rhabdomyosarcoma), breast cancer (e.g., medullary carcinoma), ER-positive cancer, bladder cancer, head and neck cancer (e.g., squamous cell carcinoma of the head and neck), lung cancer (e.g., non-small cell lung carcinoma, large cell carcinoma, bronchogenic carcinoma, and papillary adenocarcinoma), metastatic cancer, oral cavity cancer, uterine cancer, testicular cancer (e.g., seminoma and embryonal carcinoma), skin cancer (e.g., squamous cell carcinoma and basal cell carcinoma), thyroid cancer (e.g., papillary carcinoma and medullary carcinoma), brain cancer (e.g., astrocytoma and craniopharyngioma), stomach cancer, intra-epithelial cancer, bone cancer, biliary tract cancer, eye cancer, liver cancer (e.g., hepatocellular carcinoma or hepatoma), larynx cancer, kidney cancer (e.g., renal cell carcinoma and Wilms tumor), gastric cancer, blastoma (e.g., nephroblastoma, medulloblastoma, hemangioblastoma, neuroblastoma, and retinoblastoma), polycythemia vera, chordoma, synovioma, mesothelioma, adenocarcinoma, sweat gland carcinoma, sebaceous gland carcinoma, cystadenocarcinoma, bile duct carcinoma, choriocarcinoma, epithelial carcinoma, ependymoma, pinealoma, acoustic neuroma, schwannoma, meningioma, pituitary adenoma, nerve sheath tumor, cancer of the small intestine, cancer of the endocrine system, cancer of the penis, cancer of the urethra, cutaneous or intraocular melanoma, a gynecologic tumor, solid tumors of childhood, and neoplasms of the central nervous system. The term cancer includes hematological cancers (e.g., cancer of the blood, such as multiple myeloma) and solid tumors (e.g., breast cancer).

The terms "expression level" and "level of expression," as used herein, refer to the amount of a gene product in a cell, tissue, biological sample, organism, or patient, e.g., amounts of DNA, RNA (e.g. messenger RNA(mRNA)), or proteins of a given gene.

"Gene" as used herein indicates a coding or noncoding gene whose activity can be determined by measuring the produced RNA. Examples include protein coding genes, microRNAs, small nuclear RNAs and other RNAs with catalytic, regulatory or coding properties.

To "inhibit growth" as used herein means causing a reduction in cell growth (e.g., cancer cell growth, such as the NCI60 cancer cell lines) *in vivo* or *in vitro* by, e.g., 10%, 20%, 30%, 40%, 50%, 60%, 70%, 80%, 90%, 95%, or 99% or more, as evident by a reduction in the proliferation of cells exposed to a treatment (e.g., APO010), relative to the proliferation of cells in the absence of the treatment. Growth inhibition may be the result of a treatment (e.g., APO010) that induces apoptosis in a cell, induces necrosis in a cell, slows cell cycle progression, disrupts cellular metabolism, induces cell lysis, or induces some other mechanism that reduces the proliferation of cells.

"Microarray" as used herein means a device employed by any method that quantifies one or more subject oligonucleotides, e.g., RNA, DNA, cDNA, or analogues thereof, at a time. For example, many DNA microarrays, including those made by Affymetrix (e.g., an Affymetrix HG-U133A array), use several probes for determining the expression of a single gene. The DNA microarray may contain oligonucleotide probes that may be, e.g., full-length cDNAs complementary to an RNA or cDNA fragments that hybridize to part of an RNA. The DNA microarray may also contain modified versions of DNA or RNA, such as locked nucleic acids or LNA. Exemplary RNAs include mRNA, miRNA, and miRNA precursors.

As used herein, the term "percent (%) sequence identity" refers to the percentage of nucleic acid residues of a candidate sequence, e.g., a probe or primer of the invention, that are identical to the nucleic acid residues of a reference sequence, e.g., a biomarker sequence of the invention, after aligning the sequences and introducing gaps, if necessary, to achieve the maximum percent sequence identity (e.g., gaps can be introduced in one or both of the candidate and reference sequences for optimal alignment and non-homologous sequences can be disregarded for comparison purposes). Alignment for purposes of determining percent sequence identity can be achieved in various ways that are within the skill in the art, for instance, using computer software, such as BLAST, BLAST-2, BLAST-P, BLAST-N, BLAST-X, WU-BLAST-2, ALIGN, ALIGN-2, CLUSTAL, Megalign (DNASTAR). In addition, those skilled in the art can determine appropriate parameters for measuring alignment, including any algorithms needed to achieve optimal alignment over the length of the sequences being compared.

"NCI60" as used herein means a panel of 60 cancer cell lines from lung, colon, breast, ovarian, leukemia, renal, melanoma, prostate and brain cancers including the following cancer cell lines: NSCLC_NCIH23, NSCLC_NCIH522, NSCLC_A549ATCC, NSCLC_EKVX, NSCLC_NCIH226, NSCLC_NCIH332M, NSCLC_H460, NSCLC_HOP62, NSCLC_HOP92, COLON_HT29, COLON_HCC-2998, COLON_HCT116, COLON_SW620, COLON_COLO205, COLON_HCT15, COLON_KM12, BREAST_MCF7, BREAST_MCF7ADRr, BREAST_MDAMB231, BREAST_HS578T, BREAST_MDAMB435, BREAST_MDN, BREAST_BT549, BREAST_T47D, OVAR_OVCAR3, OVAR_OVCAR4, OVAR_OVCAR5, OVAR_OVCAR8, OVAR_IGROV1, OVAR_SKOV3, LEUK_CCRFCEM, LEUK_K562, LEUK_MOLT4, LEUK_HL60, LEUK_RPMI8266, LEUK_SR, RENAL_UO31, RENAL_SN12C, RENAL_A498, RENAL_CAKI1, RENAL_RXF393, RENAL_7860, RENAL_ACHN, RENAL_TK10, MELAN_LOXIMVI, MELAN_MALME3M, MELAN_SKMEL2, MELAN_SKMEL5, MELAN_SKMEL28, MELAN_M14, MELAN_UACC62, MELAN_UACC257, PROSTATE_PC3, PROSTATE_DU145, CNS_SNB19, CNS_SNB75, CNS_U251, CNS_SF268, CNS_SF295, and CNS_SF539.

The terms "patient" and "subject," as used interchangeably herein, refer to any animal (e.g., a mammal, such as a human). A patient to be treated or tested for responsiveness to a treatment (e.g., APO010) according to the methods described herein may be one who has been diagnosed with a cancer, such as multiple myeloma or breast cancer. Diagnosis may be performed by any method or techniques known in the art, such as x-ray, MRI, or biopsy, and confirmed by a physician. To minimize exposure of a patient to drug treatments that may not be therapeutic, the patient may be determined to be either responsive or non-responsive to a cancer treatment, such as APO010, according to the methods described herein.

"Resistant" or "resistance" as used herein means that a cell (e.g., a cancer cell), a tissue (e.g., a tumor), or a patient having cancer (e.g., a human having cancer) is able to withstand treatment with an anti-cancer agent (e.g., APO010). For example, the cancer treatment (e.g., APO010) does not inhibit growth of a cancer cell *in vitro* relative to the growth of a cancer cell not exposed to the treatment. Resistance to treatment may be determined by a cell proliferation assay, e.g., a cell-based assay, which measures the growth of treated cells as a function of the absorbance of the cells of an incident light beam, such as the NCI60 assays described herein. In this assay, greater absorbance indicates greater cell growth, and thus, resistance to the treatment.

The terms "responsive" and "responsiveness," as used herein, refer to the likelihood that a cancer treatment (e.g., APO010) has (e.g., induces) a desired effect, or alternatively refers to the strength of a desired effect caused or induced by the treatment in a cell (e.g., a cancer cell), a tissue (e.g., a tumor), or a patient having cancer (e.g., a human having cancer). For example, the desired effect can include inhibition of the growth of a cancer cell *in vitro* by more than 10%, 20%, 30%, 40%, 50%, 60%, 70%, 80%, 90%, or 100% relative to the growth of a cancer cell not exposed to the treatment. The desired effect can also include reduction in tumor mass by, e.g., about 10%, 20%, 30%, 40%, 50%, 60%, 70%, 80%, 90%, or 100%. Responsiveness to treatment may be determined by a cell proliferation assay, e.g., a cell-based assay, which measures the growth of treated cells as a function of the absorbance of the cells of an incident light beam, such as the NCI60 assays described herein. In this assay, lesser absorbance indicates lesser cell growth, and thus, sensitivity to the treatment. A greater reduction in growth indicates more sensitivity to the treatment. In particular, "responsiveness" is a measure of the sensitivity or resistance of a patient to a treatment for cancer (e.g., APO010).

The term "sample," as used herein, refers to any specimen (such as cells, tissue (e.g., a tissue sample obtained by biopsy), blood, serum, plasma, urine, cerebrospinal fluid, or pancreatic fluid) taken from a subject. Preferably, the sample is taken from a portion of the body affected by a cancer (e.g., a biopsy of the cancer tissue). Biopsy may involve fine needle aspiration biopsy, core needle biopsy (e.g., stereotactic core needle biopsy, vacuum-assisted core biopsy, or magnetic resonance imaging (MRI) guided biopsy), or surgical biopsy (e.g., incisional biopsy or excisional biopsy). The sample may undergo additional purification and processing, for example, to remove cell debris and other unwanted molecules. Additional processing may further involve amplification, e.g., using PCR (RT-PCR). The standard methods of sample purification, such as removal of unwanted molecules, are known in the art.

"Sensitive" and "sensitivity" as used herein refer to a cell (e.g., a cancer cell), a tissue (e.g., a tumor), or a patient having cancer (e.g., a human having cancer) that is responsive to treatment, such as an anti-cancer agent (e.g., APO010) or radiation treatment. In particular, the treatment inhibits the growth of a cancer cell *in vitro* by about 70%, 80%, 90%, 95%, 99% or 100% relative to the growth of a cancer cell not exposed to the treatment. Sensitivity to treatment may be determined by a cell proliferation assay, e.g., a cell-based assay, which measures the growth of treated cells as a function of the absorbance of the cells of an incident light beam, such as the NCI60 assays described herein. In this assay, lesser absorbance indicates lesser cell growth, and thus, sensitivity to the treatment.

"Treatment," "medical treatment," to "treat," and "therapy," as used interchangeably herein, refer to administering or exposing a patient having cancer (e.g., a human), a cell, or a tumor to an anti-cancer agent (e.g., a drug, a protein, an antibody, a nucleic acid, a chemotherapeutic agent, or a radioactive agent), or to some other form of medical intervention used to treat or prevent a disease, disorder, or condition (e.g., surgery, cryotherapy, radiation therapy, or combinations thereof). In particular, a medical treatment can include APO010. For example, the cancer to be treated is a hematological cancer or a solid tumor. Examples of cancer include, e.g., myeloma (e.g., multiple myeloma), colorectal cancer (e.g., colon cancer and rectal cancer), leukemia (e.g., acute myeloid leukemia, acute lymphoid leukemia, chronic myeloid leukemia, chronic lymphocytic leukemia, acute myeloblastic leukemia, acute promyelocytic leukemia, acute myelomonocytic leukemia, acute monocytic leukemia, acute erythroleukemia, and chronic leukemia), myelodysplastic syndrome, lymphoma (e.g., diffuse large B-cell lymphoma, cutaneous T-cell lymphoma, peripheral T-cell lymphoma, Hodgkin's lymphoma, non-Hodgkin's lymphoma, Waldenstrom's macroglobulinemia, and lymphocytic lymphoma), cervical cancer, prostate cancer, esophageal cancer, melanoma, glioma (e.g., oligodendroglioma), pancreatic cancer (e.g., adenosquamous carcinoma, signet ring cell carcinoma, hepatoid carcinoma, colloid carcinoma, islet cell carcinoma, and pancreatic neuroendocrine carcinoma), ovarian cancer (e.g., ovarian adenocarcinoma or embryonal carcinoma), gastrointestinal stromal tumor, sarcoma (e.g., fibrosarcoma, myxosarcoma, liposarcoma, chondrosarcoma, osteogenic sarcoma, angiosarcoma, endotheliosarcoma, lymphangiosarcoma, lymphangioendotheliosarcoma, leiomyosarcoma, Ewing's sarcoma, and rhabdomyosarcoma), breast cancer (e.g., medullary carcinoma), ER-positive cancer, bladder cancer, head and neck cancer (e.g., squamous cell carcinoma of the head and neck), lung cancer (e.g., non-small cell lung carcinoma, large cell carcinoma, bronchogenic carcinoma, and papillary adenocarcinoma), metastatic cancer, oral cavity cancer, uterine cancer, testicular cancer (e.g., seminoma and embryonal carcinoma), skin cancer (e.g., squamous cell carcinoma, and basal cell carcinoma), thyroid cancer (e.g., papillary carcinoma and medullary carcinoma), brain cancer (e.g., astrocytoma and craniopharyngioma), stomach cancer, intra-epithelial cancer, bone cancer, biliary tract cancer, eye cancer, liver cancer (e.g., hepatocellular carcinoma or hepatoma), larynx cancer, kidney cancer (e.g., renal cell carcinoma and Wilms tumor), gastric cancer, blastoma (e.g., nephroblastoma, medulloblastoma, hemangioblastoma, neuroblastoma, and retinoblastoma), polycythemia vera, chordoma, synovioma, mesothelioma, adenocarcinoma, sweat gland carcinoma, sebaceous gland carcinoma, cystadenocarcinoma, bile duct carcinoma, choriocarcinoma, epithelial carcinoma, ependymoma, pinealoma, acoustic neuroma, schwannoma, meningioma, pituitary adenoma, nerve sheath tumor, cancer of the small intestine, cancer of the endocrine system, cancer of the penis, cancer of the urethra, cutaneous or intraocular melanoma, a gynecologic tumor, solid tumors of childhood, or neoplasms of the central nervous system. Radiation therapy includes the administration of a radioactive agent to a patient or exposure of a patient to radiation. The radiation may be generated from sources such as particle accelerators and related medical devices or agents that emit, e.g., X-radiation, gamma radiation, or electron (Beta radiation) beams. A treatment may be or further include surgery, e.g., to remove a tumor from a subject or living organism.

Other features and advantages of the invention will be apparent from the following Detailed Description, the drawings, and the claims.

## BRIEF DESCRIPTION OF THE DRAWINGS

Figure 1 is an image showing the methods used to identify the genes listed in Tables 1 and 2 as biomarkers that can be used to predict responsiveness to APO010. The gene expression profiles and growth inhibition data of the NCI60 cancer cell lines in the presence of APO010 are compared, and the identified gene expression profile is then filtered against the mRNA expression of a collection of~3000 human tumors.

Figure 2 is a graph grouping predicted sensitivity to APO010 by cancer type. Each gray circle represents the predicted APO010 sensitivity of one patient calculated as the difference between the mean of the expression levels of the biomarkers of sensitivity and the mean of the expression levels of the biomarkers of resistance for the patient. Patients are grouped according to cancer type. The median predicted sensitivity (black bar) for a cancer type is related to the relative response rate for that cancer type. The predictions are used for relative comparisons to compare cancer types and cannot be used for absolute predictions of response rate for a given cancer type. The predictions are normalized to a scale of 0 to 100 for all 3,522 patients.

Figure 3 is a graph showing the anti-tumor activity of APO010 at dosages of 0.01 mg/kg, 0.02 mg/kg, and 0.03 mg/kg in a human multiple myeloma cell line (OPM-2). Arrows indicate administration of APO010.

Figure 4 is a graph showing a comparison of the anti-tumor activity of APO010 at a dosage of 0.02 mg/kg and bortezomib (VELCADE®) at a dosage of 0.5 mg/kg in the OPM-2 cell line. Arrows indicate administration of APO010 and bortezomib.

Figure 5 is an image showing the methods used to determine the responsiveness of multiple myeloma patients to APO0100. CD138 positive plasma cells are isolated from the bone marrow of multiple myeloma patients and screened using the biomarkers of Tables 1 and 2. Patients predicted to be responsive to APO010 will then be treated with APO010.

## DETAILED DESCRIPTION OF THE INVENTION

We have discovered that the expression levels of the biomarkers shown in Tables 1 and 2 may be detected in a patient having cancer and are useful for predicting the responsiveness of the patient to APO010. A device, such as a microarray, with one or more single-stranded oligonucleotide probes that have substantial identity (e.g., at least 85%, 90%, 95%, 99%, or 100% sequence identity) to a sequence that is complementary or identical to the nucleic acid sequence of one or more biomarkers shown in Tables 1 and 2 can be used according to the method described herein to assess the responsiveness of a cancer patient to treatment with APO010. For example, the probes can be used to detect one or more (e.g., two, three, four, five, ten, twenty, or all) of the biomarkers of sensitivity listed in Table 1, such as CORO1A (SEQ ID NO: 2), in a sample (e.g., a tumor sample) from a patient having cancer. Additionally, the probes can be used to detect one or more (e.g., two, three, four, five, ten, twenty, or all) of the biomarkers of resistance listed in Table 2, such as PDE8A (SEQ ID NO: 100), in a sample (e.g., a tumor sample) from a patient having cancer. Accordingly, the invention features individual biomarkers (e.g., CORO1A (SEQ ID NO: 2) or PDE8A(SEQ ID NO: 100)) and sets of biomarkers shown in Tables 1 and 2 can be used to determine of the responsiveness of a cancer patient to APO010 at various stages of disease progression (e.g., patients diagnosed with cancer or patients after cancer recurrence) and at different times during the treatment process (e.g., prior to administration of any cancer treatment, after administration of one or more cancer treatments other than APO010, prior to administration of APO010, or during administration of APO010).

In particular, the invention provides methods for determining whether a patient may be responsive to APO010 by, e.g., detecting the expression level (e.g., mRNA or protein expression level) of one or more of the biomarkers shown in Tables 1 and/or 2 (e.g., CORO1A (SEQ ID NO: 2)) in a biological sample (e.g., a tumor biopsy) obtained from the subject using a device (e.g., a microarray). The expression level of one or more of the biomarkers of sensitivity may then be compared to the expression level of the biomarkers in a cell or tissue known to be sensitive or resistant to APO010 to determine the patient's responsiveness to APO010.

The patient may be responsive to APO010 if the expression level of the one or more of the biomarkers of sensitivity (e.g., one or more of CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), and ITGA4 (SEQ ID NO: 36)) is substantially similar to the expression level of the biomarkers of sensitivity in a cell or tissue known to be sensitive to APO010. The patient may also be responsive to APO010 if the level of expression of one or more of the biomarkers of resistance (e.g., one or more of PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and CCND1 (SEQ ID NO: 129)) is substantially dissimilar to the expression level of the biomarkers of resistance in a cell or tissue known to be resistant to APO010.

The invention also features methods of treating a patient having cancer, such as a patient having recurrence of cancer, by detecting the expression levels of one or more of the biomarkers shown in Tables 1 and 2 (e.g., CORO1A (SEQ ID NO: 2)) in a sample (e.g., a tumor sample) from the patient, and then administering APO010 based on the expression levels of the biomarkers. In particular, a patient having cancer may be administered APO010 if the expression level of one or more biomarkers of sensitivity is substantially similar to the expression level of the biomarkers of sensitivity in a cell or tissue known to be sensitive to APO010. Additionally, a patient having cancer may be administered APO010 if the expression level of one or more biomarkers of resistance is substantially dissimilar to the expression level of the biomarkers of resistance in a cell or tissue known to be resistant to APO010. Thus, the methods can be used to treat cancer patients predicted to be responsive to APO010, such as patients having, e.g., multiple myeloma, breast cancer, acute myelogenous leukemia (AML), acute lympho-blastic leukemia (ALL), chronic lymphocytic leukemia (CLL), myelodysplastic syndrome (MDS), chronic myelogenous leukemia - chronic phase (CMLCP), diffuse large B-cell lymphoma (DLBCL), cutaneous T-cell lymphoma (CTCL), peripheral T-cell lymphoma (PTCL), Hodgkin's lymphoma, hepatocellular carcinoma (HCC), cervical cancer, prostate cancer, renal cell carcinoma (RCC), esophageal cancer, melanoma, glioma, pancreatic cancer, ovarian cancer, gastrointestinal stromal tumors (GIST), sarcoma, estrogen receptor-positive (ERpos) breast cancer, non-small cell lung carcinoma (NSCLC), colon cancer, bladder cancer, or squamous cell carcinoma of the head and neck (SCCHN)).

Methods are described herein for identifying biomarkers of drug responsiveness, detecting biomarker gene expression in cancer patients, determining the responsiveness of a cancer patient to APO010, and treating cancer patients with APO010. Also described are devices and kits for use in these methods.

**Methods for Identifying Biomarkers of Drug Responsiveness**

The invention features methods for identifying biomarkers (e.g., one or more of the biomarkers of Tables 1 and 2) for determining the responsiveness of a cancer patient to a cancer treatment, such as APO010. Such methods can involve, for example, an algorithm based on growth inhibition values (GI50) of cell lines (e.g., NCI60 cell lines) subjected to treatment with APO010, followed by measurement of gene expression (e.g., using a microarray (e.g., an Affymetrix HG-U133A Genechip array)).

*Methodology of the In Vitro Cancer Growth Inhibition Screen*

The human tumor cell lines of the cancer screening panel may be grown in RPMI 1640 medium containing 5% fetal bovine serum and 2 mM L-glutamine. Cells may be inoculated into 96 well microtiter plates in 100 μL at plating densities ranging from 5,000 to 40,000 cells/well depending on the doubling time of individual cell lines. After cell inoculation, the microtiter plates may be incubated at 37°C, 5% CO2, 95% air and 100% relative humidity for 24 hours prior to addition of experimental compounds.

After 24 hours, two plates of each cell line may be fixed in situ with TCA, to represent a measurement of the cell population for each cell line at the time of compound addition (Tz). Experimental compounds may be solubilized in dimethyl sulfoxide at 400-fold the desired final maximum test concentration and stored frozen prior to use. At the time of compound (e.g., APO010) addition, an aliquot of frozen concentrate may be thawed and diluted to twice the desired final maximum test concentration with complete medium containing 50 μg/ml Gentamicin. Additional four, 10-fold or ½ log serial dilutions are made to provide a total of five concentrations plus control. Aliquots of 100 μl of these different compound dilutions are added to the appropriate microtiter wells already containing 100 μl of medium, resulting in the required final compound concentrations.

Following compound (e.g., APO010) addition, the plates may be incubated for an additional 48 h at 37°C, 5% CO2, 95% air, and 100% relative humidity. For adherent cells, the assay may be terminated by the addition of cold TCA. Cells may be fixed in situ by the gentle addition of 50 μl of cold 50% (w/v) TCA (final concentration, 10% TCA) and incubated for 60 minutes at 4°C. The supernatant may be discarded, and the plates may be washed five times with tap water and air-dried. Sulforhodamine B (SRB) solution (100 μl) at 0.4% (w/v) in 1% acetic acid may be added to each well, and plates are incubated for 10 minutes at room temperature. After staining, unbound dye may be removed by washing five times with 1% acetic acid and the plates may be air-dried. Bound stain may be subsequently solubilized with 10 mM trizma base, and the absorbance may be read on an automated plate reader at a wavelength of 515 nm. For suspension cells, the methodology may be the same, except that the assay may be terminated by fixing settled cells at the bottom of the wells by gently adding 50 μl of 80% TCA (final concentration, 16 % TCA). Using the seven absorbance measurements [time zero, (Tz), control growth, (C), and test growth in the presence of compound (e.g., APO010) at the five concentration levels (Ti)], the percentage growth may be calculated at each of the compound concentrations levels. Percentage growth inhibition may be calculated as:

$$[(Ti-Tz)/(C-Tz)] \times 100$$

for concentrations for which Ti>/=Tz

$$[(Ti-Tz)/Tz] \times 100$$

for concentrations for which Ti<Tz

Three dose response parameters may be calculated for each experimental agent (e.g., APO010). Growth inhibition of 50% (GI50) is calculated from [(Ti-Tz)/(C-Tz)] x 100 = 50, which is the agent (e.g., APO010) concentration resulting in a 50% reduction in the net protein increase (as measured by SRB staining) in control cells during the compound incubation. The compound concentration resulting in total growth inhibition (TGI) is calculated from Ti = Tz. The LC50 (concentration of compound resulting in a 50% reduction in the measured protein at the end of the compound treatment as compared to that at the beginning) indicating a net loss of cells following treatment is calculated from [(Ti-Tz)/Tz] x 100 = -50. Values are calculated for each of these three parameters if the level of activity is reached; however, if the effect is not reached or is exceeded, the value for that parameter is expressed as greater or less than the maximum or minimum concentration tested.

*Gene Expression and Growth Inhibition Analysis*

The gene expression measurements of NCI60 cancer cell lines can be obtained from a publically available database (e.g., the National Cancer Institute and the Massachusetts Institute of Technology). Each dataset can be normalized so that sample expression measured by different chips can be compared. The preferred method of normalization is the logit transformation, which may be performed for each gene y on each chip, as follows:

$$\text{logit}(y) = \log [(y\text{-background}) / (\text{saturation} - y)],$$

where background is calculated as the minimum intensity measured on the chip minus 0.1% of the signal intensity range: min-0.001*(max-min), and saturation is calculated as the maximum intensity measured on the chip plus 0.1% of the signal intensity range: max+0.001*(max-min). The resulting logit transformed data may then be z-transformed to mean zero and standard deviation 1.

Next, gene expression can be correlated to cancer cell growth inhibition. Growth inhibition data (GI50) of the NCI60 cell lines in the presence of a cancer treatment, such as APO010, can be obtained from the NCI. The correlation between the logit-transformed expression level of each gene in each cell line and the logarithm of GI50 (the concentration of a given compound that results in a 50% inhibition of growth) can be calculated, e.g., using the Pearson correlation coefficient or the Spearman Rank-Order correlation coefficient. Instead of using GI50s, any other measure of patient sensitivity to a given treatment (e.g., APO010) may be correlated to a gene expression levels of the patient. Since a plurality of measurements may be available for a single gene, the most accurate determination of correlation coefficient can be, e.g., the median of the correlation coefficients calculated for all probes measuring expression of the same gene.

For example, the median correlation coefficient of gene expression measured on a probe to growth inhibition or patient sensitivity to APO010 can be calculated for all genes of interest. Genes that have a median correlation above, e.g., 0.30, 0.31, 0.32, 0.33, 0.34, 0.35, 0.36, 0.37, 0.38, 0.39, 0.40, or higher, can be used as biomarkers of sensitivity for assessing responsiveness of a cancer patient (e.g., a patient have recurrence of cancer) to APO010. Likewise, genes that have a median correlation below, e.g., -0.30, -0.31, -0.32, -0.33, -0.34, -0.35, -0.36, -0.37, -0.38, -0.39, -0.40, or lower, can be used as biomarkers of resistance for assessing responsiveness of a cancer patient (e.g., a patient have recurrence of cancer) to APO010. Preferably, the correlation coefficient of a biomarker of sensitivity will exceed 0.3, while the correlation coefficient of a biomarker of resistance will be less than -0.3. The result is a list of biomarker genes that correlate to sensitivity or resistance to APO010, as shown in Tables 1 and 2, respectively.

**Cancer Types**

The methods, devices, and kits of the invention can be used for diagnosing, prognosing, monitoring, treating, and/or reducing cancer in a subject suffering from, diagnosed with, or susceptible to cancer. Non-limiting examples of cancers that can be diagnosed, prognosed, monitored, treated, or reduced using the methods include hematological and solid tumors. In particular, cancers include, e.g., colorectal cancer (e.g., colon cancer and rectal cancer), leukemia (e.g., acute myeloid leukemia, acute lymphoid leukemia, chronic myeloid leukemia, chronic lymphocytic leukemia, acute myeloblastic leukemia, acute promyelocytic leukemia, acute myelomonocytic leukemia, acute monocytic leukemia, acute erythroleukemia, and chronic leukemia), myeloma (e.g., multiple myeloma), myelodysplastic syndrome, lymphoma (e.g., diffuse large B-cell lymphoma, cutaneous T-cell lymphoma, peripheral T-cell lymphoma, Hodgkin's lymphoma, non-Hodgkin's lymphoma, Waldenstrom's macroglobulinemia, and lymphocytic lymphoma), cervical cancer, prostate cancer, esophageal cancer, melanoma, glioma (e.g., oligodendroglioma), pancreatic cancer (e.g., adenosquamous carcinoma, signet ring cell carcinoma, hepatoid carcinoma, colloid carcinoma, islet cell carcinoma, and pancreatic neuroendocrine carcinoma), ovarian cancer (e.g., ovarian adenocarcinoma or embryonal carcinoma), gastrointestinal stromal tumor, sarcoma (e.g., fibrosarcoma, myxosarcoma, liposarcoma, chondrosarcoma, osteogenic sarcoma, angiosarcoma, endotheliosarcoma, lymphangiosarcoma, lymphangioendotheliosarcoma, leiomyosarcoma, Ewing's sarcoma, and rhabdomyosarcoma), breast cancer (e.g., medullary carcinoma), ER-positive cancer, bladder cancer, head and neck cancer (e.g., squamous cell carcinoma of the head and neck), lung cancer (e.g., non-small cell lung carcinoma, large cell carcinoma, bronchogenic carcinoma, and papillary adenocarcinoma), metastatic cancer, oral cavity cancer, uterine cancer, testicular cancer (e.g., seminoma and embryonal carcinoma), skin cancer (e.g., squamous cell carcinoma and basal cell carcinoma), thyroid cancer (e.g., papillary carcinoma and medullary carcinoma), brain cancer (e.g., astrocytoma and craniopharyngioma), stomach cancer, intra-epithelial cancer, bone cancer, biliary tract cancer, eye cancer, liver cancer (e.g., hepatocellular carcinoma or hepatoma), larynx cancer, kidney cancer (e.g., renal cell carcinoma and Wilms tumor), gastric cancer, blastoma (e.g., nephroblastoma, medulloblastoma, hemangioblastoma, neuroblastoma, and retinoblastoma), polycythemia vera, chordoma, synovioma, mesothelioma, adenocarcinoma, sweat gland carcinoma, sebaceous gland carcinoma, cystadenocarcinoma, bile duct carcinoma, choriocarcinoma, epithelial carcinoma, ependymoma, pinealoma, acoustic neuroma, schwannoma, meningioma, pituitary adenoma, nerve sheath tumor, cancer of the small intestine, cancer of the endocrine system, cancer of the penis, cancer of the urethra, cutaneous or intraocular melanoma, a gynecologic tumor, solid tumors of childhood, and neoplasms of the central nervous system.

In particular, the methods are useful for diagnosing, prognosing, monitoring, treating, or preventing, e.g., multiple myeloma, breast cancer, acute myelogenous leukemia (AML), acute lympho-blastic leukemia (ALL), chronic lymphocytic leukemia (CLL), myelodysplastic syndrome (MDS), chronic myelogenous leukemia - chronic phase (CMLCP), diffuse large B-cell lymphoma (DLBCL), cutaneous T-cell lymphoma (CTCL), peripheral T-cell lymphoma (PTCL), Hodgkin's lymphoma, hepatocellular carcinoma (HCC), cervical cancer, prostate cancer, renal cell carcinoma (RCC), esophageal cancer, melanoma, glioma, pancreatic cancer, ovarian cancer, gastrointestinal stromal tumors (GIST), sarcoma, estrogen receptor-positive (ERpos) breast cancer, non-small cell lung carcinoma (NSCLC), colon cancer, bladder cancer, or squamous cell carcinoma of the head and neck (SCCHN). For example, the cancer can be multiple myeloma, such as a Stage I, Stage II, or Stage III multiple myeloma. In particular, the cancer may be recurrent multiple myeloma. Alternatively, the cancer is a breast cancer, such as medullary carcinoma. The breast cancer can be, for example, a Stage 0, Stage I, Stage II, Stage III, or Stage IV breast cancer.

**Methods for Detecting Biomarker Gene Expression in Cancer Patients**

A cancer patient can be assessed for sensitivity or resistance to APO010 by detecting gene expression of a biomarker (e.g., one or more of the biomarkers of Tables 1 and 2) in a biological sample obtained from the cancer patient (e.g., a patient having recurrence of cancer). The biological sample can include, for example, cells, tissue (e.g., a tissue sample obtained by biopsy), blood, serum, plasma, urine, sputum, cerebrospinal fluid, lymph tissue or fluid, or pancreatic fluid. For example, the biological sample can be fresh frozen or formalin-fixed paraffin embedded (FFPE) tissue obtained from the subject, such as a tumor sample (e.g., a biopsy) from the tissue of interest (e.g., lymph nodes, thymus, spleen, bone marrow, breast, colorectal, pancreatic, cervical, prostate, bladder, lung, gastrointestinal, head, neck, or ovarian tissue).

*RNA Extraction and Biomarker Expression Measurement*

Cell samples or tissue samples may be snap frozen in liquid nitrogen until processing. RNA may be extracted using, e.g., Trizol Reagent from Invitrogen following manufacturer's instructions, and detected directly or converted to cDNA for detection. RNA may be amplified using, e.g., MessageAmp kit from Ambion following manufacturer's instructions. Amplified RNA may be quantified using, e.g., HG-U133A or HG-U133_Plus2 GeneChip from Affymetrix Inc. and compatible apparatus e.g. GCS3000Dx from Affymetrix, using the manufacturer's instructions. The resulting biomarker expression measurements may be further analyzed as described herein. The procedures described can be implemented using, e.g., R software available from R-Project and supplemented with packages available from Bioconductor.

One or more of the biomarkers shown in Tables 1 and 2 (e.g., CORO1A (SEQ ID NO: 2)) may be measured in a biological sample (e.g., a tumor sample) obtained from the cancer patient (e.g., a patient with any of the cancer types described herein, such as a patient with recurrence of cancer) using, e.g., polymerase chain reaction (PCR), reverse transcriptase PCR (RT-PCR), quantitative real-time PCR (qRT-PCR), an array (e.g., a microarray), a genechip, pyrosequencing, nanopore sequencing, sequencing by synthesis, sequencing by expansion, single molecule real time technology, sequencing by ligation, microfluidics, infrared fluorescence, next generation sequencing (e.g., RNA-Seq techniques), Northern blots, Western blots, Southern blots, NanoString nCounter technologies (e.g., those described in U.S. Patent Application Nos. US 2011/0201515, US 2011/0229888, and US 2013/0017971, each of which is incorporated by reference in its entirety), proteomic techniques (e.g., mass spectrometry or protein arrays), and combinations thereof.

*Devices*

Devices of the invention can be used for detecting the level of expression of one or more biomarkers shown in Tables 1 and 2. The device may include at least one single-stranded nucleic acid (e.g., a probe) having at least 85% sequence identity (e.g., 85%, 90%, 95%, 97%, 98%, 99%, or 100% sequence identity) to a nucleic acid sequence that is complementary or identical to at least 5 (e.g., at least 10, at least 15, at least 20, or more) consecutive nucleotides of one or more biomarkers shown in Tables 1 and 2 (e.g., CORO1A (SEQ ID NO: 2) or PDE8A (SEQ ID NO: 100)), in which the at least one single-stranded nucleic acid is sufficient for the detection of the expression level of the one or more biomarkers. The device may be used to detect the expression level of a given biomarker by specific hybridization between the single-stranded nucleic acid and the biomarker (e.g., an mRNA, genomic DNA, or non-coding RNA), a nucleic acid encoding the biomarker (e.g., an mRNA), or a complementary nucleic acid thereof. The device may be or include a microarray. The device may also include or be used with reagents and materials for next generation sequence (e.g., sequencing by synthesis). The device may also include or be used with NanoString reagents and at least one nCounter cartridge. The device may be or include a protein array, which contains one or more protein binding moieties (e.g., proteins, antibodies, nucleic acids, aptamers, affibodies, lipids, phospholipids, small molecules, labeled variants of any of the above, and any other moieties useful for protein detection as well known in the art) capable of detectably binding to the polypeptide product(s) of one or more biomarkers shown in Tables 1 and 2.

*Microarrays*

The expression levels of the biomarkers (e.g., the biomarkers listed in Tables 1 and 2 (e.g., CORO1A (SEQ ID NO: 2)) may be determined using high-throughput expression profiling platforms, such as microarrays. In particular, a microarray for use in the methods for assessing the responsiveness of a cancer patient (e.g., a patient with recurrence of cancer) to APO010 contains or is produced by generating oligonucleotide probes (e.g., DNA, cDNA, or RNA probes) capable of hybridizing to one or more biomarkers of interest (e.g., one or more of the biomarkers of Tables 1 and 2) or the complement sequences thereof. Each probe can have, e.g., at least 10, 15, 20, 25, 30, or more contiguous nucleic acid residues (e.g., at least 15) that are complementary or identical to a nucleic acid sequence of a selected biomarker. The probe nucleic sequence can also have at least 85% (e.g., 90%, 95%, 99%, or 100%) sequence identity to the nucleic acid sequence of the gene coding the biomarker (e.g., CORO1A (SEQ ID NO: 2) or the complement sequence thereof. In particular, the probe sequences can be complementary to all or a portion of the nucleic acid sequence of the biomarker(s).

For example, microarrays of the invention for determining APO010 responsiveness can include probes for one or more (e.g., at least 5, 10, 15, or 20 or more (e.g., all)) biomarkers of sensitivity shown in Table 1, such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), and/or ITGA4 (SEQ ID NO: 36). Microarrays of the invention for determining APO010 responsiveness can also include probes for one or more (e.g., at least 5, 10, 15, or 20 or more (e.g., all)) biomarkers of resistance listed in Table 2, such as PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129).

A microarray probe may be single-stranded or double-stranded. The probe may be labeled (e.g., detectably labeled with a fluorescent molecule, dye molecule, small molecule, epitope tag, barcode sequence, polypeptide, or any other detectable molecule). Probes can be detectably labeled and immobilized on a solid support to form the microarray.

For example, probes can be either prefabricated and spotted to the surface or directly synthesized on to the surface (*in situ*) of the microarray. The microarray can also be configured such that the sequence and position of each member (e.g., probe) of the array is known. For example, a selection of biomarkers whose expression correlates with an increased likelihood of responsiveness to APO010 can be arrayed on a solid support. Hybridization of a labeled probe with a particular target nucleic acid (e.g., an mRNA corresponding to one or more biomarkers of Tables 1 and 2) indicates that the sample from which the mRNA was derived expresses that biomarker (e.g., the biomarker of sensitivity or resistance to APO010).

*PCR-based techniques*

As few as one to 30 (e.g., 5 to 30 or 10 to 30, or at least the first 14 of the biomarkers listed in Tables 1-2) biomarkers may be used to determine patient responsiveness to APO010 using the methods described herein. Tissue or cell samples from a cancer patient (e.g., a patient having recurrence of cancer) can be conveniently assayed for gene expression levels using polymerase chain reaction (PCR) analysis, such as quantitative real-time PCR (qPCR), or quantitative loop-mediated isothermal amplification (q-LAMP). For example, an mRNA corresponding to a biomarker of Tables 1 and 2 can be detected in a biological sample by (a) producing cDNA from the sample by reverse transcription using at least one primer; (b) amplifying the cDNA so produced using a target polynucleotide as sense and antisense primers to amplify target cDNAs therein; and (c) detecting the presence of the amplified target cDNA using polynucleotide probes. The primers and probes including the target sequences shown in Tables 1 and 2, such as CORO1A (SEQ ID NO: 2) and/or PDE8A (SEQ ID NO: 100), may be used to detect expression of one or more of the indicated biomarkers using PCR. The methods can include one or more steps that allow determination of the levels of target mRNA in a biological sample (e.g., by simultaneously examining the levels of a comparative control mRNA sequence or "housekeeping" gene, such as an actin family member or GAPDH). The primers for these PCR-based assays may be labeled for detection according to methods known in the art.

*Sequencing*

The expression levels of the biomarkers shown in Tables 1 and 2, such as CORO1A (SEQ ID NO: 2) and/or PDE8A (SEQ ID NO: 100), may be determined using sequencing technologies, such as next generation sequencing platforms (e.g., RNA-Seq), as described in Mortazavi et al., Nat. Methods 5: 621-628, 2008, hereby incorporated by reference. RNA-Seq is a robust technology for monitoring expression by direct sequencing of the RNA molecules in a sample. This methodology may include fragmentation of RNA to an average length of, e.g., 200 nucleotides, conversion to cDNA by random priming, and synthesis of double-stranded cDNA (e.g., using the Just cDNA DoubleStranded cDNA Synthesis Kit from Agilent Technology). The cDNA may then be converted into a molecular library for sequencing by addition of sequence adapters for each library (e.g., from Illumina®/Solexa), and the resulting 50 to 100 nucleotide reads are mapped onto the genome. Exemplary sequencing platforms suitable for use according to the methods include, e.g., 454 pyrosequencing, Illumina sequencing by synthesis, SOLiD sequencing, Ion Torrent sequencing, and PacBio RS sequencing.

**Methods of Determining the Responsiveness of a Patient to APO010**

The invention features diagnostic methods for the detection and screening of cancer patients (e.g., patients having recurrence of cancer) that may be responsive to APO010 using one or more of the biomarkers shown in Tables 1 and/or 2 (e.g., CORO1A (SEQ ID NO: 2) or PDE8A (SEQ ID NO: 100)). The methods of the invention may be used for predicting a patient's responsiveness to APO010, and optionally, treating the cancer patient throughout the progression of cancer and/or in cases of recurrence (e.g., after a first line treatment, a second line treatment, and/or a third line treatment).

The invention provides individual biomarkers (e.g., CORO1A (SEQ ID NO: 2)) and sets of biomarkers (e.g., two or more of the biomarkers listed in Tables 1 and/or 2), the expression levels of which, as detected in a biological sample (e.g., a tumor sample, such as a biopsy) obtained from a cancer patient (e.g., a human with cancer), are indicative of responsiveness to APO010. The biomarkers were identified using methods similar to those previously described in, e.g., Chen et al. (Mol. Cancer Ther. 11:34-33, 2012), Wang et al. (J. Nat. Cancer Inst. 105: 1284-1291, 2013), and Knudsen et al. (PLoS One, 9: e87415, 2014), each of which are incorporated by reference herein in their entirety. In particular, an algorithm based on growth inhibition values (GI50) of a cell line (e.g., NCI60 cells) is subjected to treatment with APO010 and gene expression is determined (e.g., by microarray analysis, reverse transcriptase polymerase chain reaction (RT-PCR), quantitative real-time PCR (qPCR), or next generation sequencing). After normalization, genes with, e.g., a Pearson correlation coefficient greater than 0.3 or below -0.3 can be classified as biomarkers of sensitivity or resistance, respectively. In particular, a correlation coefficient of 0.3 or greater is a statistically significant cut-off known in the art for establishing whether the expression levels of, e.g., the genes shown in Tables 1 and 2, correlate with the likelihood of cancer treatment sensitivity, such as sensitivity to Apo010, as described in van't Veer et al. Nature 415(6871):530-536, 2002, hereby incorporated by reference.

*Comparison of Biomarker Expression Levels*

One or more biomarkers of sensitivity and/or resistance, identified as described herein, can be used to predict responsiveness to APO010 by measuring the expression level of the biomarkers in a biological sample obtained from the cancer patient. A single biomarker (e.g., any of the biomarkers of Tables 1 and/04 2, such as CORO1A (SEQ ID NO: 2)) may be used to determine the responsiveness of a cancer patient (e.g., a patient with cancer recurrence) to APO010. After determining the expression level of the biomarker(s) in a sample (e.g., a tumor sample) from the cancer patient, the expression level of the biomarker(s) in the sample may be compared to the expression level of the biomarker(s) in a cell (e.g., a cancer cell) or tissue (e.g., a tumor tissue) known to be sensitive to treatment with APO010. If the expression level of the biomarker(s) in the sample from the cancer patient is substantially similar (e.g., identical to or has the same trend of expression level) to the expression level of the biomarker(s) in the cell or tissue known to be sensitive to APO010, then the cancer patient is predicted to be responsive to treatment with APO010. Alternatively, if the expression level of the biomarker(s) in the sample from the cancer patient is substantially dissimilar to the expression level of the biomarker(s) in the cell or tissue known to be sensitive to APO010, then the cancer patient is predicted to be non-responsive to treatment with APO010.

The expression level of the biomarker (e.g., CORO1A (SEQ ID NO: 2)) in a sample from the cancer patient may also be compared to the expression level of the biomarker in a cell (e.g., a cancer cell) or tissue (e.g., a tumor tissue) known to be resistant to treatment with APO010. If the expression level of the biomarker in the sample from the cancer patient is substantially similar to the expression level of the biomarker in the cell or tissue known to be resistant to APO010, then the cancer patient is predicted to be non-responsive to treatment with APO010. Alternatively, if the expression level of the biomarker in the sample from the cancer patient is substantially dissimilar to the expression level of the biomarker in the cell or tissue known to be sensitive to APO010, then the cancer patient is predicted to be responsive to treatment with APO010.

The responsiveness of a cancer patient (e.g., a patient with cancer recurrence) to APO010 can also be predicted by comparing the expression level of a biomarker (e.g., CORO1A (SEQ ID NO: 2)) to the expression level of the biomarker in one or more cells or tissues (e.g., from a cancer patient population) known to be sensitive to treatment with APO010 and one or more cells or tissues (e.g., from a cancer patient population) known to be resistant to treatment with APO010. In particular, the patient may be responsive to treatment with APO010 if the expression level of the biomarker is substantially similar to the expression level of the biomarker in a cell or tissue known to be sensitive to treatment with APO010 than a cell or tissue known to be resistant to treatment with APO010. Alternatively, the patient may be non-responsive to treatment with APO010 if the expression level of the biomarker is substantially similar to the expression level of the biomarker in a cell or tissue known to be resistant to treatment with APO010 than a cell or tissue known to be sensitive to treatment with APO010.

Additionally, one or more biomarkers of sensitivity (e.g., one or more of CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), and ITGA4 (SEQ ID NO: 36)) and one or more biomarkers of resistance (e.g., one or more of PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), and ANXA2 (SEQ ID NO: 111 or 116 or 122)) may be used in combination to determine the responsiveness of a cancer patient (e.g., a patient with cancer recurrence) to treatment with APO010. For example, the predicted responsiveness of a cancer patient may be determined from, e.g., the difference score, which may be defined as the difference between the mean of the expression level of the one or more biomarkers of sensitivity of Table 1 and the mean of the expression level of the one or more biomarkers of resistance of Table 2.

The difference score of the cancer patient can then be compared to the difference score based on the expression level of the biomarkers in a cell (e.g., a cancer cell) or tissue (e.g., a tumor tissue) known to be sensitive or resistant to treatment with APO010. In particular, the patient may be responsive to treatment with APO010 if the difference score is substantially similar to the expression level of the biomarkers in a cell or tissue known to be sensitive to treatment with APO010. Alternatively, the patient may be non-responsive to treatment with APO010 if the difference score is substantially similar to the expression level of the biomarkers in a cell or tissue known to be resistant to treatment with APO010. Additionally, the patient may be responsive to treatment with APO010 if the difference score is substantially similar to the expression level of the biomarkers in a cell or tissue known to be sensitive to treatment with APO010 than a cell or tissue known to be resistant to treatment with APO010. Alternatively, the patient may be non-responsive to treatment with APO010 if the difference score is substantially similar to the expression level of the biomarkers in a cell or tissue known to be resistant to treatment with APO010 than a cell or tissue known to be sensitive to treatment with APO010.

One or more biomarkers of sensitivity and/or resistance, identified as described herein, can be used to predict responsiveness to APO010 by measuring the expression level of the biomarkers in a biological sample obtained from the cancer patient. A single biomarker (e.g., any of the biomarkers of Tables 1 and/04 2, such as CORO1A (SEQ ID NO: 2)) may be used to determine the responsiveness of a cancer patient (e.g., a patient with cancer recurrence) to APO010. After determining the expression level of the biomarker(s) in a sample (e.g., a tumor sample) from the cancer patient, the expression level of the biomarker(s) in the sample may be compared to the expression level of the biomarker(s) in a cell (e.g., a cancer cell) or tissue (e.g., a tumor tissue) known to be sensitive to treatment with APO010. If the expression level of the biomarker(s) in the sample from the cancer patient corresponds to (e.g., identical to or has the same trend of expression level) the expression level of the biomarker(s) in the cell or tissue known to be sensitive to APO010, then the cancer patient is predicted to be responsive to treatment with APO010. Alternatively, if the expression level of the biomarker(s) in the sample from the cancer patient is substantially dissimilar to the expression level of the biomarker(s) in the cell or tissue known to be sensitive to APO010, then the cancer patient is predicted to be non-responsive to treatment with APO010.

The expression level of the biomarker (e.g., CORO1A (SEQ ID NO: 2)) in a sample from the cancer patient may also be compared to the expression level of the biomarker in a cell (e.g., a cancer cell) or tissue (e.g., a tumor tissue) known to be resistant to treatment with APO010. If the expression level of the biomarker in the sample from the cancer patient corresponds to the expression level of the biomarker in the cell or tissue known to be resistant to APO010, then the cancer patient is predicted to be non-responsive to treatment with APO010. Alternatively, if the expression level of the biomarker in the sample from the cancer patient is substantially dissimilar to the expression level of the biomarker in the cell or tissue known to be sensitive to APO010, then the cancer patient is predicted to be responsive to treatment with APO010.

The responsiveness of a cancer patient (e.g., a patient with cancer recurrence) to APO010 can also be predicted by comparing the expression level of a biomarker (e.g., CORO1A (SEQ ID NO: 2)) to the expression level of the biomarker in one or more cells or tissues (e.g., from a cancer patient population) known to be sensitive to treatment with APO010 and one or more cells or tissues (e.g., from a cancer patient population) known to be resistant to treatment with APO010. In particular, the patient may be responsive to treatment with APO010 if the expression level of the biomarker corresponds to the expression level of the biomarker in a cell or tissue known to be sensitive to treatment with APO010 than a cell or tissue known to be resistant to treatment with APO010. Alternatively, the patient may be non-responsive to treatment with APO010 if the expression level of the biomarker corresponds to the expression level of the biomarker in a cell or tissue known to be resistant to treatment with APO010 than a cell or tissue known to be sensitive to treatment with APO010.

Additionally, one or more biomarkers of sensitivity (e.g., one or more of CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), and ITGA4 (SEQ ID NO: 36)) and one or more biomarkers of resistance (e.g., one or more of PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), and ANXA2 (SEQ ID NO: 111 or 116 or 122)) may be used in combination to determine the responsiveness of a cancer patient (e.g., a patient with cancer recurrence) to treatment with APO010. For example, the predicted responsiveness of a cancer patient may be determined from, e.g., the difference score, which may be defined as the difference between the mean of the expression level of the one or more biomarkers of sensitivity of Table 1 and the mean of the expression level of the one or more biomarkers of resistance of Table 2.

The difference score of the cancer patient can then be compared to the difference score based on the expression level of the biomarkers in a cell (e.g., a cancer cell) or tissue (e.g., a tumor tissue) known to be sensitive or resistant to treatment with APO010. In particular, the patient may be responsive to treatment with APO010 if the difference score corresponds to the expression level of the biomarkers in a cell or tissue known to be sensitive to treatment with APO010. Alternatively, the patient may be non-responsive to treatment with APO010 if the difference score corresponds to the expression level of the biomarkers in a cell or tissue known to be resistant to treatment with APO010. Additionally, the patient may be responsive to treatment with APO010 if the difference score corresponds to the expression level of the biomarkers in a cell or tissue known to be sensitive to treatment with APO010 than a cell or tissue known to be resistant to treatment with APO010. Alternatively, the patient may be non-responsive to treatment with APO010 if the difference score corresponds to the expression level of the biomarkers in a cell or tissue known to be resistant to treatment with APO010 than a cell or tissue known to be sensitive to treatment with APO010.

Preferably, the cell or tissue known to be either sensitive or resistant to APO010 is of the same cancer type as the cancer patient with an unknown responsiveness to APO010. For example, the cancer patient and the cell or tissue known to be either sensitive or resistant to APO010 may both have a cancer type selected from a hematological cancer or a solid tumor, such as, e.g., myeloma (e.g., multiple myeloma), colorectal cancer (e.g., colon cancer and rectal cancer), leukemia (e.g., acute myeloid leukemia, acute lymphoid leukemia, chronic myeloid leukemia, chronic lymphocytic leukemia, acute myeloblastic leukemia, acute promyelocytic leukemia, acute myelomonocytic leukemia, acute monocytic leukemia, acute erythroleukemia, and chronic leukemia), myelodysplastic syndrome, lymphoma (e.g., diffuse large B-cell lymphoma, cutaneous T-cell lymphoma, peripheral T-cell lymphoma, Hodgkin's lymphoma, non-Hodgkin's lymphoma, Waldenstrom's macroglobulinemia, and lymphocytic lymphoma), cervical cancer, prostate cancer, esophageal cancer, melanoma, glioma (e.g., oligodendroglioma), pancreatic cancer (e.g., adenosquamous carcinoma, signet ring cell carcinoma, hepatoid carcinoma, colloid carcinoma, islet cell carcinoma, and pancreatic neuroendocrine carcinoma), ovarian cancer (e.g., ovarian adenocarcinoma or embryonal carcinoma), gastrointestinal stromal tumor, sarcoma (e.g., fibrosarcoma, myxosarcoma, liposarcoma, chondrosarcoma, osteogenic sarcoma, angiosarcoma, endotheliosarcoma, lymphangiosarcoma, lymphangioendotheliosarcoma, leiomyosarcoma, Ewing's sarcoma, and rhabdomyosarcoma), breast cancer (e.g., medullary carcinoma), ER-positive cancer, bladder cancer, head and neck cancer (e.g., squamous cell carcinoma of the head and neck), lung cancer (e.g., non-small cell lung carcinoma, large cell carcinoma, bronchogenic carcinoma, and papillary adenocarcinoma), metastatic cancer, oral cavity cancer, uterine cancer, testicular cancer (e.g., seminoma and embryonal carcinoma), skin cancer (e.g., squamous cell carcinoma and basal cell carcinoma), thyroid cancer (e.g., papillary carcinoma and medullary carcinoma), brain cancer (e.g., astrocytoma and craniopharyngioma), stomach cancer, intra-epithelial cancer, bone cancer, biliary tract cancer, eye cancer, liver cancer (e.g., hepatocellular carcinoma or hepatoma), larynx cancer, kidney cancer (e.g., renal cell carcinoma and Wilms tumor), gastric cancer, blastoma (e.g., nephroblastoma, medulloblastoma, hemangioblastoma, neuroblastoma, and retinoblastoma), polycythemia vera, chordoma, synovioma, mesothelioma, adenocarcinoma, sweat gland carcinoma, sebaceous gland carcinoma, cystadenocarcinoma, bile duct carcinoma, choriocarcinoma, epithelial carcinoma, ependymoma, pinealoma, acoustic neuroma, schwannoma, meningioma, pituitary adenoma, nerve sheath tumor, cancer of the small intestine, cancer of the endocrine system, cancer of the penis, cancer of the urethra, cutaneous or intraocular melanoma, a gynecologic tumor, solid tumors of childhood, and neoplasms of the central nervous system. In particular, the cancer of the patient and the cell or tissue with known resistance or sensitivity to Apo010 is, e.g., multiple myeloma, breast cancer, acute myelogenous leukemia (AML), acute lympho-blastic leukemia (ALL), chronic lymphocytic leukemia (CLL), myelodysplastic syndrome (MDS), chronic myelogenous leukemia - chronic phase (CMLCP), diffuse large B-cell lymphoma (DLBCL), cutaneous T-cell lymphoma (CTCL), peripheral T-cell lymphoma (PTCL), Hodgkin's lymphoma, hepatocellular carcinoma (HCC), cervical cancer, prostate cancer, renal cell carcinoma (RCC), esophageal cancer, melanoma, glioma, pancreatic cancer, ovarian cancer, gastrointestinal stromal tumors (GIST), sarcoma, estrogen receptor-positive (ERpos) breast cancer, non-small cell lung carcinoma (NSCLC), colon cancer, bladder cancer, or squamous cell carcinoma of the head and neck (SCCHN).

Machine learning techniques such as Neural Networks, Support Vector Machines, K Nearest Neighbor, and Nearest Centroids may also be employed to develop models that discriminate patients sensitive to treatment with APO010 from those resistant to treatment with APO010 using biomarker expression as model variables which assign each patient a classification as sensitive or resistant to treatment with APO010. Machine learning techniques used to classify patients using various measurements are described in U.S. Patent No. 5,822,715; U.S. Patent Application Publication Nos. 2003/0073083, 2005/0227266, 2005/0208512, 2005/0123945, 2003/0129629, and 2002/0006613; and in Vapnik V N. Statistical Learning Theory, John Wiley & Sons, New York, 1998; Hastie et al., 2001, The Elements of Statistical Learning: Data Mining, Inference, and Prediction, Springer, N.Y.; Agresti, 1996, An Introduction to Categorical Data Analysis, John Wiley & Sons, New York; V. Tresp et al., "Neural Network Modeling of Physiological Processes," in Hanson S. J. et al. (Eds.), Computational Learning Theory and Natural Learning Systems 2, MIT Press, 1994, each of which are hereby incorporated by reference in their entirety.

*Biomarkers of Sensitivity and Resistance*

The expression levels of one or more biomarkers of Tables 1 and/or 2 can be used to determine cancer patient responsiveness to treatment with APO010. Once determined to be sensitive, the patient can be treated with APO010.

In particular, the biomarker CORO1A (SEQ ID NO: 2) may be used to assess a cancer patient's (e.g, a patient with cancer recurrence) responsiveness to APO010. The expression level of the biomarker CORO1A (SEQ ID NO: 2) may be assessed using nucleic acid amplification methods (e.g., PCR) or a device (e.g., a microarray). As is described above, the expression level of CORO1A (SEQ ID NO: 2) in the patient sample may then be compared, e.g., to the expression level of CORO1A (SEQ ID NO: 2) in a cell (e.g., a cancer cell) or tissue (e.g., a tumor tissue) known to be sensitive or resistant to treatment with APO010 and used to determine the cancer patient's responsiveness to APO010. The biomarker CORO1A (SEQ ID NO: 2) may be used alone to predict cancer patient responsiveness to treatment with APO010 or in combination with one or more additional biomarkers (e.g., one, two, three, four, five, ten, or all of the biomarkers shown in Tables 1 and 2), such as CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker CD47 (SEQ ID NO: 1) may be used to assess a cancer patient's (e.g, a patient with cancer recurrence) responsiveness to APO010. The expression level of the biomarker CD47 (SEQ ID NO: 1) may be assessed using nucleic acid amplification methods (e.g., PCR) or a device (e.g., a microarray). As is described above, the expression level of CD47 (SEQ ID NO: 1) in the patient sample may then be compared, e.g., to the expression level of CD47 (SEQ ID NO: 1) in a cell (e.g., a cancer cell) or tissue (e.g., a tumor tissue) known to be sensitive or resistant to treatment with APO010 and used to determine the cancer patient's responsiveness to APO010. The biomarker CD47 (SEQ ID NO: 1) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker ICAM3 (SEQ ID NO: 3) may be used to assess a cancer patient's (e.g, a patient with cancer recurrence) responsiveness to APO010. The expression level of the biomarker ICAM3 (SEQ ID NO: 3) may be assessed using nucleic acid amplification methods (e.g., PCR) or a device (e.g., a microarray). As is described above, the expression level of ICAM3 (SEQ ID NO: 3) in the patient sample may then be compared, e.g., to the expression level of ICAM3 (SEQ ID NO: 3) in a cell (e.g., a cancer cell) or tissue (e.g., a tumor tissue) known to be sensitive or resistant to treatment with APO010 and used to determine the cancer patient's responsiveness to APO010. The biomarker ICAM3 (SEQ ID NO: 3) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker HCLS1 (SEQ ID NO: 4) may be used to assess a cancer patient's (e.g, a patient with cancer recurrence) responsiveness to APO010. The expression level of the biomarker HCLS1 (SEQ ID NO: 4) may be assessed using nucleic acid amplification methods (e.g., PCR) or a device (e.g., a microarray). As is described above, the expression level of HCLS1 (SEQ ID NO: 4) in the patient sample may then be compared, e.g., to the expression level of HCLS1 (SEQ ID NO: 4) in a cell (e.g., a cancer cell) or tissue (e.g., a tumor tissue) known to be sensitive or resistant to treatment with APO010 and used to determine the cancer patient's responsiveness to APO010. The biomarker HCLS1 (SEQ ID NO: 4) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker DOCK2 (SEQ ID NO 5) may be used to assess a cancer patient's (e.g, a patient with cancer recurrence) responsiveness to APO010. The expression level of the biomarker DOCK2 (SEQ ID NO 5), may be assessed using nucleic acid amplification methods (e.g., PCR) or a device (e.g., a microarray). As is described above, the expression level of DOCK2 (SEQ ID NO 5) in the patient sample may then be compared, e.g., to the expression level of DOCK2 (SEQ ID NO 5) in a cell (e.g., a cancer cell) or tissue (e.g., a tumor tissue) known to be sensitive or resistant to treatment with APO010 and used to determine the cancer patient's responsiveness to APO010. As is described above, the expression level of DOCK2 (SEQ ID NO 5) may then be compared, e.g., to a cell (e.g., a cancer cell) or tissue (e.g., a tumor tissue) known to be sensitive or resistant to treatment with APO010 and used to determine the cancer patient's responsiveness to APO010. The biomarker DOCK2 (SEQ ID NO 5) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker ARHGAP15 (SEQ ID NO: 6) may be used to assess a cancer patient's (e.g, a patient with cancer recurrence) responsiveness to APO010. The expression level of the biomarker ARHGAP15 (SEQ ID NO: 6) may be assessed using nucleic acid amplification methods (e.g., PCR) or a device (e.g., a microarray). As is described above, the expression level of ARHGAP15 (SEQ ID NO: 6) in the patient sample may then be compared, e.g., to the expression level of ARHGAP15 (SEQ ID NO: 6) in a cell (e.g., a cancer cell) or tissue (e.g., a tumor tissue) known to be sensitive or resistant to treatment with APO010 and used to determine the cancer patient's responsiveness to APO010. The biomarker ARHGAP15 (SEQ ID NO: 6) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker CXCR4 (SEQ ID NO: 7 or 9 or 16) may be used to assess a cancer patient's (e.g, a patient with cancer recurrence) responsiveness to APO010. The expression level of the biomarker CXCR4 (SEQ ID NO: 7 or 9 or 16) may be assessed using nucleic acid amplification methods (e.g., PCR) or a device (e.g., a microarray). As is described above, the expression level of CXCR4 (SEQ ID NO: 7 or 9 or 16) in the patient sample may then be compared, e.g., to the expression level of CXCR4 (SEQ ID NO: 7 or 9 or 16) in a cell (e.g., a cancer cell) or tissue (e.g., a tumor tissue) known to be sensitive or resistant to treatment with APO010 and used to determine the cancer patient's responsiveness to APO010. The biomarker CXCR4 (SEQ ID NO: 7 or 9 or 16) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker NCKAP1L (SEQ ID NO: 8) may be used to assess a cancer patient's (e.g, a patient with cancer recurrence) responsiveness to APO010. The expression level of the biomarker NCKAP1L (SEQ ID NO: 8) may be assessed using nucleic acid amplification methods (e.g., PCR) or a device (e.g., a microarray). As is described above, the expression level of NCKAP1L (SEQ ID NO: 8) in the patient sample may then be compared, e.g., to the expression level of NCKAP1L (SEQ ID NO: 8) in a cell (e.g., a cancer cell) or tissue (e.g., a tumor tissue) known to be sensitive or resistant to treatment with APO010 and used to determine the cancer patient's responsiveness to APO010. The biomarker NCKAP1L (SEQ ID NO: 8) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker MAP4K1 (SEQ ID NO: 10 or 17 or 19) may be used to assess a cancer patient's (e.g, a patient with cancer recurrence) responsiveness to APO010. The expression level of the biomarker MAP4K1 (SEQ ID NO: 10 or 17 or 19) may be assessed using nucleic acid amplification methods (e.g., PCR) or a device (e.g., a microarray). As is described above, the expression level of MAP4K1 (SEQ ID NO: 10 or 17 or 19) in the patient sample may then be compared, e.g., to the expression level of MAP4K1 (SEQ ID NO: 10 or 17 or 19) in a cell (e.g., a cancer cell) or tissue (e.g., a tumor tissue) known to be sensitive or resistant to treatment with APO010 and used to determine the cancer patient's responsiveness to APO010. The biomarker MAP4K1 (SEQ ID NO: 10 or 17 or 19) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker SLA (SEQ ID NO: 11) may be used to assess a cancer patient's (e.g, a patient with cancer recurrence) responsiveness to APO010. The expression level of the biomarker SLA (SEQ ID NO: 11) may be assessed using nucleic acid amplification methods (e.g., PCR) or a device (e.g., a microarray). As is described above, the expression level of SLA (SEQ ID NO: 11) in the patient sample may then be compared, e.g., to the expression level of SLA (SEQ ID NO: 11) in a cell (e.g., a cancer cell) or tissue (e.g., a tumor tissue) known to be sensitive or resistant to treatment with APO010 and used to determine the cancer patient's responsiveness to APO010. The biomarker SLA (SEQ ID NO: 11) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker DENND2D (SEQ ID NO: 12) may be used to assess a cancer patient's (e.g, a patient with cancer recurrence) responsiveness to APO010. The expression level of the biomarker DENND2D (SEQ ID NO: 12) may be assessed using nucleic acid amplification methods (e.g., PCR) or a device (e.g., a microarray). As is described above, the expression level of DENND2D (SEQ ID NO: 12) in the patient sample may then be compared, e.g., to the expression level of DENND2D (SEQ ID NO: 12) in a cell (e.g., a cancer cell) or tissue (e.g., a tumor tissue) known to be sensitive or resistant to treatment with APO010 and used to determine the cancer patient's responsiveness to APO010. The biomarker DENND2D (SEQ ID NO: 12) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker CYFIP2 (SEQ ID NO: 13 or 98) may be used to assess a cancer patient's (e.g, a patient with cancer recurrence) responsiveness to APO010. The expression level of the biomarker CYFIP2 (SEQ ID NO: 13 or 98) may be assessed using nucleic acid amplification methods (e.g., PCR) or a device (e.g., a microarray). As is described above, the expression level of CYFIP2 (SEQ ID NO: 13 or 98) in the patient sample may then be compared, e.g., to the expression level of CYFIP2 (SEQ ID NO: 13 or 98) in a cell (e.g., a cancer cell) or tissue (e.g., a tumor tissue) known to be sensitive or resistant to treatment with APO010 and used to determine the cancer patient's responsiveness to APO010. The biomarker CYFIP2 (SEQ ID NO: 13 or 98) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker PSME2 (SEQ ID NO: 14) may be used to assess a cancer patient's (e.g, a patient with cancer recurrence) responsiveness to APO010. The expression level of the biomarker PSME2 (SEQ ID NO: 14) may be assessed using nucleic acid amplification methods (e.g., PCR) or a device (e.g., a microarray). As is described above, the expression level of PSME2 (SEQ ID NO: 14) in the patient sample may then be compared, e.g., to the expression level of PSME2 (SEQ ID NO: 14) in a cell (e.g., a cancer cell) or tissue (e.g., a tumor tissue) known to be sensitive or resistant to treatment with APO010 and used to determine the cancer patient's responsiveness to APO010. The biomarker PSME2 (SEQ ID NO: 14) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker PTPRCAP (SEQ ID NO: 15) may be used to assess a cancer patient's (e.g, a patient with cancer recurrence) responsiveness to APO010. The expression level of the biomarker PTPRCAP (SEQ ID NO: 15) may be assessed using nucleic acid amplification methods (e.g., PCR) or a device (e.g., a microarray). As is described above, the expression level of PTPRCAP (SEQ ID NO: 15) in the patient sample may then be compared, e.g., to the expression level of PTPRCAP (SEQ ID NO: 15) in a cell (e.g., a cancer cell) or tissue (e.g., a tumor tissue) known to be sensitive or resistant to treatment with APO010 and used to determine the cancer patient's responsiveness to APO010. The biomarker PTPRCAP (SEQ ID NO: 15) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker UBE2L6 (SEQ ID NO: 18) may be used to assess a cancer patient's (e.g, a patient with cancer recurrence) responsiveness to APO010. The expression level of the biomarker UBE2L6 (SEQ ID NO: 18) may be assessed using nucleic acid amplification methods (e.g., PCR) or a device (e.g., a microarray). As is described above, the expression level of UBE2L6 (SEQ ID NO: 18) in the patient sample may then be compared, e.g., to the expression level of UBE2L6 (SEQ ID NO: 18) in a cell (e.g., a cancer cell) or tissue (e.g., a tumor tissue) known to be sensitive or resistant to treatment with APO010 and used to determine the cancer patient's responsiveness to APO010. The biomarker UBE2L6 (SEQ ID NO: 18) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker AIF1 (SEQ ID NO: 20) may be used to assess a cancer patient's (e.g, a patient with cancer recurrence) responsiveness to APO010. The expression level of the biomarker AIF1 (SEQ ID NO: 20) may be assessed using nucleic acid amplification methods (e.g., PCR) or a device (e.g., a microarray). As is described above, the expression level of AIF1 (SEQ ID NO: 20) in the patient sample may then be compared, e.g., to the expression level of AIF1 (SEQ ID NO: 20) in a cell (e.g., a cancer cell) or tissue (e.g., a tumor tissue) known to be sensitive or resistant to treatment with APO010 and used to determine the cancer patient's responsiveness to APO010. The biomarker AIF1 (SEQ ID NO: 20) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker PSMB9 (SEQ ID NO: 21) may be used to assess a cancer patient's (e.g, a patient with cancer recurrence) responsiveness to APO010. The expression level of the biomarker PSMB9 (SEQ ID NO: 21) may be assessed using nucleic acid amplification methods (e.g., PCR) or a device (e.g., a microarray). As is described above, the expression level of PSMB9 (SEQ ID NO: 21) in the patient sample may then be compared, e.g., to the expression level of PSMB9 (SEQ ID NO: 21) in a cell (e.g., a cancer cell) or tissue (e.g., a tumor tissue) known to be sensitive or resistant to treatment with APO010 and used to determine the cancer patient's responsiveness to APO010. The biomarker PSMB9 (SEQ ID NO: 21) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker TRIM22 (SEQ ID NO: 22) may be used to assess a cancer patient's (e.g, a patient with cancer recurrence) responsiveness to APO010. The expression level of the biomarker TRIM22 (SEQ ID NO: 22) may be assessed using nucleic acid amplification methods (e.g., PCR) or a device (e.g., a microarray). As is described above, the expression level of TRIM22 (SEQ ID NO: 22) in the patient sample may then be compared, e.g., to the expression level of TRIM22 (SEQ ID NO: 22) in a cell (e.g., a cancer cell) or tissue (e.g., a tumor tissue) known to be sensitive or resistant to treatment with APO010 and used to determine the cancer patient's responsiveness to APO010. The biomarker TRIM22 (SEQ ID NO: 22) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker BIN2 (SEQ ID NO: 23) may be used to assess a cancer patient's (e.g, a patient with cancer recurrence) responsiveness to APO010. The expression level of the biomarker BIN2 (SEQ ID NO: 23) may be assessed using nucleic acid amplification methods (e.g., PCR) or a device (e.g., a microarray). As is described above, the expression level of BIN2 (SEQ ID NO: 23) in the patient sample may then be compared, e.g., to the expression level of BIN2 (SEQ ID NO: 23) in a cell (e.g., a cancer cell) or tissue (e.g., a tumor tissue) known to be sensitive or resistant to treatment with APO010 and used to determine the cancer patient's responsiveness to APO010. The biomarker BIN2 (SEQ ID NO: 23) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker CD247 (SEQ ID NO: 24) may be used to assess a cancer patient's (e.g, a patient with cancer recurrence) responsiveness to APO010. The expression level of the biomarker CD247 (SEQ ID NO: 24) may be assessed using nucleic acid amplification methods (e.g., PCR) or a device (e.g., a microarray). As is described above, the expression level of CD247 (SEQ ID NO: 24) in the patient sample may then be compared, e.g., to the expression level of CD247 (SEQ ID NO: 24) in a cell (e.g., a cancer cell) or tissue (e.g., a tumor tissue) known to be sensitive or resistant to treatment with APO010 and used to determine the cancer patient's responsiveness to APO010. The biomarker CD247 (SEQ ID NO: 24) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker APOL3 (SEQ ID NO: 25) may be used to assess a cancer patient's (e.g, a patient with cancer recurrence) responsiveness to APO010. The expression level of the biomarker APOL3 (SEQ ID NO: 25) may be assessed using nucleic acid amplification methods (e.g., PCR) or a device (e.g., a microarray). As is described above, the expression level of APOL3 (SEQ ID NO: 25) in the patient sample may then be compared, e.g., to the expression level of APOL3 (SEQ ID NO: 25) in a cell (e.g., a cancer cell) or tissue (e.g., a tumor tissue) known to be sensitive or resistant to treatment with APO010 and used to determine the cancer patient's responsiveness to APO010. The biomarker APOL3 (SEQ ID NO: 25) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker PTPRC (SEQ ID NO: 26) may be used to assess a cancer patient's (e.g, a patient with cancer recurrence) responsiveness to APO010. The expression level of the biomarker PTPRC (SEQ ID NO: 26) may be assessed using nucleic acid amplification methods (e.g., PCR) or a device (e.g., a microarray). As is described above, the expression level of PTPRC (SEQ ID NO: 26) in the patient sample may then be compared, e.g., to the expression level of PTPRC (SEQ ID NO: 26) in a cell (e.g., a cancer cell) or tissue (e.g., a tumor tissue) known to be sensitive or resistant to treatment with APO010 and used to determine the cancer patient's responsiveness to APO010. The biomarker PTPRC (SEQ ID NO: 26) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker CD53 (SEQ ID NO: 27) may be used to assess a cancer patient's (e.g, a patient with cancer recurrence) responsiveness to APO010. The expression level of the biomarker CD53 (SEQ ID NO: 27) may be assessed using nucleic acid amplification methods (e.g., PCR) or a device (e.g., a microarray). As is described above, the expression level of CD53 (SEQ ID NO: 27) in the patient sample may then be compared, e.g., to the expression level of CD53 (SEQ ID NO: 27) in a cell (e.g., a cancer cell) or tissue (e.g., a tumor tissue) known to be sensitive or resistant to treatment with APO010 and used to determine the cancer patient's responsiveness to APO010. The biomarker CD53 (SEQ ID NO: 27) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker SELPLG (SEQ ID NO: 28) may be used to assess a cancer patient's (e.g, a patient with cancer recurrence) responsiveness to APO010. The expression level of the biomarker SELPLG (SEQ ID NO: 28) may be assessed using nucleic acid amplification methods (e.g., PCR) or a device (e.g., a microarray). As is described above, the expression level of SELPLG (SEQ ID NO: 28) in the patient sample may then be compared, e.g., to the expression level of SELPLG (SEQ ID NO: 28) in a cell (e.g., a cancer cell) or tissue (e.g., a tumor tissue) known to be sensitive or resistant to treatment with APO010 and used to determine the cancer patient's responsiveness to APO010. The biomarker SELPLG (SEQ ID NO: 28) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker LAIR1 (SEQ ID NO: 29) may be used to assess a cancer patient's (e.g, a patient with cancer recurrence) responsiveness to APO010. The expression level of the biomarker LAIR1 (SEQ ID NO: 29) may be assessed using nucleic acid amplification methods (e.g., PCR) or a device (e.g., a microarray). As is described above, the expression level of LAIR1 (SEQ ID NO: 29) in the patient sample may then be compared, e.g., to the expression level of LAIR1 (SEQ ID NO: 29) in a cell (e.g., a cancer cell) or tissue (e.g., a tumor tissue) known to be sensitive or resistant to treatment with APO010 and used to determine the cancer patient's responsiveness to APO010. The biomarker LAIR1 (SEQ ID NO: 29) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker PSME1 (SEQ ID NO: 30) may be used to assess a cancer patient's (e.g, a patient with cancer recurrence) responsiveness to APO010. The expression level of the biomarker PSME1 (SEQ ID NO: 30) may be assessed using nucleic acid amplification methods (e.g., PCR) or a device (e.g., a microarray). As is described above, the expression level of PSME1 (SEQ ID NO: 30) in the patient sample may then be compared, e.g., to the expression level of PSME1 (SEQ ID NO: 30) in a cell (e.g., a cancer cell) or tissue (e.g., a tumor tissue) known to be sensitive or resistant to treatment with APO010 and used to determine the cancer patient's responsiveness to APO010. The biomarker PSME1 (SEQ ID NO: 30) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker MYC (SEQ ID NO: 31) may be used to assess a cancer patient's (e.g, a patient with cancer recurrence) responsiveness to APO010. The expression level of the biomarker MYC (SEQ ID NO: 31) may be assessed using nucleic acid amplification methods (e.g., PCR) or a device (e.g., a microarray). As is described above, the expression level of MYC (SEQ ID NO: 31) in the patient sample may then be compared, e.g., to the expression level of MYC (SEQ ID NO: 31) in a cell (e.g., a cancer cell) or tissue (e.g., a tumor tissue) known to be sensitive or resistant to treatment with APO010 and used to determine the cancer patient's responsiveness to APO010. The biomarker MYC (SEQ ID NO: 31) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker LCP1 (SEQ ID NO: 32) may be used to assess a cancer patient's (e.g, a patient with cancer recurrence) responsiveness to APO010. The expression level of the biomarker LCP1 (SEQ ID NO: 32) may be assessed using nucleic acid amplification methods (e.g., PCR) or a device (e.g., a microarray). As is described above, the expression level of LCP1 (SEQ ID NO: 32) in the patient sample may then be compared, e.g., to the expression level of LCP1 (SEQ ID NO: 32) in a cell (e.g., a cancer cell) or tissue (e.g., a tumor tissue) known to be sensitive or resistant to treatment with APO010 and used to determine the cancer patient's responsiveness to APO010. The biomarker LCP1 (SEQ ID NO: 32) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker ZAP70 (SEQ ID NO: 33) may be used to assess a cancer patient's (e.g, a patient with cancer recurrence) responsiveness to APO010. The expression level of the biomarker ZAP70 (SEQ ID NO: 33) may be assessed using nucleic acid amplification methods (e.g., PCR) or a device (e.g., a microarray). As is described above, the expression level of ZAP70 (SEQ ID NO: 33) in the patient sample may then be compared, e.g., to the expression level of ZAP70 (SEQ ID NO: 33) in a cell (e.g., a cancer cell) or tissue (e.g., a tumor tissue) known to be sensitive or resistant to treatment with APO010 and used to determine the cancer patient's responsiveness to APO010. The biomarker ZAP70 (SEQ ID NO: 33) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker SMARCA4 (SEQ ID NO: 34) may be used to assess a cancer patient's (e.g, a patient with cancer recurrence) responsiveness to APO010. The expression level of the biomarker SMARCA4 (SEQ ID NO: 34) may be assessed using nucleic acid amplification methods (e.g., PCR) or a device (e.g., a microarray). As is described above, the expression level of SMARCA4 (SEQ ID NO: 34) in the patient sample may then be compared, e.g., to the expression level of SMARCA4 (SEQ ID NO: 34) in a cell (e.g., a cancer cell) or tissue (e.g., a tumor tissue) known to be sensitive or resistant to treatment with APO010 and used to determine the cancer patient's responsiveness to APO010. The biomarker SMARCA4 (SEQ ID NO: 34) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker PTPN7 (SEQ ID NO: 35) may be used to assess a cancer patient's (e.g, a patient with cancer recurrence) responsiveness to APO010. The expression level of the biomarker PTPN7 (SEQ ID NO: 35) may be assessed using nucleic acid amplification methods (e.g., PCR) or a device (e.g., a microarray). As is described above, the expression level of PTPN7 (SEQ ID NO: 35) in the patient sample may then be compared, e.g., to the expression level of PTPN7 (SEQ ID NO: 35) in a cell (e.g., a cancer cell) or tissue (e.g., a tumor tissue) known to be sensitive or resistant to treatment with APO010 and used to determine the cancer patient's responsiveness to APO010. The biomarker PTPN7 (SEQ ID NO: 35) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker ITGA4 (SEQ ID NO: 36) may be used to assess a cancer patient's (e.g, a patient with cancer recurrence) responsiveness to APO010. The expression level of the biomarker ITGA4 (SEQ ID NO: 36) may be assessed using nucleic acid amplification methods (e.g., PCR) or a device (e.g., a microarray). As is described above, the expression level of ITGA4 (SEQ ID NO: 36) in the patient sample may then be compared, e.g., to the expression level of ITGA4 (SEQ ID NO: 36) in a cell (e.g., a cancer cell) or tissue (e.g., a tumor tissue) known to be sensitive or resistant to treatment with APO010 and used to determine the cancer patient's responsiveness to APO010. The biomarker ITGA4 (SEQ ID NO: 36) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker PDE8A (SEQ ID NO: 100) may be used to assess a cancer patient's (e.g, a patient with cancer recurrence) responsiveness to APO010. The expression level of the biomarker PDE8A (SEQ ID NO: 100) may be assessed using nucleic acid amplification methods (e.g., PCR) or a device (e.g., a microarray). As is described above, the expression level of PDE8A (SEQ ID NO: 100) in the patient sample may then be compared, e.g., to the expression level of PDE8A (SEQ ID NO: 100) in a cell (e.g., a cancer cell) or tissue (e.g., a tumor tissue) known to be sensitive or resistant to treatment with APO010 and used to determine the cancer patient's responsiveness to APO010. The biomarker PDE8A (SEQ ID NO: 100) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker HOXB7 (SEQ ID NO: 101 or 103) may be used to assess a cancer patient's (e.g, a patient with cancer recurrence) responsiveness to APO010. The expression level of the biomarker HOXB7 (SEQ ID NO: 101 or 103) may be assessed using nucleic acid amplification methods (e.g., PCR) or a device (e.g., a microarray). As is described above, the expression level of HOXB7 (SEQ ID NO: 101 or 103) in the patient sample may then be compared, e.g., to the expression level of HOXB7 (SEQ ID NO: 101 or 103) in a cell (e.g., a cancer cell) or tissue (e.g., a tumor tissue) known to be sensitive or resistant to treatment with APO010 and used to determine the cancer patient's responsiveness to APO010. The biomarker HOXB7 (SEQ ID NO: 101 or 103) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO:

111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker PRC1 (SEQ ID NO: 102) may be used to assess a cancer patient's (e.g, a patient with cancer recurrence) responsiveness to APO010. The expression level of the biomarker PRC1 (SEQ ID NO: 102) may be assessed using nucleic acid amplification methods (e.g., PCR) or a device (e.g., a microarray). As is described above, the expression level of PRC1 (SEQ ID NO: 102) in the patient sample may then be compared, e.g., to the expression level of PRC1 (SEQ ID NO: 102) in a cell (e.g., a cancer cell) or tissue (e.g., a tumor tissue) known to be sensitive or resistant to treatment with APO010 and used to determine the cancer patient's responsiveness to APO010. The biomarker PRC1 (SEQ ID NO: 102) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker SCRN1 (SEQ ID NO: 104) may be used to assess a cancer patient's (e.g, a patient with cancer recurrence) responsiveness to APO010. The expression level of the biomarker SCRN1 (SEQ ID NO: 104) may be assessed using nucleic acid amplification methods (e.g., PCR) or a device (e.g., a microarray). As is described above, the expression level of SCRN1 (SEQ ID NO: 104) in the patient sample may then be compared, e.g., to the expression level of SCRN1 (SEQ ID NO: 104) in a cell (e.g., a cancer cell) or tissue (e.g., a tumor tissue) known to be sensitive or resistant to treatment with APO010 and used to determine the cancer patient's responsiveness to APO010. The biomarker SCRN1 (SEQ ID NO: 104) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker ACTN1 (SEQ ID NO: 105 or 109) may be used to assess a cancer patient's (e.g, a patient with cancer recurrence) responsiveness to APO010. The expression level of the biomarker ACTN1 (SEQ ID NO: 105 or 109) may be assessed using nucleic acid amplification methods (e.g., PCR) or a device (e.g., a microarray). As is described above, the expression level of ACTN1 (SEQ ID NO: 105 or 109) in the patient sample may then be compared, e.g., to the expression level of ACTN1 (SEQ ID NO: 105 or 109) in a cell (e.g., a cancer cell) or tissue (e.g., a tumor tissue) known to be sensitive or resistant to treatment with APO010 and used to determine the cancer patient's responsiveness to APO010. The biomarker ACTN1 (SEQ ID NO: 105 or 109) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker NGRN (SEQ ID NO: 106) may be used to assess a cancer patient's (e.g, a patient with cancer recurrence) responsiveness to APO010. The expression level of the biomarker NGRN (SEQ ID NO: 106) may be assessed using nucleic acid amplification methods (e.g., PCR) or a device (e.g., a microarray). As is described above, the expression level of NGRN (SEQ ID NO: 106) in the patient sample may then be compared, e.g., to the expression level of NGRN (SEQ ID NO: 106) in a cell (e.g., a cancer cell) or tissue (e.g., a tumor tissue) known to be sensitive or resistant to treatment with APO010 and used to determine the cancer patient's responsiveness to APO010. The biomarker NGRN (SEQ ID NO: 106) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker DKK1 (SEQ ID NO: 107) may be used to assess a cancer patient's (e.g, a patient with cancer recurrence) responsiveness to APO010. The expression level of the biomarker DKK1 (SEQ ID NO: 107) may be assessed using nucleic acid amplification methods (e.g., PCR) or a device (e.g., a microarray). As is described above, the expression level of DKK1 (SEQ ID NO: 107) in the patient sample may then be compared, e.g., to the expression level of DKK1 (SEQ ID NO: 107) in a cell (e.g., a cancer cell) or tissue (e.g., a tumor tissue) known to be sensitive or resistant to treatment with APO010 and used to determine the cancer patient's responsiveness to APO010. The biomarker DKK1 (SEQ ID NO: 107) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker IER3 (SEQ ID NO: 108) may be used to assess a cancer patient's (e.g, a patient with cancer recurrence) responsiveness to APO010. The expression level of the biomarker IER3 (SEQ ID NO: 108) may be assessed using nucleic acid amplification methods (e.g., PCR) or a device (e.g., a microarray). As is described above, the expression level of IER3 (SEQ ID NO: 108) in the patient sample may then be compared, e.g., to the expression level of IER3 (SEQ ID NO: 108) in a cell (e.g., a cancer cell) or tissue (e.g., a tumor tissue) known to be sensitive or resistant to treatment with APO010 and used to determine the cancer patient's responsiveness to APO010. The biomarker IER3 (SEQ ID NO: 108) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker NQO1 (SEQ ID NO: 110 or 121 or 127) may be used to assess a cancer patient's (e.g, a patient with cancer recurrence) responsiveness to APO010. The expression level of the biomarker NQO1 (SEQ ID NO: 110 or 121 or 127) may be assessed using nucleic acid amplification methods (e.g., PCR) or a device (e.g., a microarray). As is described above, the expression level of NQO1 (SEQ ID NO: 110 or 121 or 127) in the patient sample may then be compared, e.g., to the expression level of NQO1 (SEQ ID NO: 110 or 121 or 127) in a cell (e.g., a cancer cell) or tissue (e.g., a tumor tissue) known to be sensitive or resistant to treatment with APO010 and used to determine the cancer patient's responsiveness to APO010. The biomarker NQO1 (SEQ ID NO: 110 or 121 or 127) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker ANXA2 (SEQ ID NO: 111 or 116 or 122) may be used to assess a cancer patient's (e.g, a patient with cancer recurrence) responsiveness to APO010. The expression level of the biomarker ANXA2 (SEQ ID NO: 111 or 116 or 122) may be assessed using nucleic acid amplification methods (e.g., PCR) or a device (e.g., a microarray). As is described above, the expression level of ANXA2 (SEQ ID NO: 111 or 116 or 122) in the patient sample may then be compared, e.g., to the expression level of ANXA2 (SEQ ID NO: 111 or 116 or 122) in a cell (e.g., a cancer cell) or tissue (e.g., a tumor tissue) known to be sensitive or resistant to treatment with APO010 and used to determine the cancer patient's responsiveness to APO010. The biomarker ANXA2 (SEQ ID NO: 111 or 116 or 122) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker MRPL40 (SEQ ID NO: 112) may be used to assess a cancer patient's (e.g, a patient with cancer recurrence) responsiveness to APO010. The expression level of the biomarker MRPL40 (SEQ ID NO: 112) may be assessed using nucleic acid amplification methods (e.g., PCR) or a device (e.g., a microarray). As is described above, the expression level of MRPL40 (SEQ ID NO: 112) in the patient sample may then be compared, e.g., to the expression level of MRPL40 (SEQ ID NO: 112) in a cell (e.g., a cancer cell) or tissue (e.g., a tumor tissue) known to be sensitive or resistant to treatment with APO010 and used to determine the cancer patient's responsiveness to APO010. The biomarker MRPL40 (SEQ ID NO: 112) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker TJP1 (SEQ ID NO: 113) may be used to assess a cancer patient's (e.g, a patient with cancer recurrence) responsiveness to APO010. The expression level of the biomarker TJP1 (SEQ ID NO: 113) may be assessed using nucleic acid amplification methods (e.g., PCR) or a device (e.g., a microarray). As is described above, the expression level of TJP1 (SEQ ID NO: 113) in the patient sample may then be compared, e.g., to the expression level of TJP1 (SEQ ID NO: 113) in a cell (e.g., a cancer cell) or tissue (e.g., a tumor tissue) known to be sensitive or resistant to treatment with APO010 and used to determine the cancer patient's responsiveness to APO010. The biomarker TJP1 (SEQ ID NO: 113) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker SERPINB6 (SEQ ID NO: 114) may be used to assess a cancer patient's (e.g, a patient with cancer recurrence) responsiveness to APO010. The expression level of the biomarker SERPINB6 (SEQ ID NO: 114) may be assessed using nucleic acid amplification methods (e.g., PCR) or a device (e.g., a microarray). As is described above, the expression level of SERPINB6 (SEQ ID NO: 114) in the patient sample may then be compared, e.g., to the expression level of SERPINB6 (SEQ ID NO: 114) in a cell (e.g., a cancer cell) or tissue (e.g., a tumor tissue) known to be sensitive or resistant to treatment with APO010 and used to determine the cancer patient's responsiveness to APO010. The biomarker SERPINB6 (SEQ ID NO: 114) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker ALDH7A1 (SEQ ID NO: 115) may be used to assess a cancer patient's (e.g, a patient with cancer recurrence) responsiveness to APO010. The expression level of the biomarker ALDH7A1 (SEQ ID NO: 115) may be assessed using nucleic acid amplification methods (e.g., PCR) or a device (e.g., a microarray). As is described above, the expression level of ALDH7A1 (SEQ ID NO: 115) in the patient sample may then be compared, e.g., to the expression level of ALDH7A1 (SEQ ID NO: 115) in a cell (e.g., a cancer cell) or tissue (e.g., a tumor tissue) known to be sensitive or resistant to treatment with APO010 and used to determine the cancer patient's responsiveness to APO010. The biomarker ALDH7A1 (SEQ ID NO: 115) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), SOGA2 (SEQ ID NO: 117), G6PD (SEQ ID NO: 118), RHOBTB3 (SEQ ID NO: 119), PPP4R1 (SEQ ID NO:

120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker SOGA2 (SEQ ID NO: 117) may be used to assess a cancer patient's (e.g, a patient with cancer recurrence) responsiveness to APO010. The expression level of the biomarker SOGA2 (SEQ ID NO: 117) may be assessed using nucleic acid amplification methods (e.g., PCR) or a device (e.g., a microarray). As is described above, the expression level of SOGA2 (SEQ ID NO: 117) in the patient sample may then be compared, e.g., to the expression level of SOGA2 (SEQ ID NO: 117) in a cell (e.g., a cancer cell) or tissue (e.g., a tumor tissue) known to be sensitive or resistant to treatment with APO010 and used to determine the cancer patient's responsiveness to APO010. The biomarker SOGA2 (SEQ ID NO: 117) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker G6PD (SEQ ID NO: 118) may be used to assess a cancer patient's (e.g, a patient with cancer recurrence) responsiveness to APO010. The expression level of the biomarker G6PD (SEQ ID NO: 118) may be assessed using nucleic acid amplification methods (e.g., PCR) or a device (e.g., a microarray). As is described above, the expression level of G6PD (SEQ ID NO: 118) in the patient sample may then be compared, e.g., to the expression level of G6PD (SEQ ID NO: 118) in a cell (e.g., a cancer cell) or tissue (e.g., a tumor tissue) known to be sensitive or resistant to treatment with APO010 and used to determine the cancer patient's responsiveness to APO010. The biomarker G6PD (SEQ ID NO: 118) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker RHOBTB3 (SEQ ID NO: 119) may be used to assess a cancer patient's (e.g, a patient with cancer recurrence) responsiveness to APO010. The expression level of the biomarker RHOBTB3 (SEQ ID NO: 119) may be assessed using nucleic acid amplification methods (e.g., PCR) or a device (e.g., a microarray). As is described above, the expression level of RHOBTB3 (SEQ ID NO: 119) in the patient sample may then be compared, e.g., to the expression level of RHOBTB3 (SEQ ID NO: 119) in a cell (e.g., a cancer cell) or tissue (e.g., a tumor tissue) known to be sensitive or resistant to treatment with APO010 and used to determine the cancer patient's responsiveness to APO010.

The biomarker RHOBTB3 (SEQ ID NO: 119) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker PPP4R1 (SEQ ID NO: 120) may be used to assess a cancer patient's (e.g, a patient with cancer recurrence) responsiveness to APO010. The expression level of the biomarker PPP4R1 (SEQ ID NO: 120) may be assessed using nucleic acid amplification methods (e.g., PCR) or a device (e.g., a microarray). As is described above, the expression level of PPP4R1 (SEQ ID NO: 120) in the patient sample may then be compared, e.g., to the expression level of PPP4R1 (SEQ ID NO: 120) in a cell (e.g., a cancer cell) or tissue (e.g., a tumor tissue) known to be sensitive or resistant to treatment with APO010 and used to determine the cancer patient's responsiveness to APO010. The biomarker PPP4R1 (SEQ ID NO: 120) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker ADARB1 (SEQ ID NO: 123) may be used to assess a cancer patient's (e.g, a patient with cancer recurrence) responsiveness to APO010. The expression level of the biomarker ADARB1 (SEQ ID NO: 123) may be assessed using nucleic acid amplification methods (e.g., PCR) or a device (e.g., a microarray). As is described above, the expression level of ADARB1 (SEQ ID NO: 123) in the patient sample may then be compared, e.g., to the expression level of ADARB1 (SEQ ID NO: 123) in a cell (e.g., a cancer cell) or tissue (e.g., a tumor tissue) known to be sensitive or resistant to treatment with APO010 and used to determine the cancer patient's responsiveness to APO010. The biomarker ADARB1 (SEQ ID NO: 123) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), KIAA0355 (SEQ ID NO: 124), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker KIAA0355 (SEQ ID NO: 124) may be used to assess a cancer patient's (e.g, a patient with cancer recurrence) responsiveness to APO010. The expression level of the biomarker KIAA0355 (SEQ ID NO: 124) may be assessed using nucleic acid amplification methods (e.g., PCR) or a device (e.g., a microarray). As is described above, the expression level of KIAA0355 (SEQ ID NO: 124) in the patient sample may then be compared, e.g., to the expression level of KIAA0355 (SEQ ID NO: 124) in a cell (e.g., a cancer cell) or tissue (e.g., a tumor tissue) known to be sensitive or resistant to treatment with APO010 and used to determine the cancer patient's responsiveness to APO010. The biomarker KIAA0355 (SEQ ID NO: 124) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), RFC4 (SEQ ID NO: 125), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker RFC4 (SEQ ID NO: 125) may be used to assess a cancer patient's (e.g, a patient with cancer recurrence) responsiveness to APO010. The expression level of the biomarker RFC4 (SEQ ID NO: 125) may be assessed using nucleic acid amplification methods (e.g., PCR) or a device (e.g., a microarray). As is described above, the expression level of RFC4 (SEQ ID NO: 125) in the patient sample may then be compared, e.g., to the expression level of RFC4 (SEQ ID NO: 125) in a cell (e.g., a cancer cell) or tissue (e.g., a tumor tissue) known to be sensitive or resistant to treatment with APO010 and used to determine the cancer patient's responsiveness to APO010. The biomarker RFC4 (SEQ ID NO: 125) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), MAFF (SEQ ID NO: 126), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker MAFF (SEQ ID NO: 126) may be used to assess a cancer patient's (e.g, a patient with cancer recurrence) responsiveness to APO010. The expression level of the biomarker MAFF (SEQ ID NO: 126) may be assessed using nucleic acid amplification methods (e.g., PCR) or a device (e.g., a microarray). As is described above, the expression level of MAFF (SEQ ID NO: 126) in the patient sample may then be compared, e.g., to the expression level of MAFF (SEQ ID NO: 126) in a cell (e.g., a cancer cell) or tissue (e.g., a tumor tissue) known to be sensitive or resistant to treatment with APO010 and used to determine the cancer patient's responsiveness to APO010. The biomarker MAFF (SEQ ID NO: 126) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), FOXK2 (SEQ ID NO: 128), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker FOXK2 (SEQ ID NO: 128) may be used to assess a cancer patient's (e.g, a patient with cancer recurrence) responsiveness to APO010. The expression level of the biomarker FOXK2 (SEQ ID NO: 128) may be assessed using nucleic acid amplification methods (e.g., PCR) or a device (e.g., a microarray). As is described above, the expression level of FOXK2 (SEQ ID NO: 128) in the patient sample may then be compared, e.g., to the expression level of FOXK2 (SEQ ID NO: 128) in a cell (e.g., a cancer cell) or tissue (e.g., a tumor tissue) known to be sensitive or resistant to treatment with APO010 and used to determine the cancer patient's responsiveness to APO010. The biomarker FOXK2 (SEQ ID NO: 128) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), and/or CCND1 (SEQ ID NO: 129). The expression level of the biomarker(s) may be determined using, e.g., a microarray, PCR, or other techniques described herein, for example, using a nucleic acid probe sequence based on the target sequences shown in Tables 1 and 2.

The biomarker CCND1 (SEQ ID NO: 129) may be used to assess a cancer patient's (e.g, a patient with cancer recurrence) responsiveness to APO010. The expression level of the biomarker CCND1 (SEQ ID NO: 129) may be assessed using nucleic acid amplification methods (e.g., PCR) or a device (e.g., a microarray). As is described above, the expression level of CCND1 (SEQ ID NO: 129) in the patient sample may then be compared, e.g., to the expression level of CCND1 (SEQ ID NO: 129) in a cell (e.g., a cancer cell) or tissue (e.g., a tumor tissue) known to be sensitive or resistant to treatment with APO010 and used to determine the cancer patient's responsiveness to APO010. The biomarker CCND1 (SEQ ID NO: 129) may be used alone to predict cancer patient responsiveness to treatment with APO010 or in combination with one or more additional biomarkers (e.g., one, two, three, four, five, ten, or all of the biomarkers shown in Tables 1 and 2), such as CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), ITGA4 (SEQ ID NO: 36), PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), and/or FOXK2 (SEQ ID NO: 128). The expression level of the biomarker(s) may be determined using, e.g., a microarray, PCR, or other techniques described herein, for example, using a nucleic acid probe sequence based on the target sequences shown in Tables 1 and 2.

**Methods of Treatment**

The diagnostic methods of the invention permit the assessment of whether a patient is likely to be responsive to treatment with APO010, and can thus be used to direct the patient's treatment (e.g., as a first line therapy and/or as a second or third line therapy). A patient to be treated or tested for responsiveness to APO010 according to the methods may include, e.g., a patient that has been diagnosed with cancer, a patient that has not received a cancer treatment (e.g., an anti-cancer agent other than APO010 or radiation), a patient that has received a cancer treatment (e.g., an anti-cancer agent other than APO010 or radiation), or a patient during treatment with APO010.

For example, the patient may have a hematological cancer or a solid tumor, such as a cancer type selected from myeloma (e.g., multiple myeloma), colorectal cancer (e.g., colon cancer and rectal cancer), leukemia (e.g., acute myeloid leukemia, acute lymphoid leukemia, chronic myeloid leukemia, chronic lymphocytic leukemia, acute myeloblastic leukemia, acute promyelocytic leukemia, acute myelomonocytic leukemia, acute monocytic leukemia, acute erythroleukemia, and chronic leukemia), myelodysplastic syndrome, lymphoma (e.g., diffuse large B-cell lymphoma, cutaneous T-cell lymphoma, peripheral T-cell lymphoma, Hodgkin's lymphoma, non-Hodgkin's lymphoma, Waldenstrom's macroglobulinemia, and lymphocytic lymphoma), cervical cancer, prostate cancer, esophageal cancer, melanoma, glioma (e.g., oligodendroglioma), pancreatic cancer (e.g., adenosquamous carcinoma, signet ring cell carcinoma, hepatoid carcinoma, colloid carcinoma, islet cell carcinoma, and pancreatic neuroendocrine carcinoma), ovarian cancer (e.g., ovarian adenocarcinoma or embryonal carcinoma), gastrointestinal stromal tumor, sarcoma (e.g., fibrosarcoma, myxosarcoma, liposarcoma, chondrosarcoma, osteogenic sarcoma, angiosarcoma, endotheliosarcoma, lymphangiosarcoma, lymphangioendotheliosarcoma, leiomyosarcoma, Ewing's sarcoma, and rhabdomyosarcoma), breast cancer (e.g., medullary carcinoma), ER-positive cancer, bladder cancer, head and neck cancer (e.g., squamous cell carcinoma of the head and neck), lung cancer (e.g., non-small cell lung carcinoma, large cell carcinoma, bronchogenic carcinoma, and papillary adenocarcinoma), metastatic cancer, oral cavity cancer, uterine cancer, testicular cancer (e.g., seminoma and embryonal carcinoma), skin cancer (e.g., squamous cell carcinoma and basal cell carcinoma), thyroid cancer (e.g., papillary carcinoma and medullary carcinoma), brain cancer (e.g., astrocytoma and craniopharyngioma), stomach cancer, intra-epithelial cancer, bone cancer, biliary tract cancer, eye cancer, liver cancer (e.g., hepatocellular carcinoma or hepatoma), larynx cancer, kidney cancer (e.g., renal cell carcinoma and Wilms tumor), gastric cancer, blastoma (e.g., nephroblastoma, medulloblastoma, hemangioblastoma, neuroblastoma, and retinoblastoma), polycythemia vera, chordoma, synovioma, mesothelioma, adenocarcinoma, sweat gland carcinoma, sebaceous gland carcinoma, cystadenocarcinoma, bile duct carcinoma, choriocarcinoma, epithelial carcinoma, ependymoma, pinealoma, acoustic neuroma, schwannoma, meningioma, pituitary adenoma, nerve sheath tumor, cancer of the small intestine, cancer of the endocrine system, cancer of the penis, cancer of the urethra, cutaneous or intraocular melanoma, a gynecologic tumor, solid tumors of childhood, and neoplasms of the central nervous system. In particular, the cancer of the patient is, e.g., multiple myeloma, breast cancer, acute myelogenous leukemia (AML), acute lympho-blastic leukemia (ALL), chronic lymphocytic leukemia (CLL), myelodysplastic syndrome (MDS), chronic myelogenous leukemia - chronic phase (CMLCP), diffuse large B-cell lymphoma (DLBCL), cutaneous T-cell lymphoma (CTCL), peripheral T-cell lymphoma (PTCL), Hodgkin's lymphoma, hepatocellular carcinoma (HCC), cervical cancer, prostate cancer, renal cell carcinoma (RCC), esophageal cancer, melanoma, glioma, pancreatic cancer, ovarian cancer, gastrointestinal stromal tumors (GIST), sarcoma, estrogen receptor-positive (ERpos) breast cancer, non-small cell lung carcinoma (NSCLC), colon cancer, bladder cancer, or squamous cell carcinoma of the head and neck (SCCHN). The patient may also have recurrence of cancer, such as a recurrent form of any of the above cancer types, e.g., recurrent multiple myeloma or recurrent breast cancer.

A patient found to be responsive to APO010 according to the methods of the invention may be preferentially selected for treatment with APO010. For example, a patient can be identified as responsive to APO010 by determining the expression level of one or more biomarkers (e.g., one or more of the biomarkers shown in Tables 1 and/or 2, such as CORO1A) in a biological sample (e.g., a tumor sample) obtained from the patient, and subsequently administered APO010. Alternatively, a patient can be, e.g., identified as less likely to be responsive to APO010 by determining the expression level of one or more biomarkers (e.g., one or more of the biomarkers shown in Tables 1 and 2, such as CORO1A) in a biological sample obtained from the patient. If the patient exhibits expression levels of one or more biomarkers indicative of non-responsiveness to APO010, the patient is subsequently a treatment other than APO010. In particular, the patient may be treated with, e.g., radiation and/or administration of a therapeutic agent, such as histone deacetylase (HDAC) inhibitor, ipilimumab, bortezomib, carfilzomib, thalidomide, lenalidomide, pomalidomide, prednisone, dexamethasone, cyclophosphamide, vincristine, doxorubicin, melphalan, capecitabine, tegafur, irinotecan, oxaliplatin, cetuximab, leucovorin, SN-38, everolimus, temsirolimus, bleomycin, lomustine, depsipeptide, carboplatin, erlotinib, gemcitabine, mitoxantrone, cisplatin, busulfan, epirubicin, arsenic trioxide, bendamustine, fulvestrant, teniposide, adriamycin, decitabine, estramustine, etoposide, azaguanine, aclarubicin, mitoxantrone, mitomycin, paclitaxel, taxotere, Irofulven, 5-FU, ara-c, methylprednisolone, methotrexate, methyl-gag, belinostat, carboplatin, idarubicin, IL4-PR38, valproic acid, all-trans retinoic acid (ATRA), cytoxan, topotecan, suberoylanilide hydroxamic acid, leukeran, fludarabine, vinblastine, dacarbazine, hydroxyurea, tegafur, daunorubicin, mechlorethamine, streptozocin, carmustine, mercaptopurine, dactinomycin, tretinoin, ifosfamide, tamoxifen, floxuridine, thioguanine, PSC 833, herceptin, bevacizumab, celecoxib, iressa, anastrozole, letrozole, and rituximab.

*Administration of APO010*

Once a patient has been determined to be responsive to APO010, according to the methods described herein, APO010 may be administered to the patient, for example, parenterally, enterally, or topically. Enteral routes of APO010 administration include oral, buccal, sublabial, sublingual, or by inhalation. Parenteral routes of APO010 administration include intravenous, transdermal, intradermal, intramuscular, intra-arterial, intracranial, subcutaneous, intraorbital, intraventricular, intraspinal, intraperitoneal, or intranasal. The preferred route for administration of APO010 may be intravenous.

APO010, can be administered at, e.g., a dose of about 2 $\mu g/m^2$ to 500 $\mu g/m^2$, 5 $\mu g/m^2$ to 450 $\mu g/m^2$, about 10 $\mu g/m^2$ to 400 $\mu g/m^2$, about 15 $\mu g/m^2$ to 375 $\mu g/m^2$, about 20 $\mu g/m^2$ to 350 $\mu g/m^2$, about 30 $\mu g/m^2$ to 300 $\mu g/m^2$, about 35 $\mu g/m^2$ to 300 $\mu g/m^2$, about 40 $\mu g/m^2$ to 250 $\mu g/m^2$, or about 45 $\mu g/m^2$ to 200 $\mu g/m^2$. In particular, APO010 is administered at a dose of about 45 $\mu g/m^2$ to 200 $\mu g/m^2$. APO010 may be administered at a frequency of, e.g., at least once hourly, once daily, twice daily, once weekly, once every two weeks, once every three weeks, once every four weeks, once monthly, once every two months, once every three months, once every six months, or once every year. The administration of APO010 can be repeated at such a frequency for a certain period of time, followed by a period without treatment. Such repeated administrations can occur over a course of therapy lasting a specified length of time (e.g., at least 1 week, 2 weeks, 3 weeks, 1 month, 2 months, 3 months, 6 months, 8 months, 10 months, 12 months, 18 months, 24 months, 36 months, 48 months, or 60 months).

APO010 can be administered in a pharmaceutical composition that includes one or more pharmaceutically acceptable carriers, excipients, or diluents. Examples of suitable carriers, excipients, or diluents of APO010 include, e.g., saline, sterile water, polyalkylene glycols, oils of vegetable origin, hydrogenated napthalenes, suitable buffer, 1,3-butanediol, Ringer's solution and/or sodium chloride solution. Exemplary formulations for parenteral administration can include solutions prepared in water suitably mixed with a surfactant, e.g., hydroxypropylcellulose. Dispersions can also be prepared in glycerol, liquid polyethylene glycols, DMSO and mixtures thereof with or without alcohol, and in oils. Under ordinary conditions of storage and use, these preparations may contain a preservative to prevent the growth of microorganisms. Other exemplary carriers, excipients, or diluents are described in the Handbook of Pharmaceutical Excipients, 6th Edition, Rowe et al., Eds., Pharmaceutical Press (2009), hereby incorporated by reference in its entirety.

## Kits

Kits of the invention can be used for determining the responsiveness of a cancer patient (e.g., a hematological cancer, such as multiple myeloma, or a solid tumor, such as breast cancer) to APO010. Kits of the invention can include reagents and/or materials for, e.g., collecting and/or purifying nucleic acids from biological samples (such as those obtained from a patient to be treated with a target drug(s) of the invention), reagents for amplifying such nucleic acids to produce an amplified sample, and/or at least one device of the invention. Reagents for amplifying nucleic acids may include, e.g., PCR reagents, including but not limited to DNA polymerase, RNA polymerase, PCR buffer, magnesium chloride solutions, nucleic acid primers (e.g., primers designed to target particular biomarkers of responsiveness to a target drug(s) of interest), and/or any other PCR reagents as are well known in the art. In particular, kits useful in the method may include includes one or more of the following: a kit for RNA extraction from tumors (e.g., Trizol for mRNA, mirVana miRNA isolation kit from Ambion Inc), a kit for RNA labeling (e.g., MessageAmp from Ambion Inc., FlashTag from Genisphere Inc), a microarray for measuring biomarker expression (e.g., HG-U133A, HG-U133_Plus2 or miRNA-1.0 from Affymetrix Inc), a microarray hybridization station and scanner (e.g., GeneChip System 3000Dx from Affymetrix Inc), and/or software for analyzing the expression of biomarker genes or RNAs (e.g., miRNAs) as described in herein (e.g., implemented in R from R-Project or S-Plus from Insightful Corp.).

For example, a kit of the invention can include one or more probes capable of detecting one or more biomarkers of Tables 1 and/or 2 (e.g., the kit may include probes for the biomarkers of Tables 1 and 2). Such probes can, for example, include nucleic acids capable of hybridizing to the biomarker based on nucleic acid sequence complementarity. In particular, a probe has at least 85% sequence identity (e.g., 85%, 90%, 95%, 97%, 98%, 99%, or 100% sequence identity) to a nucleic acid sequence that is complementary or identical to at least 5 (e.g., at least 15) consecutive nucleotides of one or more biomarkers. The probes can be attached a solid surface, such as a microarray. The kit may include NanoString capture probes, NanoString reporter probes, and/or one or more nCounter cartridges. The kit may include reagents for next generation sequencing, including but not limited to poly(T) oligonucleotides, dye terminators, sequencing adapters, adapter ligation reagents, reverse transcriptase, primers (e.g., random primers), DNA-cleaving enzymes, polymerases, and/or any combination thereof. The kit may also be one that includes a protein array and/or reagents for detection of the polypeptide product(s) of one or more biomarkers of Tables 1 and/or 2.

The following examples are intended to illustrate, rather than limit, the invention.

## EXAMPLES

### Example 1. Identification of biomarkers of sensitivity and resistance to APO010.

DNA chip measurements of the 60 cancer cell lines of the NCI60 data set were performed using Affymetrix HG-U133A arrays and logit normalized (Figure 1). For each array, the logit transformation was performed followed by a Z-transformation to mean zero and SD 1, and correlated to growth inhibition (-log(GI50)). Growth inhibition data of APO010 against the same cell lines were downloaded from the National Cancer Institute. Each gene's expression in each cell line was correlated to the growth of those cell lines (log(GI50)) in the presence of APO010. The Pearson correlation coefficient was then determined to identify genes positively and negatively correlated to sensitivity to APO010. Tables 1 and 2 show the top positively correlated genes (the biomarkers of sensitivity) and negatively correlated genes (the biomarkers of resistance). In particular, genes with a Pearson correlation greater than 0.3 or below - 0.3 can be classified as biomarkers of sensitivity or resistance, respectively.

**Table 1.** Biomarkers of sensitivity to APO010. Dashes indicate that the Affymetrix probeset has not been mapped to a specific gene. Affymetrix IDs refer to the array type HG-U133A.

| Gene | Affymetrix ID | Correlation | Affymetrix Probe Sequence | Affymetrix Probe SEQ ID NO: |
|---|---|---|---|---|
| CD47 | 211075_s_at | 0.43 | GCGGCGTGTATACCAATGCATGGCC | 1 |
| CORO1A | 209083_at | 0.416 | GCTCCAGAAGCGCTTGGACAGGCTG | 2 |
| ICAM3 | 204949_at | 0.394 | AGCGTCCAGCTCACGAGGCAAATAC | 3 |
| HCLS1 | 202957_at | 0.384 | CTGTCTACTGCAACTGTGATTTCCC | 4 |
| DOCK2 | 213160_at | 0.377 | GATTCCTGAACTCAAGGTACCAGCA | 5 |
| ARHGAP15 | 218870_at | 0.375 | TGGCGATCCACATGGTCTACCAGAA | 6 |
| CXCR4 | 211919_s_at | 0.37 | GTGGTCTATGTTGGCGTCTGGATCC | 7 |
| NCKAP1L | 209734_at | 0.367 | CCTGCTTCGAAATGCCTATCGGGAG | 8 |
| CXCR4 | 209201_x_at | 0.366 | GCCAAATTTAAAACCTCTGCCCAGC | 9 |
| MAP4K1 | 214219_x_at | 0.364 | GAGACACGCCCAGTGGATGATCCTA | 10 |
| SLA | 203761_at | 0.363 | AAGCTCATCGTGCTACTGGTATGTG | 11 |
| DENND2D | 221081_s_at | 0.359 | GGTGTCTGAGATTTGGATCCCTGGT | 12 |
| CYFIP2 | 215785_s_at | 0.356 | GACAGACAGTTCCACTGTGGAGCAT | 13 |
| PSME2 | 201762_s_at | 0.355 | GAGAGGGTGAATGCCGTCAAGACCA | 14 |
| PTPRCAP | 204960_at | 0.354 | CACTGTAGAGGCCGGTCTTGGTGTC | 15 |
| CXCR4 | 217028_at | 0.353 | GTATGTCTCGTGGTAGGACTGTAGA | 16 |

(continued)

| Gene | Affymetrix ID | Correlation | Affymetrix Probe Sequence | Affymetrix Probe SEQ ID NO: |
|---|---|---|---|---|
| MAP4K1 | 206296_x_at | 0.343 | GTGTCAGGAACTAGTCCTTGCACCC | 17 |
| UBE2L6 | 201649_at | 0.341 | GAAACCATGTTCTTGCTTAAGCCAT | 18 |
| MAP4K1 | 214339_s_at | 0.337 | TCTAGGCTCGGATCAGCTGCTACAG | 19 |
| AIF1 | 215051_x_at | 0.337 | TTCAGCTACCCTGACTTTCTCAGGA | 20 |
| PSMB9 | 204279_at | 0.332 | GATGGGTTCTGATTCCCGAGTGTCT | 21 |
| TRIM22 | 213293_s_at | 0.331 | TCCTCTGCCCCTTAAAAGATTGAAG | 22 |
| BIN2 | 219191_s_at | 0.33 | ATAAGCTTATCTCAGCTGACTCCTC | 23 |
| CD247 | 210031_at | 0.326 | TGCCGCACCATTGAACTGTACCATG | 24 |
| APOL3 | 221087_s_at | 0.324 | GTTAGAACTTTTGGATACAGCAGAA | 25 |
| PTPRC | 212587_s_at | 0.31 | AAGTGTGCAGAATACTGGCCGTCAA | 26 |
| CD53 | 203416_at | 0.309 | AGCAAGACAATCTTTCACTCACTGA | 27 |
| SELPLG | 209879_at | 0.309 | TTGTCTTTTGGTTGCCATGGTCACC | 28 |
| LAIR1 | 210644_s_at | 0.309 | TTCTCTGGGTTGTGCTTTACTCCAC | 29 |
| PSME1 | 200814_at | 0.308 | TCTGCAGCGCTTGAAGCCTGAGATC | 30 |
| MYC | 202431_s_at | 0.307 | GCAACAACCGAAAATGCACCAGCCC | 31 |
| LCP1 | 208885_at | 0.306 | ACCCCTCTGCTTTTAACTCTAGAAT | 32 |
| ZAP70 | 214032_at | 0.306 | GGTGGTCAGGCGTAGATCACCAGAA | 33 |
| SMARCA4 | 214728_x_at | 0.306 | GAGGAAGGCTCCGAATCCGAATCTC | 34 |
| PTPN7 | 204852_s_at | 0.301 | GGGTACAAGCTCCAGAACAGTAACC | 35 |
| ITGA4 | 213416_at | 0.3 | GTACTATGGTTGTCCAACACAGGCC | 36 |
| SLA | 203760_s_at | 0.299 | GTCTGGGTTTGCAGATGGGTGCCCT | 37 |
| ACAP1 | 205213_at | 0.299 | CCCTACTGTTTCGAGCGTCTGGGCA | 38 |
| TAPBPL | 218747_s_at | 0.298 | CAGAGACGGCAAGCACCTACAGGAC | 39 |
| CD3G | 206804_at | 0.297 | TGGAGTTCGCCAGTCGAGAGCTTCA | 40 |
| CD3D | 213539_at | 0.297 | GGGAACACTGCTCTCAGACATTACA | 41 |
| ALOX5AP | 204174_at | 0.293 | GTAGGCAATGTTGTCCTGTTGGCCA | 42 |
| SASH3 | 204923_at | 0.291 | AAGGACAGAATTGCCTCTCGGAAGC | 43 |
| P2RX5 | 210448_s_at | 0.291 | GTATTCTCTGACACTCTTATTTGGT | 44 |
| LAT | 209881_s_at | 0.29 | GAGCGCAGAAGCGTCTCTGGATGGC | 45 |
| IL2RG | 204116_at | 0.289 | GGCTGATTTGGAATTTTGTGCCCCC | 46 |
| RHOH | 204951_at | 0.288 | CTACAAGTGAACTCCTTGCCCAGGC | 47 |
| RASGRP2 | 208206_s_at | 0.288 | AGCAGTGCAAGGATCGCCTGTCAGT | 48 |
| LCP2 | 205270_s_at | 0.287 | AACAACCAATCCATATGTCCTCATG | 49 |
| TPK1 | 221218_s_at | 0.287 | GTACACAAATCCCTATGTTGCTATA | 50 |
| BCL2 | 203685_at | 0.283 | GTCACATTGCCATTAAACTTTCCTT | 51 |
| ELF1 | 212420_at | 0.283 | TTGGTGATCCAGCTATTTTTCCTGC | 52 |

(continued)

| Gene | Affymetrix ID | Correlation | Affymetrix Probe Sequence | Affymetrix Probe SEQ ID NO: |
|---|---|---|---|---|
| SEMA4D | 203528_at | 0.282 | TCGGTCAGAACCTCCTGTGAGACAG | 53 |
| VAV1 | 206219_s_at | 0.282 | TTTCCGGGGGCTTACGGAGCTGGTG | 54 |
| MZB1 | 221286_s_at | 0.282 | GCATTGCTATGTGGGGGACCCCAGG | 55 |
| SP110 | 208012_x_at | 0.281 | GGAATGACCCTAGGAGAGCTGCTGA | 56 |
| IFI16 | 208965_s_at | 0.281 | ACTTCATTTTCTTAGCGTTTCTGGA | 57 |
| MX1 | 202086_at | 0.279 | TCTGCTTATCCGTTAGCCGTGGTGA | 58 |
| ARHGDIB | 201288_at | 0.276 | AGCTGGGAGTGGAACCTGTCGATTA | 59 |
| RPL17 RPL17-C18ORF32 | 212270_x_at | 0.276 | GCCGGACCTACAGAGCTCATGGTCG | 60 |
| TRAF3IP3 | 213888_s_at | 0.275 | GGACTACATGACCTCCTGGAGTCCC | 61 |
| PTPRC | 207238_s_at | 0.273 | AACCTCCTGTTAGCTGTTATTTCTA | 62 |
| LAT | 211005_at | 0.273 | CCATGAGACAGTCCCAGAACACGGC | 63 |
| MAL | 204777_s_at | 0.272 | AGATCTTTCCAGAGGTCCATGGTGG | 64 |
| TARP | 209813_x_at | 0.271 | CCTCCTGCTCCTCAAGAGTGTGGTC | 65 |
| DDX60 | 218986_s_at | 0.271 | GTTCTGTCATGAATACTCACAAATT | 66 |
| IGLL1 | 206660_at | 0.27 | CACCCAGGGCGTGGAGATGACCACG | 67 |
| CD1A | 210325_at | 0.27 | GTCCTCTCAACTCTCTTTGTAAAAA | 68 |
| SH2D1A | 210116_at | 0.269 | GGATGCCGTGGGAGTACAAAAGTGG | 69 |
| ISG15 | 205483_s_at | 0.268 | GTGGACAAATGCGACGAACCTCTGA | 70 |
| TARP TRGC2 | 215806_x_at | 0.267 | AAATGATACACTACTGCTGCAGCTC | 71 |
| IKZF1 | 205038_at | 0.266 | TGTGGAAAGCCTGGATCTCAGCTCC | 72 |
| NOTCH1 | 218902_at | 0.266 | ATACCAAGTATAGCCTATGGCAGAA | 73 |
| MFNG | 204153_s_at | 0.264 | TGGCTAATGTCTCTCAGTCATTCCC | 74 |
| CD1B | 206749_at | 0.264 | TCATCCTCTACTGGAGAAACCCCAC | 75 |
| IFITM1 IFITM2 | 201601_x_at | 0.263 | TTCCCCAAAGCCAGAAGATGCACAA | 76 |
| IFIT3 | 204747_at | 0.263 | CCTGCTAAGGGATGCCCCTTCAGGC | 77 |
| STAT6 | 201331_s_at | 0.262 | GAACATGTGTCTATCTGCTTTTGCC | 78 |
| TAP1 | 202307_s_at | 0.262 | AGAATGAAAGCCTTCTCAGACCTGC | 79 |
| STAT1 | 209969_s_at | 0.262 | TATCGCCATCACAGCTGAACTTGTT | 80 |
| HOXA10-HOXA9 HOXA9 MIR196B | 214651_s_at | 0.262 | TAGCTCTGTAGTGGAATATGTCTTC | 81 |
| ANK3 | 206385_s_at | 0.26 | AAGCCAATCATTTGTAACCATTCTA | 82 |
| LPAR6 | 218589_at | 0.26 | AGAAGCCTGCTTTGAAAATTTTCCA | 83 |
| PSMB8 | 209040_s_at | 0.259 | CTTGGCCGACTCAGGGACCTAAGCC | 84 |
| SH2D1A | 211210_x_at | 0.259 | TCTGCCTGAAAGCCCCATGAAGAAA | 85 |

(continued)

| Gene | Affymetrix ID | Correlation | Affymetrix Probe Sequence | Affymetrix Probe SEQ ID NO: |
|---|---|---|---|---|
| TRAT1 | 217147_s_at | 0.258 | TCTCCTTTCTCACCAATGGGCAATA | 86 |
| IFI16 | 208966_x_at | 0.257 | TGGACGACTGACCACAATCAACTGT | 87 |
| CD1E | 215784_at | 0.257 | AACTCTGGTGACATTTGCTTTACCT | 88 |
| TARP TRGC2 | 216920_s_at | 0.257 | GCTCACAAACACCTCTGCATATTAC | 89 |
| TARP TRGC2 | 211144_x_at | 0.255 | GAAGCGTCTTCTGAGGATCTAGTTG | 90 |
| PVRIG | 219812_at | 0.255 | AGGACCCTTAGGAGTTCGATGAGAG | 91 |
| DPP4 | 203716_s_at | 0.254 | ACACAGAACGTTACATGGGTCTCCC | 92 |
| CA2 | 209301_at | 0.254 | TGAATCTTCGGGTGTTTCCCTTTAG | 93 |
| HMHA1 | 212873_at | 0.254 | ACGGCATAGCTTAGGTGCGCCGTCC | 94 |
| LRMP | 204674_at | 0.253 | CTCTGGCTCTCTATTGCATTCATTG | 95 |
| IKZF1 | 205039_s_at | 0.253 | ATTAAGGTCATTACTCTCAACCACC | 96 |
| ARHGEF6 | 209539_at | 0.252 | ATGCATGTTTCGGGGACTTGGCCCT | 97 |
| CYFIP2 | 220999_s_at | 0.252 | GGTGACATATTTACGCTTGTGATCA | 98 |
| PTPRC | 212588_at | 0.251 | GTGAGCTTATCATGCTGTCTTTACA | 99 |

**Table 2.** Biomarkers of resistance to APO010. Dashes indicate that the Affymetrix probeset has not been mapped to a specific gene. Affymetrix IDs refer to the array type HG-U133A.

| Gene | Affymetrix ID | Correlation | Affymetrix Probe Sequence | Affymetrix Probe SEQ ID NO: |
|---|---|---|---|---|
| PDE8A | 212522_at | -0.395 | GCTGGGGTCGTATTCTAAGTGCTAA | 100 |
| HOXB7 | 204779_s_at | -0.37 | GCAGTTTTGTAAGCCCTCTTTAATG | 101 |
| PRC1 | 218009_s_at | -0.366 | TTGCACATGTCACTACTGGGGAGGT | 102 |
| HOXB7 | 216973_s_at | -0.364 | GTAAGCCCTCTTTAATGCTGTCTTT | 103 |
| SCRN1 | 201462_at | -0.362 | TCATGTGCACATGCCGTTGCAGCAC | 104 |
| ACTN1 | 208636_at | -0.353 | AACTATTTGCACCGAAATGTCTTGT | 105 |
| NGRN | 217722_s_at | -0.353 | GACCTCTGTAGACCTTCAGTACTCA | 106 |
| DKK1 | 204602_at | -0.347 | GAGTCTAGAACGCAAGGATCTCTTG | 107 |
| IER3 | 201631_s_at | -0.345 | TGAGATCCGTGAGATCCTTCCATCT | 108 |
| ACTN1 | 208637_x_at | -0.342 | CCGACCAGGCTGAGTACTGCATCGC | 109 |
| NQO1 | 210519_s_at | -0.337 | GTGGCTTCCAAGTCTTAGAACCTCA | 110 |

(continued)

| Gene | Affymetrix ID | Correlation | Affymetrix Probe Sequence | Affymetrix Probe SEQ ID NO: |
|---|---|---|---|---|
| ANXA2 | 210427_x_ at | -0.332 | GAAAATGCTTTCCTGAACCTGGTTC | 111 |
| MRPL40 | 203152_at | -0.331 | TCCATGCTGAGGCCATCAAGCGGGA | 112 |
| TJP1 | 202011_at | -0.33 | AAGTATCCCTACTGTAATTTGTGAT | 113 |
| SERPINB6 | 211474_s_ at | -0.328 | GATGATGCGGTGTGCCAGATTCGTC | 114 |
| ALDH7A1 | 208951_at | -0.326 | GCAAGGTTATGGATCGCCCTGGAAA | 115 |
| ANXA2 | 213503_x_ at | -0.323 | GAGGGTGACGTTAGCATTACCCCCA | 116 |
| SOGA2 | 213358_at | -0.321 | ATGTATATACCGCTACTGTGTCCTC | 117 |
| G6PD | 202275_at | -0.316 | GTCTGTCCCAGAGCTTATTGGCCAC | 118 |
| RHOBTB3 | 202976_s_ at | -0.314 | GGACAGACTGATAGTTTCTTTCTTT | 119 |
| PPP4R1 | 201594_s_ at | -0.31 | GAGGTGCAACTTCTTCACATACTGT | 120 |
| NQO1 | 201467_s_ at | -0.307 | CTTTCCATCACCACTGGTGGCAGTG | 121 |
| ANXA2 | 201590_x_ at | -0.306 | GCTGATCGGCTGTATGACTCCATGA | 122 |
| ADARB1 | 203865_s_ at | -0.305 | GTTTGCTTTTTCAGGCTTTGGATTA | 123 |
| KIAA0355 | 203288_at | -0.303 | GACAGAGGTCAATCCTTGATGCTCC | 124 |
| RFC4 | 204023_at | -0.302 | ACCCCTGACCTCTAGATGTTCAAAA | 125 |
| MAFF | 36711_at | -0.301 | AGCAAGCGAGTTATCGTCTTCTGTA | 126 |
| NQO1 | 201468_s_ at | -0.3 | ACTGCCCTCTTGTGGTGCATTGAAA | 127 |
| FOXK2 | 203064_s_ at | -0.3 | AGACTTTCAGAATCACACAGGCCCT | 128 |
| CCND1 | 208712_at | -0.3 | GACGCGCAAGTCTGAGGGTCTGGGC | 129 |
| YES1 | 202933_s_ at | -0.298 | GATGGTATTCATTAGGTTTTAACTG | 130 |
| CTPS1 | 202613_at | -0.296 | GAGACAAAATCTCTATACTGCCCTG | 131 |
| FADS1 MIR1908 | 208963_x_ at | -0.296 | GATGAGCGTCCTAAGGCATGTTGGG | 132 |
| SHC1 | 214853_s_ at | -0.296 | AACTGGGATCGCACCTTTTATACCA | 133 |
| ETF1 | 201574_at | -0.291 | GCTTTGGGCTCTGCTATTTGTTTGC | 134 |
| ARL4A | 205020_s_ at | -0.291 | GAGGAACTCATTGTCACTTTCAGAA | 135 |
| AP2A2 | 211779_x_ at | -0.291 | GCAGATGAGCACCGTGTCCAGTGCC | 136 |

(continued)

| Gene | Affymetrix ID | Correlation | Affymetrix Probe Sequence | Affymetrix Probe SEQ ID NO: |
|---|---|---|---|---|
| EGR1 | 201694_s_at | -0.29 | ACGTCTTGGTGCCTTTTGTGTGATG | 137 |
| PMP22 | 210139_s_at | -0.29 | GATGCTAAGACTCCAGACCTTTTGT | 138 |
| FLOT1 | 208749_x_at | -0.289 | TGGTGTCCAGCGGCAGTGGGACCAT | 139 |
| P2RX5-TAX1BP3 TAX1BP3 | 215464_s_at | -0.285 | GCCAGAGGCGCTACGAAGCTTTGCC | 140 |
| ANXA2P2 | 208816_x_at | -0.284 | TGCCCCACCTCCAGAAAGTATTTGA | 141 |
| SOCS2 | 203373_at | -0.283 | TGTCCCAACCTGTTGCCATTGATTT | 142 |
| C2orf18 CENPA | 204962_s_at | -0.282 | AGACCACTTTGAGCAGTTGCCTGGA | 143 |
| TOX3 | 214774_x_at | -0.281 | GGTGACATAAACCATTCATTGCTAC | 144 |
| CTNNAL1 | 202468_s_at | -0.279 | ACGGATGGGTCTCAGTTACAAATAA | 145 |
| PTRF | 208789_at | -0.279 | GCAGCCCGACCAATAAACCTGCTTT | 146 |
| MICA | 205904_at | -0.278 | AGCCTCTGATGTCAGATCTTGGGTC | 147 |
| SYNM | 212730_at | -0.276 | AGCTCCATGTCCTTTAAAATCAGTC | 148 |
| IGF2BP2 | 218847_at | -0.276 | AGCTACCTCAGGTG TTTTTACCTCA | 149 |
| DBNDD2 SYS1 SYS1-DBNDD2 | 218094_s_at | -0.275 | GTGGGATTGTGTACCTTTCTAAGAA | 150 |
| CDC14B | 221556_at | -0.275 | TACCCACATCAGGTTATTCTCCATG | 151 |
| COMMD4 | 206441_s_at | -0.272 | TCCTTGTCCAGTGAACTGCAGCAGC | 152 |
| MIR21 VMP1 | 220990_s_at | -0.272 | GTTCAGCAGCAAACTTGCAACAGAC | 153 |
| ECT2 | 219787_s_at | -0.271 | TAGCTGTTTCAGAGAGAGTACGGTA | 154 |
| ANXA1 | 201012_at | -0.265 | CCTTTGCCAAGCCATCCTGGATGAA | 155 |
| CRK | 202225_at | -0.265 | CCACTGGGCTGGATTTGACTGTTGA | 156 |
| PTRF | 208790_s_at | -0.265 | GGACTCAAAGCCAAAACTGCCCAAA | 157 |
| TFE3 | 212457_at | -0.264 | GCCAGGCCCCATGGAGGGTATACTG | 158 |
| RAB31 | 217763_s_at | -0.264 | TGAGAAGCCAACCATGCAAGCCAGC | 159 |
| FAM50A | 203262_s_at | -0.263 | TCTACGACTTCATCGTCACCAAGGC | 160 |
| YES1 | 202932_at | -0.262 | ACGTAACCTGCTTAGTATTGACACT | 161 |
| OBSL1 | 212775_at | -0.262 | GTCACAGAGTCTTACCAAAGTCAGG | 162 |

(continued)

| Gene | Affymetrix ID | Correlation | Affymetrix Probe Sequence | Affymetrix Probe SEQ ID NO: |
|---|---|---|---|---|
| C16orf80 | 217957_at | -0.26 | GAGCTGCCGGCAGAGTTCAAACTGT | 163 |
| CKB | 200884_at | -0.259 | TGTAAGGAAGGCTTCCGTCACCCTT | 164 |
| VOPP1 | 208091_s_at | -0.257 | GACAACTGCGTGGGTCCAAACACTC | 165 |
| ZIC1 | 206373_at | -0.255 | TAGTACGAGCCTGGATCTGCGTGTC | 166 |
| RAB31 | 217762_at | -0.254 | GAAATGGTTAATCATGCTGTGTGCT | 167 |
| SEPT10 | 212698_s_at | -0.253 | GATCTTAAGCTTTTGTGTCAGATTA | 168 |
| SLC35D2 | 213083_at | -0.252 | GCAAAGGGCCAGTTGTTTCAGTTTA | 169 |

**Example 2. Predicting responsiveness to APO010 in various cancer patient populations.**

An mRNA-based predictor of responsiveness to APO010 developed according to the methods of the invention was applied to 3,522 patients having a variety of cancers. Each patient had a pre-treatment measurement of gene expression with an Affymetrix array of either type U133A or U133Plus2. The predicted APO010 sensitivity of each patient was calculated as the difference between the mean of the expression levels of the biomarkers of sensitivity and the mean of the expression levels of the biomarkers of resistance for the patient. When the patients were grouped by cancer types, and cancer types predicted to be more responsive to APO010 were identified (Figure 2).

Of 27 different cancer types, hematological cancers were predicted to be more responsive to APO010 treatment than solid tumor cancers. In particular, patients with multiple myeloma, acute myelogenous leukemia (AML), acute lympho-blastic leukemia (ALL), chronic lymphocytic leukemia (CLL), myelodysplastic syndrome (MDS), chronic myelogenous leukemia - chronic phase (CMLCP), diffuse large B-cell lymphoma (DLBCL), cutaneous T-cell lymphoma (CTCL), peripheral T-cell lymphoma (PTCL), and Hodgkin's lymphoma were predicted to be the most responsive to APO010. Patients with solid tumor types were also predicted to be responsive to APO010 treatment, in particular, breast cancer, hepatocellular carcinoma (HCC), cervical cancer, prostate cancer, renal cell carcinoma (RCC), esophageal cancer, melanoma, glioma, pancreatic cancer, ovarian cancer, gastrointestinal stromal tumors (GIST), sarcoma, estrogen receptor-positive (ERpos) breast cancer, non-small cell lung carcinoma (NSCLC), colon cancer, bladder cancer, and squamous cell carcinoma of the head and neck (SCCHN).

The median of the boxplots shown in Figure 3 is a cutoff that may be used to separate patients predicted to be responsive to APO010 treatment from patients predicted to be non-responsive to APO010 treatment for a given cancer type. Values above the median indicate patients predicted to be responsive to APO010, while values below the median indicate patients predicted to be non-responsive to APO010. For a test sample from an individual patient, it is useful to compare the test sample to the reference population for the same cancer type. If the test sample is above the median for the reference population of the same cancer type, then the patient is predicted to be responsive to APO010 treatment. If the test sample is below the median for the reference population of the same cancer type, then the patient is predicted to be non-responsive to APO010 treatment. This method for predicting patient responsiveness can also be used when the reference cancer population consists of only two patients: a patient responsive to APO010 treatment and a patient non-responsive to APO010 treatment.

**Example 3. Responsiveness of multiple myeloma cells to APO010.**

A human multiple myeloma cell line (OPM-2) was tested for responsiveness to APO010. There was a decrease in relative tumor size of the OPM-2 cells exposed to APO010 at dosages of 0.01 mg/kg, 0.02 mg/kg, and 0.03 mg/kg after 5 days, 10 days, and 15 days relative to control OPM-2 cells exposed to vehicle (Figure 3). The responsiveness of OPM-2 cells to APO010 was the compared to a conventional treatment for multiple myeloma (bortezomib (VELCADE®)). There was a greater decrease in relative tumor size of OPM-2 cells exposed to APO010 at a dosage of 0.02 mg/kg than OPM-2 cells exposed to bortezomib at a dosage of 0.5 mg/kg after 5 to 10 days of treatment (Figure 4). These results demonstrate that multiple myeloma cells are responsive to APO010 as predicted using the methods for predicting responsiveness of cancer patient populations to APO010 described in Example 2.

**Example 4. Predicting responsiveness of multiple myeloma patients to APO010.**

The diagnostic methods of the present invention can be used to predict the responsiveness of a multiple myeloma patient to treatment with APO010. In particular, the multiple myeloma patient may be one that has not previously received any cancer treatment or one that has received a cancer treatment other than APO010. Moreover, the patient may be one diagnosed with multiple myeloma or one with recurrence of multiple myeloma.

A biological sample (e.g., plasma cells isolated from the bone marrow of the patient, such as CD138 positive plasma cells) may be obtained from the patient through methods well known in the art (Figure 5). The sample may be frozen and/or prepared, e.g., by formalin fixation and paraffin embedding. In particular, mRNA can be isolated from the sample and a gene expression profile can be determined, e.g., using a microarray platform, such as the Affymetrix HG-U133A, for one or more of the biomarkers shown in Tables 1 and 2. One or more of the biomarkers shown in Tables 1 and 2 can also be measured, e.g., by sequencing or PCR-based techniques, such as those described herein.

For example, the expression level of one or more biomarkers of sensitivity to APO010 can be determined in the sample from the patient, such as one or more of CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), and ITGA4 (SEQ ID NO: 36). In particular, the biomarker is CORO1A (SEQ ID NO: 2). The expression level of one or more biomarkers of resistance to APO010 can also be determined in the sample from the patient, such as one or more of PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and CCND1 (SEQ ID NO: 129). In particular, the biomarker is PDE8A (SEQ ID NO: 100).

The multiple myeloma patient may be responsive to APO010 if the expression level of one or more of the biomarkers of sensitivity is similar (e.g., substantially similar) to the expression level of the biomarkers of sensitivity in a cell or tissue known to be sensitive to APO010. The multiple myeloma patient may also be responsive to APO010 if the expression level of one or more of the biomarkers of resistance is dissimilar (e.g., substantially dissimilar) to the expression level of the biomarkers of resistance in a cell or tissue known to be resistant to APO010. If the patient is predicted to be responsive, then the patient can be administered APO010, such as APO010 administered intravenously at a dose of about 2 $\mu g/m^2$ to 500 $\mu g/m^2$ (e.g., about 45 $\mu g/m^2$ to 200 $\mu g/m^2$). Conversely, if the patient is predicted to be non-responsive to APO010 treatment, then the patient can be administered one or more therapies other than APO010, such as radiation or a therapeutic agent (e.g., a histone deacetylase (HDAC) inhibitor, ipilimumab, bortezomib, carfilzomib, thalidomide, lenalidomide, pomalidomide, prednisone, dexamethasone, cyclophosphamide, vincristine, doxorubicin, melphalan, and/or another therapeutic agent described herein).

**Example 5. Predicting responsiveness of breast cancer patients to APO010.**

The diagnostic methods of the present invention can be used to predict the responsiveness of a breast cancer patient to treatment with APO010. In particular, the breast cancer patient may be one that has not previously received any cancer treatment or one that has received a cancer treatment other than APO010. Moreover, the patient may be one diagnosed with breast cancer or with recurrence of breast cancer.

A biological sample (e.g., a breast tissue sample, such as a breast tissue sample obtained by biopsy) may be obtained from the patient through methods well known in the art. The sample may be frozen and/or prepared, e.g., by formalin fixation and paraffin embedding. In particular, mRNA can be isolated from the sample and a gene expression profile can be determined, e.g., using a microarray platform, such as the Affymetrix HG-U133A, for one or more of the biomarkers shown in Tables 1 and 2. One or more of the biomarkers shown in Tables 1 and 2 can also be measured, e.g., by sequencing or PCR-based techniques, such as those described herein.

For example, the expression level of one or more biomarkers of sensitivity to APO010 can be determined in the sample from the patient, such as one or more of CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), and ITGA4 (SEQ ID NO: 36). In particular, the biomarker is CORO1A (SEQ ID NO: 2). The expression level of one or more biomarkers of resistance to APO010 can also be determined in the sample from the patient, such as one or more of PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and CCND1 (SEQ ID NO: 129). In particular, the biomarker is PDE8A (SEQ ID NO: 100).

The breast cancer patient may be responsive to APO010 if the expression level of the one or more of biomarkers of sensitivity is similar (e.g., substantially similar) to the expression level of the biomarkers of sensitivity in a cell or tissue known to be sensitive to APO010. The breast cancer patient may also be responsive to APO010 if the expression level of one or more of the biomarkers of resistance is dissimilar (e.g., substantially dissimilar) to the expression level of the biomarkers of resistance in a cell or tissue known to be resistant to APO010. If the patient is predicted to be responsive, then the patient can be administered APO010, such as APO010 administered intravenously at a dose of about 2 $\mu g/m^2$ to 500 $\mu g/m^2$ (e.g., about 45 $\mu g/m^2$ to 200 $\mu g/m^2$). Conversely, if the patient is predicted to be non-responsive to APO010 treatment, then the patient can be administered one or more therapies other than APO010, such as radiation or a therapeutic agent (e.g., doxorubicin, epirubicin, paclitaxel, docetaxel, 5-fluorouracil (5-FU), cyclophosphamide, carboplatin, cisplatin, vinorelbine, gemcitabine, mitoxantrone, ixabepilone, eribulin, irinotecan, capecitabine, tegafur, oxaliplatin, and/or another therapeutic agent described herein).

## OTHER EMBODIMENTS

All publications, patents, and patent applications mentioned in the above specification are hereby incorporated by reference. Various modifications and variations of the described device and methods of use of the invention will be apparent to those skilled in the art without departing from the scope and spirit of the invention. Although the invention has been described in connection with specific embodiments, it should be understood that the invention as claimed should not be unduly limited to such specific embodiments. Indeed, various modifications of the described modes for carrying out the invention that are obvious to those skilled in the art are intended to be within the scope of the invention. For example, it is anticipated that measuring the level of proteins, metabolites, identifying genetic mutations and DNA copy number variations, all will be useful in determining patient responsiveness.

**Claims**

1. A method for detecting gene expression of a biomarker in a patient with cancer or having recurrence of cancer comprising:

    (a) contacting a sample from the patient comprising one or more nucleic acid molecules with:

        i) one or more single-stranded nucleic acid molecules capable of specifically hybridizing with the nucleotides of one or more biomarkers of sensitivity selected from the biomarkers of Table 1; and/or
        ii) one or more single-stranded nucleic acid molecules capable of specifically hybridizing with the nucleotides of one or more biomarkers of resistance selected from the biomarkers of Table 2; and

    (b) detecting a level of expression of one or more of the biomarkers of sensitivity and/or one or more of the biomarkers of resistance.

2. The method of claim 1, wherein the method is for determining responsiveness of the cancer of the patient to APO010.

3. The method of claim 1 or 2, wherein:

    a) the method is performed using a device comprising the one or more single-stranded nucleic acid molecules, preferably wherein the device comprises at least two, at least three, at least four, at least five, at least six, at least seven, at least eight, at least nine, at least ten, or more single-stranded nucleic acid molecules of i) and/or ii) or wherein the device is a microarray, such as a deoxyribonucleic acid (DNA)-based platform; and/or
    b) step (b) comprises measuring hybridization between the one or more nucleic acid molecules from the patient and the single-stranded nucleic acid molecules to detect a level of expression of one or more of the biomarkers of sensitivity and/or one or more of the biomarkers of resistance; preferably wherein:

        - the patient is determined to be responsive to APO010 if:

            i) the level of expression of the biomarkers of sensitivity is substantially similar to the level of expression of the biomarkers of sensitivity in a cell or tissue known to be sensitive to APO010; and/or
            ii) the level of expression of the biomarkers of resistance is substantially dissimilar to the level of expression of the biomarkers of resistance in a cell or tissue known to be resistant to APO010; or

- the method comprises performing microarray analysis or quantitative reverse transcriptase polymerase chain reaction (qRT-PCR); and/or

c) steps (a) and (b) occur multiple times; and/or
d) the method comprises converting the level of expression of one or more of the biomarkers of sensitivity and/or one or more of the biomarkers of resistance into a mean score, wherein the mean score indicates the responsiveness of the patient to APO010, wherein preferably the method further comprises subtracting the mean score for one or more of the biomarkers of resistance from the mean score for one or more of the biomarkers of sensitivity to obtain a difference score, wherein the difference score indicates the responsiveness of the patient to APO010.

**4.** The method of claim 3, wherein the mean score and/or the difference score above a cutoff value indicates that the patient is responsive to APO010, wherein preferably the cutoff value is about 20, about 40, about 60, or about 80.

**5.** The method of any one of claims 1 to 4, wherein one or more of the single-stranded nucleic acid molecules have a length in the range of 10 to 100 nucleotides in length, preferably wherein one or more of the single-stranded nucleic acid molecules have a length in the range of 20 to 60 nucleotides.

**6.** The method of any one of claims 1 to 5, wherein:

a) the biomarker of sensitivity is selected from one or more of CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), SLA (SEQ ID NO: 11), DENND2D (SEQ ID NO: 12), CYFIP2 (SEQ ID NO: 13 or 98), PSME2 (SEQ ID NO: 14), PTPRCAP (SEQ ID NO: 15), UBE2L6 (SEQ ID NO: 18), AIF1 (SEQ ID NO: 20), PSMB9 (SEQ ID NO: 21), TRIM22 (SEQ ID NO: 22), BIN2 (SEQ ID NO: 23), CD247 (SEQ ID NO: 24), APOL3 (SEQ ID NO: 25), PTPRC (SEQ ID NO: 26), CD53 (SEQ ID NO: 27), SELPLG (SEQ ID NO: 28), LAIR1 (SEQ ID NO: 29), PSME1 (SEQ ID NO: 30), MYC (SEQ ID NO: 31), LCP1 (SEQ ID NO: 32), ZAP70 (SEQ ID NO: 33), SMARCA4 (SEQ ID NO: 34), PTPN7 (SEQ ID NO: 35), and ITGA4 (SEQ ID NO: 36); preferably wherein the biomarkers of sensitivity comprise:

i) CORO1A (SEQ ID NO: 2) and CD47 (SEQ ID NO: 1);
ii) CORO1A (SEQ ID NO: 2), CD47 (SEQ ID NO: 1), and ICAM3 (SEQ ID NO: 3);
iii) CORO1A (SEQ ID NO: 2), CD47 (SEQ ID NO: 1), ICAM3 (SEQ ID NO: 3), and HCLS1 (SEQ ID NO: 4);
iv) CORO1A (SEQ ID NO: 2), CD47 (SEQ ID NO: 1), ICAM3 (SEQ ID NO: 3), HCLS1 (SEQ ID NO: 4), and DOCK2 (SEQ ID NO 5);
v) CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), and ARHGAP15 (SEQ ID NO: 6);
vi) CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), and CXCR4 (SEQ ID NO: 7 or 9 or 16);
vii) CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), and NCKAP1L (SEQ ID NO: 8);
viii) CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), and MAP4K1 (SEQ ID NO: 10 or 17 or 19); or
ix) CORO1A (SEQ ID NO: 2), CD47 (SEQ ID NO: 1), ICAM3 (SEQ ID NO: 3), HCLS1 (SEQ ID NO: 4), DOCK2 (SEQ ID NO 5), ARHGAP15 (SEQ ID NO: 6), CXCR4 (SEQ ID NO: 7 or 9 or 16), NCKAP1L (SEQ ID NO: 8), MAP4K1 (SEQ ID NO: 10 or 17 or 19), and SLA (SEQ ID NO: 11); and/or

b) the biomarker of resistance is selected from one or more of PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), ANXA2 (SEQ ID NO: 111 or 116 or 122), MRPL40 (SEQ ID NO: 112), TJP1 (SEQ ID NO: 113), SERPINB6 (SEQ ID NO: 114), ALDH7A1 (SEQ ID NO: 115), SOGA2 (SEQ ID NO: 117), G6PD (SEQ ID NO: 118), RHOBTB3 (SEQ ID NO: 119), PPP4R1 (SEQ ID NO: 120), ADARB1 (SEQ ID NO: 123), KIAA0355 (SEQ ID NO: 124), RFC4 (SEQ ID NO: 125), MAFF (SEQ ID NO: 126), FOXK2 (SEQ ID NO: 128), and CCND1 (SEQ ID NO: 129), preferably wherein the biomarkers of resistance comprise:

i) PDE8A (SEQ ID NO: 100) and HOXB7 (SEQ ID NO: 101 or 103);
ii) PDE8A (SEQ ID NO: 100), HOXB7 (SEQ ID NO: 101 or 103), and PRC1 (SEQ ID NO: 102).
iii) PDE8A (SEQ ID NO: 100), HOXB7 (SEQ ID NO: 101 or 103), PRC1 (SEQ ID NO: 102), and SCRN1 (SEQ ID NO: 104);
iv) PDE8A (SEQ ID NO: 100), HOXB7 (SEQ ID NO: 101 or 103), PRC1 (SEQ ID NO: 102), SCRN1 (SEQ ID NO: 104), and ACTN1 (SEQ ID NO: 105 or 109);
v) PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), and NGRN (SEQ ID NO: 106);
vi) PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), and DKK1 (SEQ ID NO: 107);
vii) PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), and IER3 (SEQ ID NO: 108);
viii) PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), and NQO1 (SEQ ID NO: 110 or 121 or 127); or
ix) PDE8A (SEQ ID NO: 100), HOXB7 (SEQ ID NO: 101 or 103), PRC1 (SEQ ID NO: 102), SCRN1 (SEQ ID NO: 104), ACTN1 (SEQ ID NO: 105 or 109), NGRN (SEQ ID NO: 106), DKK1 (SEQ ID NO: 107), IER3 (SEQ ID NO: 108), NQO1 (SEQ ID NO: 110 or 121 or 127), and ANXA2 (SEQ ID NO: 111 or 116 or 122).

**7.** The method of any one of claims 1 to 6, wherein the level of expression of the biomarkers of sensitivity and/or biomarkers of resistance is determined by detecting the level of mRNA transcribed from a gene coding one or more of the biomarkers of Tables 1 and/or 2.

**8.** The method of any one of claims 1 to 7, wherein the cancer is selected from a solid tumor cancer and a hematological cancer or wherein the cancer is selected from the group consisting of
multiple myeloma, breast cancer, acute myelogenous leukemia (AML), acute lympho-blastic leukemia (ALL), chronic lymphocytic leukemia (CLL), myelodysplastic syndrome (MDS), chronic myelogenous leukemia - chronic phase (CMLCP), diffuse large B-cell lymphoma (DLBCL), cutaneous T-cell lymphoma (CTCL), peripheral T-cell lymphoma (PTCL), Hodgkin's lymphoma, hepatocellular carcinoma (HCC), cervical cancer, prostate cancer, renal cell carcinoma (RCC), esophageal cancer, melanoma, glioma, pancreatic cancer, ovarian cancer, gastrointestinal stromal tumors (GIST), sarcoma, estrogen receptor-positive (ERpos) breast cancer, non-small cell lung carcinoma (NSCLC), colon cancer, bladder cancer, and squamous cell carcinoma of the head and neck (SCCHN), wherein preferably the cancer is multiple myeloma or breast cancer.

**9.** The method of any one of claims 1 to 8, wherein:

- the sample is a tumor sample; and/or
- the patient has recurrence of cancer; and/or
- the patient is a human; and/or
- steps (a) and (b) occur prior to, concurrent with, or after the patient has been administered APO010.

**10.** A composition comprising APO010 for use in treating a patient with cancer that has been determined to be responsive to APO010 according to the method of any one of the claims 1 to 9, preferably wherein the cancer of the patient is determined to be responsive to the composition if:

i) the level of expression of the biomarkers of sensitivity is substantially similar to the level of expression of the biomarkers of sensitivity in a cell or tissue known to be sensitive to APO010; and/or
ii) the level of expression of the biomarkers of resistance is substantially dissimilar to the level of expression of the biomarkers of resistance in a cell or tissue known to be resistant to APO010.

**11.** The composition for use according to claim 10, wherein the composition is formulated for administration to the patient:

a) two or more times, preferably wherein the composition is formulated for administration to the patient one or more times daily, weekly, every two weeks, every three weeks, or monthly or once daily for up to six days; and/or
b) in two doses, wherein a second dose is formulated for administration to the patient two weeks, three weeks, four weeks, or five weeks after administration of a first dose;
c) in dosage form; and/or

d) at a dose of about 2 $\mu$g/m$^2$ to 500 $\mu$g/m$^2$, preferably wherein the dose is about 45 $\mu$g/m$^2$ to 200 $\mu$g/m$^2$.

**12.** The composition for use according to claim 10 or 11, wherein the composition is formulated for administration in combination with one or more additional therapies, preferably wherein:

a) the additional therapies comprise surgery, radiation, or a therapeutic agent, preferably wherein the therapeutic agent is selected from the group consisting of a histone deacetylase (HDAC) inhibitor, ipilimumab, bortezomib, carfilzomib, thalidomide, lenalidomide, pomalidomide, prednisone, dexamethasone, cyclophosphamide, vincristine, doxorubicin, melphalan, capecitabine, tegafur, irinotecan, oxaliplatin, cetuximab, leucovorin, SN-38, everolimus, temsirolimus, bleomycin, lomustine, depsipeptide, carboplatin, erlotinib, gemcitabine, mitoxantrone, cisplatin, busulfan, epirubicin, arsenic trioxide, bendamustine, fulvestrant, teniposide, adriamycin, decitabine, estramustine, etoposide, azaguanine, aclarubicin, mitoxantrone, mitomycin, paclitaxel, taxotere, Irofulven, 5-FU, ara-c, methylprednisolone, methotrexate, methyl-gag, belinostat, carboplatin, idarubicin, IL4-PR38, valproic acid, all-trans retinoic acid (ATRA), cytoxan, topotecan, suberoylanilide hydroxamic acid, leukeran, fludarabine, vinblastine, dacarbazine, hydroxyurea, tegafur, daunorubicin, mechlorethamine, streptozocin, carmustine, mercaptopurine, dactinomycin, tretinoin, ifosfamide, tamoxifen, floxuridine, thioguanine, PSC 833, herceptin, bevacizumab, celecoxib, iressa, anastrozole, letrozole, and rituximab, preferably wherein the therapeutic agent is formulated for administration parenterally, enterally, or topically, such as intravenously, intramuscularly, transdermally, intradermally, intra-arterially, intracranially, subcutaneously, intraorbitally, intraventricularly, intraspinally, intraperitoneally, or intranasally (e.g., by injection); or
b) the additional therapies are formulated for administration to the patient prior to, concurrently with, or after administration of APO010.

**13.** A composition comprising a cancer therapy other than APO010 for use in treating a patient with cancer that has been determined to be non-responsive to APO010 according to the method of any one of the claims 1 to 9, preferably wherein the cancer of the patient is determined to be responsive to the composition if:

i) the level of expression of the biomarkers of sensitivity is substantially dissimilar to the level of expression of the biomarkers of sensitivity in a cell or tissue known to be sensitive to APO010; and/or
ii) the level of expression of the biomarkers of resistance is substantially similar to the level of expression of the biomarkers of resistance in a cell or tissue known to be resistant to APO010.

**14.** The composition for use according to claim 13, wherein the cancer therapy is surgery, radiation, or a therapeutic agent, preferably wherein the therapeutic agent is selected from the group consisting of a histone deacetylase (HDAC) inhibitor, ipilimumab, bortezomib, carfilzomib, thalidomide, lenalidomide, pomalidomide, prednisone, dexamethasone, cyclophosphamide, vincristine, doxorubicin, melphalan, capecitabine, tegafur, irinotecan, oxaliplatin, cetuximab, leucovorin, SN-38, everolimus, temsirolimus, bleomycin, lomustine, depsipeptide, carboplatin, erlotinib, gemcitabine, mitoxantrone, cisplatin, busulfan, epirubicin, arsenic trioxide, bendamustine, fulvestrant, teniposide, adriamycin, decitabine, estramustine, etoposide, azaguanine, aclarubicin, mitoxantrone, mitomycin, paclitaxel, taxotere, Irofulven, 5-FU, ara-c, methylprednisolone, methotrexate, methyl-gag, belinostat, carboplatin, idarubicin, IL4-PR38, valproic acid, all-trans retinoic acid (ATRA), cytoxan, topotecan, suberoylanilide hydroxamic acid, leukeran, fludarabine, vinblastine, dacarbazine, hydroxyurea, tegafur, daunorubicin, mechlorethamine, streptozocin, carmustine, mercaptopurine, dactinomycin, tretinoin, ifosfamide, tamoxifen, floxuridine, thioguanine, PSC 833, herceptin, bevacizumab, celecoxib, iressa, anastrozole, letrozole, and rituximab.

**15.** The composition for use according to any one of claims 10 to 14, wherein the cancer is selected from a solid tumor cancer and a hematological cancer or wherein the cancer is selected from the group consisting of multiple myeloma, breast cancer, acute myelogenous leukemia (AML), acute lympho-blastic leukemia (ALL), chronic lymphocytic leukemia (CLL), myelodysplastic syndrome (MDS), chronic myelogenous leukemia - chronic phase (CMLCP), diffuse large B-cell lymphoma (DLBCL), cutaneous T-cell lymphoma (CTCL), peripheral T-cell lymphoma (PTCL), Hodgkin's lymphoma, hepatocellular carcinoma (HCC), cervical cancer, prostate cancer, renal cell carcinoma (RCC), esophageal cancer, melanoma, glioma, pancreatic cancer, ovarian cancer, gastrointestinal stromal tumors (GIST), sarcoma, estrogen receptor-positive (ERpos) breast cancer, non-small cell lung carcinoma (NSCLC), colon cancer, bladder cancer, and squamous cell carcinoma of the head and neck (SCCHN), wherein preferably the cancer is multiple myeloma or breast cancer.

FIG. 1

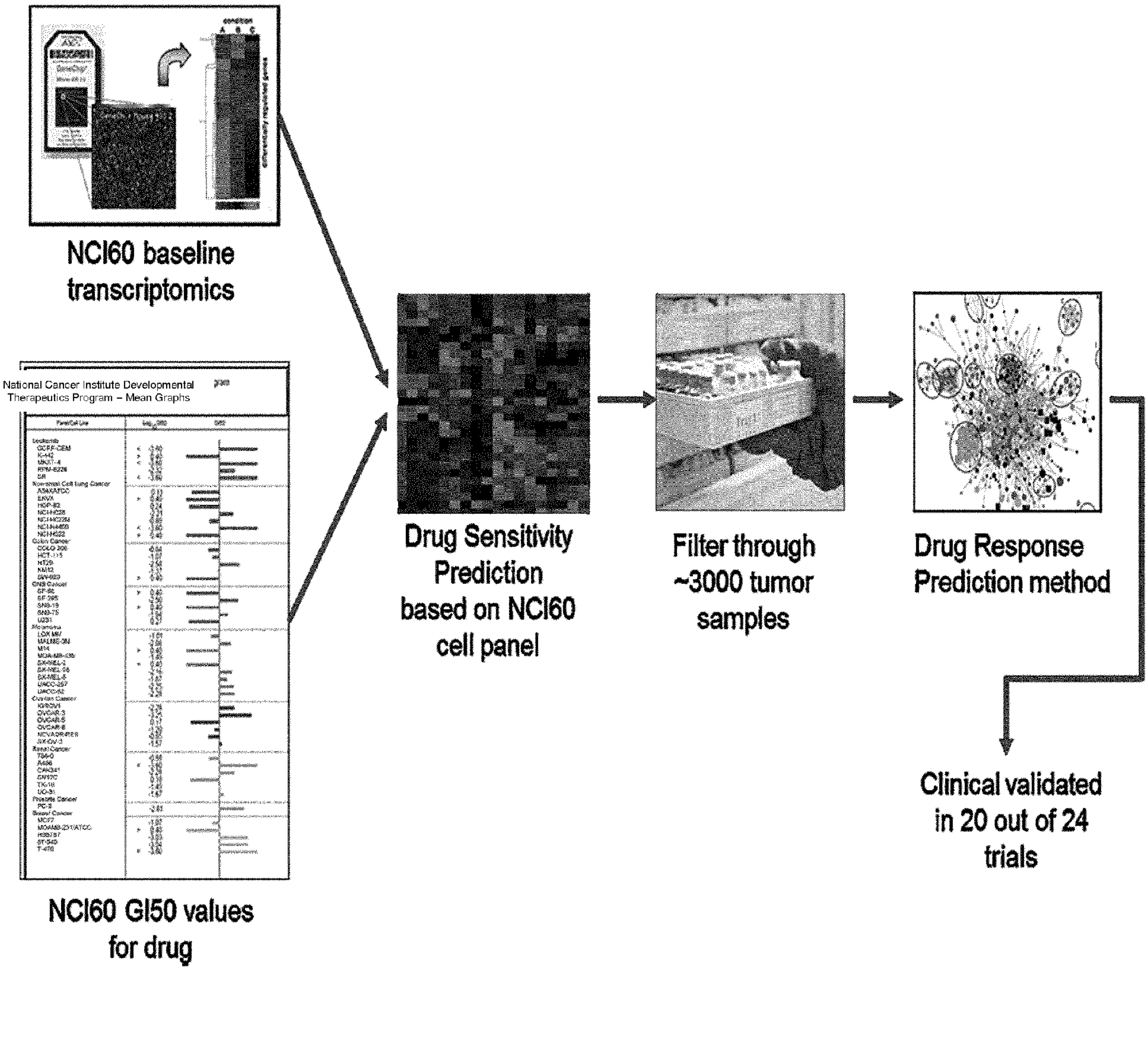
NCI60 baseline transcriptomics
National Cancer Institute Developmental Therapeutics Program – Mean Graphs
NCI60 GI50 values for drug
Drug Sensitivity Prediction based on NCI60 cell panel
Filter through ~3000 tumor samples
Drug Response Prediction method
Clinical validated in 20 out of 24 trials

FIG. 2

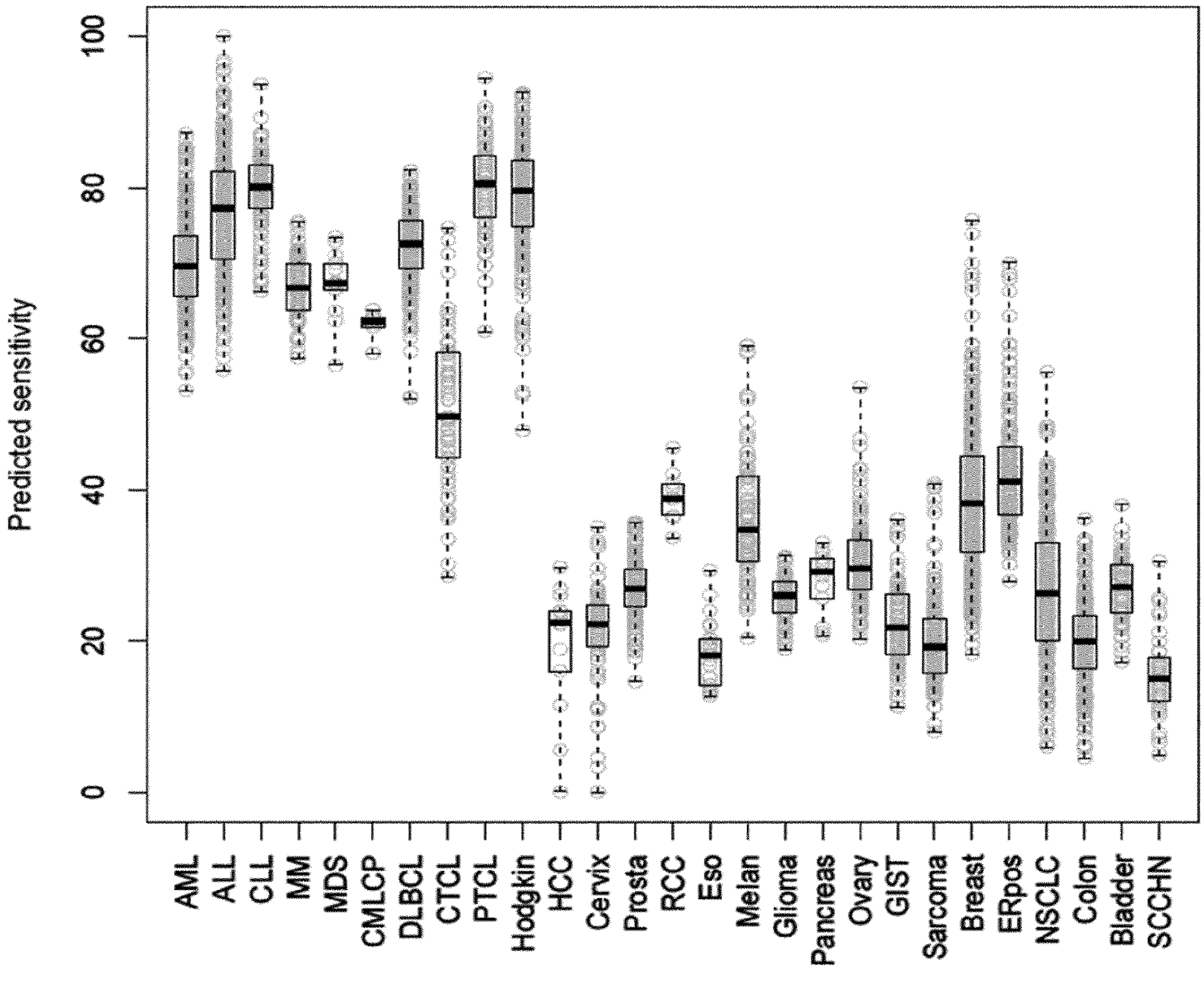
Predicted sensitivity
0
20
40
60
80
100
AML
ALL
CLL
MM
MDS
CMLCP
DLBCL
CTCL
PTCL
Hodgkin
HCC
Cervix
Prosta
RCC
Eso
Melan
Glioma
Pancreas
Ovary
GIST
Sarcoma
Breast
ERpos
NSCLC
Colon
Bladder
SCCHN

FIG. 3

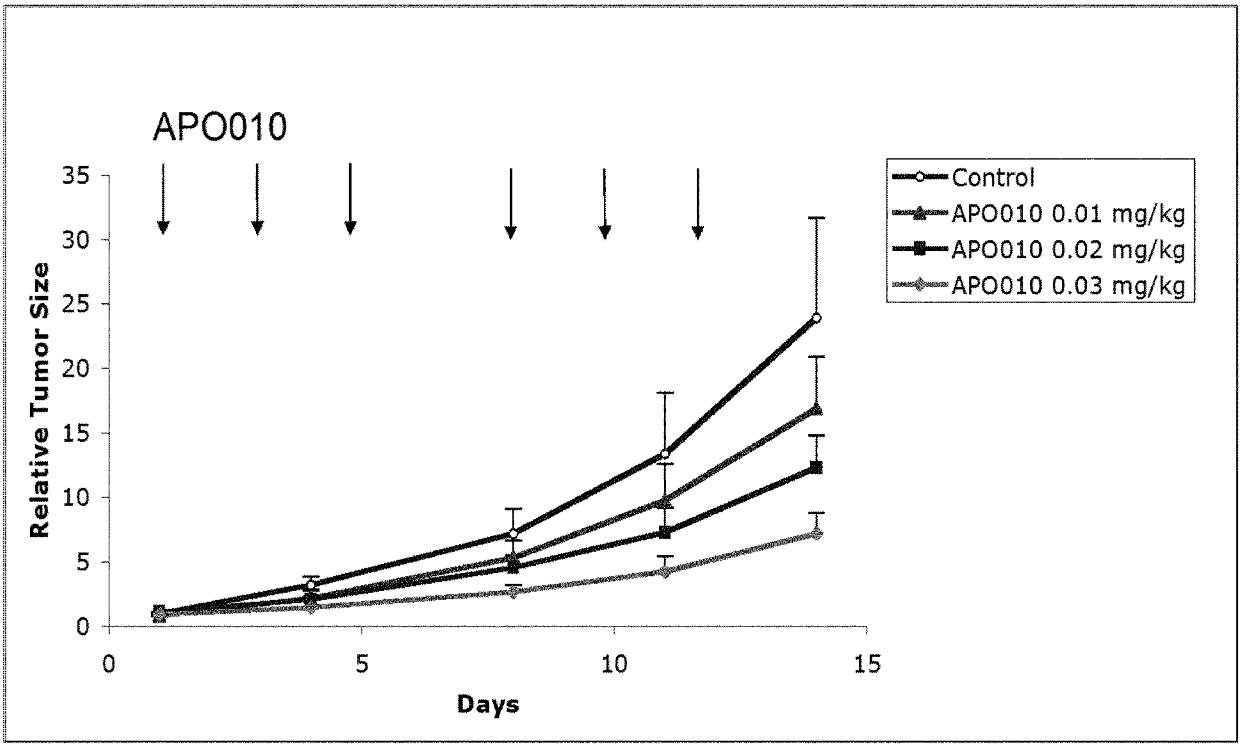
APO010
35
30
25
20
15
10
5
0
Relative Tumor Size
0
5
10
15
Days
Control
APO010 0.01 mg/kg
APO010 0.02 mg/kg
APO010 0.03 mg/kg

FIG. 4

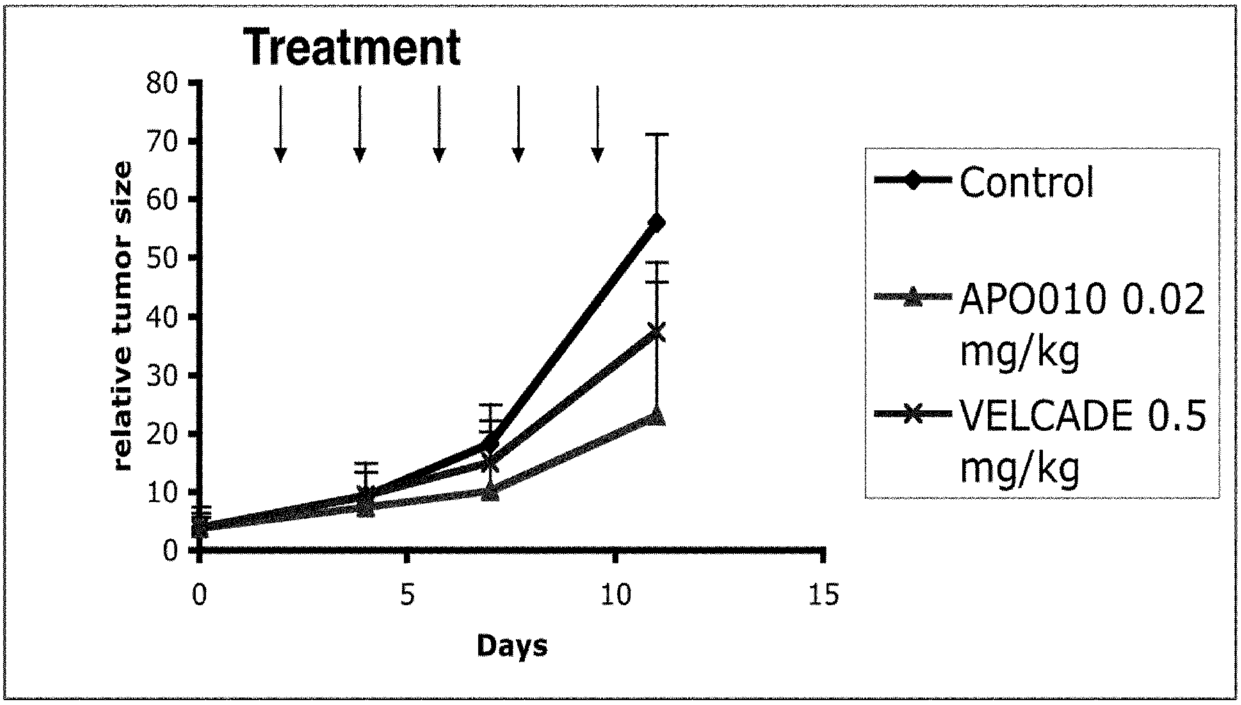
Treatment
relative tumor size
0
10
20
30
40
50
60
70
80
0
5
10
15
Days
Control
APO010 0.02 mg/kg
VELCADE 0.5 mg/kg

FIG. 5

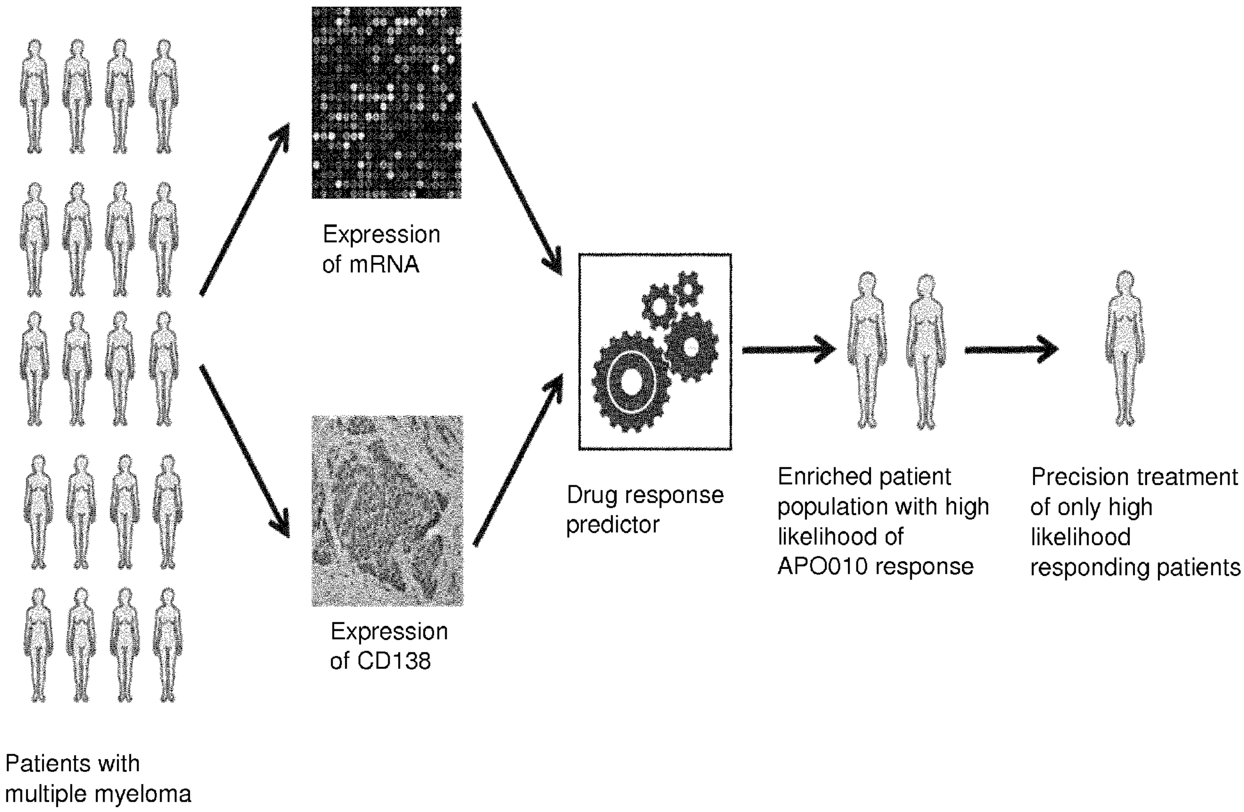
Expression
of mRNA
Expression
of CD138
Drug response
predictor
Enriched patient
population with high
likelihood of
APO010 response
Precision treatment
of only high
likelihood
responding patients
Patients with
multiple myeloma

Europäisches Patentamt
European Patent Office
Office européen des brevets

# EUROPEAN SEARCH REPORT

Application Number
EP 17 19 3243

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Enrique Ocio ET AL: "The Activation of Fas Receptor by APO010, a Recombinant Form of Fas Ligand, Induces In Vitro and In Vivo Antimyeloma Activity", Blood, vol. 110, 1 January 2007 (2007-01-01), page 1515, XP055444374, Retrieved from the Internet: URL:http://www.bloodjournal.org/content/110/11/1515 [retrieved on 2018-01-25] * abstract * | 1-15 | INV. C12Q1/6886 |
| A | W. WANG ET AL: "Independent Validation of a Model Using Cell Line Chemosensitivity to Predict Response to Therapy", JOURNAL OF THE NATIONAL CANCER INSTITUTE, vol. 105, no. 17, 20 August 2013 (2013-08-20), pages 1284-1291, XP055253160, GB ISSN: 0027-8874, DOI: 10.1093/jnci/djt202 * the whole document * | 1-15 | TECHNICAL FIELDS SEARCHED (IPC): C12Q |
| A | T NARITA ET AL: "Lower expression of activating transcription factors 3 and 4 correlates with shorter progression-free survival in multiple myeloma patients receiving bortezomib plus dexamethasone therapy", LEUKEMIA., vol. 5, no. 12, 1 December 2015 (2015-12-01), pages e373-e373, XP055444430, US ISSN: 0887-6924, DOI: 10.1038/bcj.2015.98 * abstract * | 1-15 | |

-/--

The present search report has been drawn up for all claims

1

| Place of search | Date of completion of the search | Examiner |
|---|---|---|
| The Hague | 25 January 2018 | Aguilera, Miguel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 17 19 3243

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MICHAEL MEDINGER ET AL: "Gene-expression Profiling in Patients with Plasma Cell Myeloma Treated with Novel Agents", CANCER GENOMICS & PROTEOMICS, vol. 13, 1 August 2016 (2016-08-01), pages 275-280, XP055444435, * abstract * ----- | 1-15 | |
| A | JEANNETTE GERSPACH ET AL: "Therapeutic Targeting of CD95 and the TRAIL Death Receptors", RECENT PATENTS ON ANTI-CANCER DRUG DISCOVERY, vol. 6, no. 3, 1 September 2011 (2011-09-01), pages 294-310, XP055444414, GB ISSN: 1574-8928, DOI: 10.2174/157489211796957739 * the whole document * ----- | 1-15 | |
| X,P | A.J. VANGSTED ET AL: "APO010 sensitivity in relapsed multiple myeloma patients", ANNALS OF ONCOLOGY., vol. 27, no. suppl_6, 1 October 2016 (2016-10-01), XP055444417, NL ISSN: 0923-7534, DOI: 10.1093/annonc/mdw363.82 * the whole document * ----- -/-- | 1-15 | TECHNICAL FIELDS SEARCHED (IPC) |

The present search report has been drawn up for all claims

1

| Place of search | Date of completion of the search | Examiner |
|---|---|---|
| The Hague | 25 January 2018 | Aguilera, Miguel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 17 19 3243

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | A.J. VANGSTED ET AL: "APO010 sensitivity in relapsed multiple myeloma patients", ANNALS OF ONCOLOGY., vol. 27, no. suppl_6, 1 October 2016 (2016-10-01), XP055444420, NL ISSN: 0923-7534, DOI: 10.1093/annonc/mdw363.82 * abstract * ----- | 1-15 | |

TECHNICAL FIELDS SEARCHED (IPC)

The present search report has been drawn up for all claims

1

| Place of search | Date of completion of the search | Examiner |
|---|---|---|
| The Hague | 25 January 2018 | Aguilera, Miguel |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20110201515 A
- US 20110229888 A
- US 20130017971 A
- US 5822715 A
- US 20030073083 A
- US 20050227266 A
- US 20050208512 A
- US 20050123945 A
- US 20030129629 A
- US 20020006613 A


### Non-patent literature cited in the description


- **EISELE et al.** *Neuro. Oncol.,* 2010, vol. 13, 155-164
- **MORTAZAVI et al.** *Nat. Methods,* 2008, vol. 5, 621-628
- **CHEN et al.** *Mol. Cancer Ther.,* 2012, vol. 11, 34-33
- **WANG et al.** *J. Nat. Cancer Inst.,* 2013, vol. 105, 1284-1291
- **KNUDSEN et al.** *PLoS One,* 2014, vol. 9, e87415
- **VAN'T VEER et al.** *Nature,* 2002, vol. 415 (6871), 530-536
- **VAPNIK V N.** Statistical Learning Theory. John Wiley & Sons, 1998
- The Elements of Statistical Learning: Data Mining. **HASTIE et al.** Inference, and Prediction. Springer, 2001
- **AGRESTI.** An Introduction to Categorical Data Analysis. John Wiley & Sons, 1996
- Neural Network Modeling of Physiological Processes. **V. TRESP et al.** Computational Learning Theory and Natural Learning Systems. MIT Press, 1994, vol. 2
- Handbook of Pharmaceutical Excipients. Pharmaceutical Press, 2009

SEQUENCE LISTING

<110> Oncology Venture ApS

<120> METHODS FOR PREDICTING DRUG RESPONSIVENESS IN CANCER PATIENTS

<130> P071225EP

<150> US15/277,750
<151> 2016-09-27

<160> 169

<170> PatentIn version 3.5

<210> 1
<211> 25
<212> DNA
<213> Homo sapiens

<400> 1
gcggcgtgta taccaatgca tggcc 25

<210> 2
<211> 25
<212> DNA
<213> Homo sapiens

<400> 2
gctccagaag cgcttggaca ggctg 25

<210> 3
<211> 25
<212> DNA
<213> Homo sapiens

<400> 3
agcgtccagc tcacgaggca aatac 25

<210> 4
<211> 25
<212> DNA
<213> Homo sapiens

<400> 4
ctgtctactg caactgtgat ttccc 25

<210> 5
<211> 25
<212> DNA
<213> Homo sapiens

<400> 5
gattcctgaa ctcaaggtac cagca 25

<210> 6
<211> 25
<212> DNA

<213> Homo sapiens

<400> 6
tggcgatcca catggtctac cagaa 25

<210> 7
<211> 25
<212> DNA
<213> Homo sapiens

<400> 7
gtggtctatg ttggcgtctg gatcc 25

<210> 8
<211> 25
<212> DNA
<213> Homo sapiens

<400> 8
cctgcttcga aatgcctatc gggag 25

<210> 9
<211> 25
<212> DNA
<213> Homo sapiens

<400> 9
gccaaattta aaacctctgc ccagc 25

<210> 10
<211> 25
<212> DNA
<213> Homo sapiens

<400> 10
gagacacgcc cagtggatga tccta 25

<210> 11
<211> 25
<212> DNA
<213> Homo sapiens

<400> 11
aagctcatcg tgctactggt atgtg 25

<210> 12
<211> 25
<212> DNA
<213> Homo sapiens

<400> 12
ggtgtctgag atttggatcc ctggt 25

<210> 13
<211> 25
<212> DNA

<213> Homo sapiens

<400> 13
gacagacagt tccactgtgg agcat 25

<210> 14
<211> 25
<212> DNA
<213> Homo sapiens

<400> 14
gagagggtga atgccgtcaa gacca 25

<210> 15
<211> 25
<212> DNA
<213> Homo sapiens

<400> 15
cactgtagag gccggtcttg gtgtc 25

<210> 16
<211> 25
<212> DNA
<213> Homo sapiens

<400> 16
gtatgtctcg tggtaggact gtaga 25

<210> 17
<211> 25
<212> DNA
<213> Homo sapiens

<400> 17
gtgtcaggaa ctagtccttg caccc 25

<210> 18
<211> 25
<212> DNA
<213> Homo sapiens

<400> 18
gaaaccatgt tcttgcttaa gccat 25

<210> 19
<211> 25
<212> DNA
<213> Homo sapiens

<400> 19
tctaggctcg gatcagctgc tacag 25

<210> 20
<211> 25
<212> DNA

<213> Homo sapiens

<400> 20
ttcagctacc ctgactttct cagga 25

<210> 21
<211> 25
<212> DNA
<213> Homo sapiens

<400> 21
gatgggttct gattcccgag tgtct 25

<210> 22
<211> 25
<212> DNA
<213> Homo sapiens

<400> 22
tcctctgccc cttaaaagat tgaag 25

<210> 23
<211> 25
<212> DNA
<213> Homo sapiens

<400> 23
ataagcttat ctcagctgac tcctc 25

<210> 24
<211> 25
<212> DNA
<213> Homo sapiens

<400> 24
tgccgcacca ttgaactgta ccatg 25

<210> 25
<211> 25
<212> DNA
<213> Homo sapiens

<400> 25
gttagaactt ttggatacag cagaa 25

<210> 26
<211> 25
<212> DNA
<213> Homo sapiens

<400> 26
aagtgtgcag aatactggcc gtcaa 25

<210> 27
<211> 25
<212> DNA

<213> Homo sapiens

<400> 27
agcaagacaa tctttcactc actga 25

<210> 28
<211> 25
<212> DNA
<213> Homo sapiens

<400> 28
ttgtcttttg gttgccatgg tcacc 25

<210> 29
<211> 25
<212> DNA
<213> Homo sapiens

<400> 29
ttctctgggt tgtgctttac tccac 25

<210> 30
<211> 25
<212> DNA
<213> Homo sapiens

<400> 30
tctgcagcgc ttgaagcctg agatc 25

<210> 31
<211> 25
<212> DNA
<213> Homo sapiens

<400> 31
gcaacaaccg aaaatgcacc agccc 25

<210> 32
<211> 25
<212> DNA
<213> Homo sapiens

<400> 32
acccctctgc ttttaactct agaat 25

<210> 33
<211> 25
<212> DNA
<213> Homo sapiens

<400> 33
ggtggtcagg cgtagatcac cagaa 25

<210> 34
<211> 25
<212> DNA

<213> Homo sapiens

<400> 34
gaggaaggct ccgaatccga atctc 25

<210> 35
<211> 25
<212> DNA
<213> Homo sapiens

<400> 35
gggtacaagc tccagaacag taacc 25

<210> 36
<211> 25
<212> DNA
<213> Homo sapiens

<400> 36
gtactatggt tgtccaacac aggcc 25

<210> 37
<211> 25
<212> DNA
<213> Homo sapiens

<400> 37
gtctgggttt gcagatgggt gccct 25

<210> 38
<211> 25
<212> DNA
<213> Homo sapiens

<400> 38
ccctactgtt tcgagcgtct gggca 25

<210> 39
<211> 25
<212> DNA
<213> Homo sapiens

<400> 39
cagagacggc aagcacctac aggac 25

<210> 40
<211> 25
<212> DNA
<213> Homo sapiens

<400> 40
tggagttcgc cagtcgagag cttca 25

<210> 41
<211> 25
<212> DNA

<213> Homo sapiens

<400> 41
gggaacactg ctctcagaca ttaca 25

<210> 42
<211> 25
<212> DNA
<213> Homo sapiens

<400> 42
gtaggcaatg ttgtcctgtt ggcca 25

<210> 43
<211> 25
<212> DNA
<213> Homo sapiens

<400> 43
aaggacagaa ttgcctctcg gaagc 25

<210> 44
<211> 25
<212> DNA
<213> Homo sapiens

<400> 44
gtattctctg acactcttat ttggt 25

<210> 45
<211> 25
<212> DNA
<213> Homo sapiens

<400> 45
gagcgcagaa gcgtctctgg atggc 25

<210> 46
<211> 25
<212> DNA
<213> Homo sapiens

<400> 46
ggctgatttg gaattttgtg ccccc 25

<210> 47
<211> 25
<212> DNA
<213> Homo sapiens

<400> 47
ctacaagtga actccttgcc caggc 25

<210> 48
<211> 25
<212> DNA

<213> Homo sapiens

<400> 48
agcagtgcaa ggatcgcctg tcagt 25

<210> 49
<211> 25
<212> DNA
<213> Homo sapiens

<400> 49
aacaaccaat ccatatgtcc tcatg 25

<210> 50
<211> 25
<212> DNA
<213> Homo sapiens

<400> 50
gtacacaaat ccctatgttg ctata 25

<210> 51
<211> 25
<212> DNA
<213> Homo sapiens

<400> 51
gtcacattgc cattaaactt tcctt 25

<210> 52
<211> 25
<212> DNA
<213> Homo sapiens

<400> 52
ttggtgatcc agctattttt cctgc 25

<210> 53
<211> 25
<212> DNA
<213> Homo sapiens

<400> 53
tcggtcagaa cctcctgtga gacag 25

<210> 54
<211> 25
<212> DNA
<213> Homo sapiens

<400> 54
tttccggggg cttacggagc tggtg 25

<210> 55
<211> 25
<212> DNA

<213> Homo sapiens

<400> 55
gcattgctat gtgggggacc ccagg 25

<210> 56
<211> 25
<212> DNA
<213> Homo sapiens

<400> 56
ggaatgaccc taggagagct gctga 25

<210> 57
<211> 25
<212> DNA
<213> Homo sapiens

<400> 57
acttcatttt cttagcgttt ctgga 25

<210> 58
<211> 25
<212> DNA
<213> Homo sapiens

<400> 58
tctgcttatc cgttagccgt ggtga 25

<210> 59
<211> 25
<212> DNA
<213> Homo sapiens

<400> 59
agctgggagt ggaacctgtc gatta 25

<210> 60
<211> 25
<212> DNA
<213> Homo sapiens

<400> 60
gccggaccta cagagctcat ggtcg 25

<210> 61
<211> 25
<212> DNA
<213> Homo sapiens

<400> 61
ggactacatg acctcctgga gtccc 25

<210> 62
<211> 25
<212> DNA

<213> Homo sapiens

<400> 62
aacctcctgt tagctgttat ttcta 25

<210> 63
<211> 25
<212> DNA
<213> Homo sapiens

<400> 63
ccatgagaca gtcccagaac acggc 25

<210> 64
<211> 25
<212> DNA
<213> Homo sapiens

<400> 64
agatctttcc agaggtccat ggtgg 25

<210> 65
<211> 25
<212> DNA
<213> Homo sapiens

<400> 65
cctcctgctc ctcaagagtg tggtc 25

<210> 66
<211> 25
<212> DNA
<213> Homo sapiens

<400> 66
gttctgtcat gaatactcac aaatt 25

<210> 67
<211> 25
<212> DNA
<213> Homo sapiens

<400> 67
cacccagggc gtggagatga ccacg 25

<210> 68
<211> 25
<212> DNA
<213> Homo sapiens

<400> 68
gtcctctcaa ctctctttgt aaaaa 25

<210> 69
<211> 25
<212> DNA

<213> Homo sapiens

<400> 69
ggatgccgtg ggagtacaaa agtgg 25

<210> 70
<211> 25
<212> DNA
<213> Homo sapiens

<400> 70
gtggacaaat gcgacgaacc tctga 25

<210> 71
<211> 25
<212> DNA
<213> Homo sapiens

<400> 71
aaatgataca ctactgctgc agctc 25

<210> 72
<211> 25
<212> DNA
<213> Homo sapiens

<400> 72
tgtggaaagc ctggatctca gctcc 25

<210> 73
<211> 25
<212> DNA
<213> Homo sapiens

<400> 73
ataccaagta tagcctatgg cagaa 25

<210> 74
<211> 25
<212> DNA
<213> Homo sapiens

<400> 74
tggctaatgt ctctcagtca ttccc 25

<210> 75
<211> 25
<212> DNA
<213> Homo sapiens

<400> 75
tcatcctcta ctggagaaac cccac 25

<210> 76
<211> 25
<212> DNA

<213> Homo sapiens

<400> 76
ttccccaaag ccagaagatg cacaa 25

<210> 77
<211> 25
<212> DNA
<213> Homo sapiens

<400> 77
cctgctaagg gatgcccctt caggc 25

<210> 78
<211> 25
<212> DNA
<213> Homo sapiens

<400> 78
gaacatgtgt ctatctgctt ttgcc 25

<210> 79
<211> 25
<212> DNA
<213> Homo sapiens

<400> 79
agaatgaaag ccttctcaga cctgc 25

<210> 80
<211> 25
<212> DNA
<213> Homo sapiens

<400> 80
tatcgccatc acagctgaac ttgtt 25

<210> 81
<211> 25
<212> DNA
<213> Homo sapiens

<400> 81
tagctctgta gtggaatatg tcttc 25

<210> 82
<211> 25
<212> DNA
<213> Homo sapiens

<400> 82
aagccaatca tttgtaacca ttcta 25

<210> 83
<211> 25
<212> DNA

<213> Homo sapiens

<400> 83
agaagcctgc tttgaaaatt ttcca 25

<210> 84
<211> 25
<212> DNA
<213> Homo sapiens

<400> 84
cttggccgac tcagggacct aagcc 25

<210> 85
<211> 25
<212> DNA
<213> Homo sapiens

<400> 85
tctgcctgaa agccccatga agaaa 25

<210> 86
<211> 25
<212> DNA
<213> Homo sapiens

<400> 86
tctcctttct caccaatggg caata 25

<210> 87
<211> 25
<212> DNA
<213> Homo sapiens

<400> 87
tggacgactg accacaatca actgt 25

<210> 88
<211> 25
<212> DNA
<213> Homo sapiens

<400> 88
aactctggtg acatttgctt tacct 25

<210> 89
<211> 25
<212> DNA
<213> Homo sapiens

<400> 89
gctcacaaac acctctgcat attac 25

<210> 90
<211> 25
<212> DNA

<213> Homo sapiens

<400> 90
gaagcgtctt ctgaggatct agttg 25

<210> 91
<211> 25
<212> DNA
<213> Homo sapiens

<400> 91
aggaccctta ggagttcgat gagag 25

<210> 92
<211> 25
<212> DNA
<213> Homo sapiens

<400> 92
acacagaacg ttacatgggt ctccc 25

<210> 93
<211> 25
<212> DNA
<213> Homo sapiens

<400> 93
tgaatcttcg ggtgtttccc tttag 25

<210> 94
<211> 25
<212> DNA
<213> Homo sapiens

<400> 94
acggcatagc ttaggtgcgc cgtcc 25

<210> 95
<211> 25
<212> DNA
<213> Homo sapiens

<400> 95
ctctggctct ctattgcatt cattg 25

<210> 96
<211> 25
<212> DNA
<213> Homo sapiens

<400> 96
attaaggtca ttactctcaa ccacc 25

<210> 97
<211> 25
<212> DNA

<213> Homo sapiens

<400> 97
atgcatgttt cggggacttg gccct 25

<210> 98
<211> 25
<212> DNA
<213> Homo sapiens

<400> 98
ggtgacatat ttacgcttgt gatca 25

<210> 99
<211> 25
<212> DNA
<213> Homo sapiens

<400> 99
gtgagcttat catgctgtct ttaca 25

<210> 100
<211> 25
<212> DNA
<213> Homo sapiens

<400> 100
gctggggtcg tattctaagt gctaa 25

<210> 101
<211> 25
<212> DNA
<213> Homo sapiens

<400> 101
gcagttttgt aagccctctt taatg 25

<210> 102
<211> 25
<212> DNA
<213> Homo sapiens

<400> 102
ttgcacatgt cactactggg gaggt 25

<210> 103
<211> 25
<212> DNA
<213> Homo sapiens

<400> 103
gtaagccctc tttaatgctg tcttt 25

<210> 104
<211> 25
<212> DNA

<213> Homo sapiens

<400> 104
tcatgtgcac atgccgttgc agcac 25

<210> 105
<211> 25
<212> DNA
<213> Homo sapiens

<400> 105
aactatttgc accgaaatgt cttgt 25

<210> 106
<211> 25
<212> DNA
<213> Homo sapiens

<400> 106
gacctctgta gaccttcagt actca 25

<210> 107
<211> 25
<212> DNA
<213> Homo sapiens

<400> 107
gagtctagaa cgcaaggatc tcttg 25

<210> 108
<211> 25
<212> DNA
<213> Homo sapiens

<400> 108
tgagatccgt gagatccttc catct 25

<210> 109
<211> 25
<212> DNA
<213> Homo sapiens

<400> 109
ccgaccaggc tgagtactgc atcgc 25

<210> 110
<211> 25
<212> DNA
<213> Homo sapiens

<400> 110
gtggcttcca agtcttagaa cctca 25

<210> 111
<211> 25
<212> DNA

<213> Homo sapiens

<400> 111
gaaaatgctt tcctgaacct ggttc 25

<210> 112
<211> 25
<212> DNA
<213> Homo sapiens

<400> 112
tccatgctga ggccatcaag cggga 25

<210> 113
<211> 25
<212> DNA
<213> Homo sapiens

<400> 113
aagtatccct actgtaattt gtgat 25

<210> 114
<211> 25
<212> DNA
<213> Homo sapiens

<400> 114
gatgatgcgg tgtgccagat tcgtc 25

<210> 115
<211> 25
<212> DNA
<213> Homo sapiens

<400> 115
gcaaggttat ggatcgccct ggaaa 25

<210> 116
<211> 25
<212> DNA
<213> Homo sapiens

<400> 116
gagggtgacg ttagcattac cccca 25

<210> 117
<211> 25
<212> DNA
<213> Homo sapiens

<400> 117
atgtatatac cgctactgtg tcctc 25

<210> 118
<211> 25
<212> DNA

<213> Homo sapiens

<400> 118
gtctgtccca gagcttattg gccac 25

<210> 119
<211> 25
<212> DNA
<213> Homo sapiens

<400> 119
ggacagactg atagtttctt tcttt 25

<210> 120
<211> 25
<212> DNA
<213> Homo sapiens

<400> 120
gaggtgcaac ttcttcacat actgt 25

<210> 121
<211> 25
<212> DNA
<213> Homo sapiens

<400> 121
ctttccatca ccactggtgg cagtg 25

<210> 122
<211> 25
<212> DNA
<213> Homo sapiens

<400> 122
gctgatcggc tgtatgactc catga 25

<210> 123
<211> 25
<212> DNA
<213> Homo sapiens

<400> 123
gtttgctttt tcaggctttg gatta 25

<210> 124
<211> 25
<212> DNA
<213> Homo sapiens

<400> 124
gacagaggtc aatccttgat gctcc 25

<210> 125
<211> 25
<212> DNA

<213> Homo sapiens

<400> 125
acccctgacc tctagatgtt caaaa 25

<210> 126
<211> 25
<212> DNA
<213> Homo sapiens

<400> 126
agcaagcgag ttatcgtctt ctgta 25

<210> 127
<211> 25
<212> DNA
<213> Homo sapiens

<400> 127
actgccctct tgtggtgcat tgaaa 25

<210> 128
<211> 25
<212> DNA
<213> Homo sapiens

<400> 128
agactttcag aatcacacag gccct 25

<210> 129
<211> 25
<212> DNA
<213> Homo sapiens

<400> 129
gacgcgcaag tctgagggtc tgggc 25

<210> 130
<211> 25
<212> DNA
<213> Homo sapiens

<400> 130
gatggtattc attaggtttt aactg 25

<210> 131
<211> 25
<212> DNA
<213> Homo sapiens

<400> 131
gagacaaaat ctctatactg ccctg 25

<210> 132
<211> 25
<212> DNA

<213> Homo sapiens

<400> 132
gatgagcgtc ctaaggcatg ttggg 25

<210> 133
<211> 25
<212> DNA
<213> Homo sapiens

<400> 133
aactgggatc gcacctttta tacca 25

<210> 134
<211> 25
<212> DNA
<213> Homo sapiens

<400> 134
gctttgggct ctgctatttg tttgc 25

<210> 135
<211> 25
<212> DNA
<213> Homo sapiens

<400> 135
gaggaactca ttgtcacttt cagaa 25

<210> 136
<211> 25
<212> DNA
<213> Homo sapiens

<400> 136
gcagatgagc accgtgtcca gtgcc 25

<210> 137
<211> 25
<212> DNA
<213> Homo sapiens

<400> 137
acgtcttggt gccttttgtg tgatg 25

<210> 138
<211> 25
<212> DNA
<213> Homo sapiens

<400> 138
gatgctaaga ctccagacct tttgt 25

<210> 139
<211> 25
<212> DNA

<213> Homo sapiens

<400> 139
tggtgtccag cggcagtggg accat 25

<210> 140
<211> 25
<212> DNA
<213> Homo sapiens

<400> 140
gccagaggcg ctacgaagct ttgcc 25

<210> 141
<211> 25
<212> DNA
<213> Homo sapiens

<400> 141
tgccccacct ccagaaagta tttga 25

<210> 142
<211> 25
<212> DNA
<213> Homo sapiens

<400> 142
tgtcccaacc tgttgccatt gattt 25

<210> 143
<211> 25
<212> DNA
<213> Homo sapiens

<400> 143
agaccacttt gagcagttgc ctgga 25

<210> 144
<211> 25
<212> DNA
<213> Homo sapiens

<400> 144
ggtgacataa accattcatt gctac 25

<210> 145
<211> 25
<212> DNA
<213> Homo sapiens

<400> 145
acggatgggt ctcagttaca aataa 25

<210> 146
<211> 25
<212> DNA

<213> Homo sapiens

<400> 146
gcagcccgac caataaacct gcttt 25

<210> 147
<211> 25
<212> DNA
<213> Homo sapiens

<400> 147
agcctctgat gtcagatctt gggtc 25

<210> 148
<211> 25
<212> DNA
<213> Homo sapiens

<400> 148
agctccatgt cctttaaaat cagtc 25

<210> 149
<211> 25
<212> DNA
<213> Homo sapiens

<400> 149
agctacctca ggtgttttta cctca 25

<210> 150
<211> 25
<212> DNA
<213> Homo sapiens

<400> 150
gtgggattgt gtacctttct aagaa 25

<210> 151
<211> 25
<212> DNA
<213> Homo sapiens

<400> 151
tacccacatc aggttattct ccatg 25

<210> 152
<211> 25
<212> DNA
<213> Homo sapiens

<400> 152
tccttgtcca gtgaactgca gcagc 25

<210> 153
<211> 25
<212> DNA

<213> Homo sapiens

<400> 153
gttcagcagc aaacttgcaa cagac 25

<210> 154
<211> 25
<212> DNA
<213> Homo sapiens

<400> 154
tagctgtttc agagagagta cggta 25

<210> 155
<211> 25
<212> DNA
<213> Homo sapiens

<400> 155
cctttgccaa gccatcctgg atgaa 25

<210> 156
<211> 25
<212> DNA
<213> Homo sapiens

<400> 156
ccactgggct ggatttgact gttga 25

<210> 157
<211> 25
<212> DNA
<213> Homo sapiens

<400> 157
ggactcaaag ccaaaactgc ccaaa 25

<210> 158
<211> 25
<212> DNA
<213> Homo sapiens

<400> 158
gccaggcccc atggagggta tactg 25

<210> 159
<211> 25
<212> DNA
<213> Homo sapiens

<400> 159
tgagaagcca accatgcaag ccagc 25

<210> 160
<211> 25
<212> DNA

<213> Homo sapiens

<400> 160
tctacgactt catcgtcacc aaggc 25

<210> 161
<211> 25
<212> DNA
<213> Homo sapiens

<400> 161
acgtaacctg cttagtattg acact 25

<210> 162
<211> 25
<212> DNA
<213> Homo sapiens

<400> 162
gtcacagagt cttaccaaag tcagg 25

<210> 163
<211> 25
<212> DNA
<213> Homo sapiens

<400> 163
gagctgccgg cagagttcaa actgt 25

<210> 164
<211> 25
<212> DNA
<213> Homo sapiens

<400> 164
tgtaaggaag gcttccgtca ccctt 25

<210> 165
<211> 25
<212> DNA
<213> Homo sapiens

<400> 165
gacaactgcg tgggtccaaa cactc 25

<210> 166
<211> 25
<212> DNA
<213> Homo sapiens

<400> 166
tagtacgagc ctggatctgc gtgtc 25

<210> 167
<211> 25
<212> DNA

```
<213>  Homo sapiens

<400>  167
gaaatggtta atcatgctgt gtgct                                          25


<210>  168
<211>  25
<212>  DNA
<213>  Homo sapiens

<400>  168
gatcttaagc ttttgtgtca gatta                                          25


<210>  169
<211>  25
<212>  DNA
<213>  Homo sapiens

<400>  169
gcaaagggcc agttgtttca gttta                                          25
```